# EUROPEAN PATENT SPECIFICATION

(11) **EP 4 351 623 B1**
(45) Date of publication and mention of the grant of the patent: **05.08.2026**
(21) Application number: 22741886.0
(22) Date of filing: 08.06.2022
(51) Int. Cl.: A61K 38/17, A61P 31/12, A61K 31/7105, C07K 14/47, C12N 15/85, A61K 9/51

(54) **GENE COMBINATION AS A BROAD SPECTRUM ANTIVIRAL**
GENKOMBINATION ALS ANTIVIRALES BREITBAND MEDIKAMENT
COMBINAISON DE GÈNES EN TANT QU'ANTIVIRAL À LARGE SPECTRE

(30) Priority: 09.06.2021 US 202163208827 P
(43) Date of publication of application: 17.04.2024
(73) Proprietor: Icahn School of Medicine at Mount Sinai, New York, NY 10029 (US)
(72) Inventor: BOGUNOVIC, Dusan, New York, NY 10029 (US); TAFT, Justin, New York, NY 10029 (US)
(74) Representative: Dehns
(86) International application number: PCT/US2022/032673
(87) International publication number: WO 2022/261206

(56) References cited:
- SCHOGGINS JOHN W: "Annual Review of Virology Interferon-Stimulated Genes: What Do They All Do?", ANNU. REV. VIROL, 5 July 2019 (2019-07-05), pages 567 - 84, XP055961069, Retrieved from the Internet <URL:https://www.annualreviews.org/doi/pdf/10.1146/annurev-virology-092818-015756> [retrieved on 20220914], DOI: 10.1146/annurev-virology-092818-
- SCHOGGINS JOHN W ET AL: "Interferon-stimulated genes and their antiviral effector functions", CURRENT OPINION IN VIROLOGY, vol. 1, no. 6, 1 December 2011 (2011-12-01), United Kingdom, pages 519 - 525, XP055961088, ISSN: 1879-6257, DOI: 10.1016/j.coviro.2011.10.008
- VERHELST JUDITH ET AL: "Interferon-Inducible Protein Mx1 Inhibits Influenza Virus by Interfering with Functional Viral Ribonucleoprotein Complex Assembly", JOURNAL OF VIROLOGY, vol. 86, no. 24, 15 December 2012 (2012-12-15), US, pages 13445 - 13455, XP055960813, ISSN: 0022-538X, Retrieved from the Internet <URL:https://journals.asm.org/doi/pdf/10.1128/JVI.01682-12> [retrieved on 20220914], DOI: 10.1128/JVI.01682-12
- STAEHELI PETER ET AL: "Human MX2/MxB: a Potent Interferon-Induced Postentry Inhibitor of Herpesviruses and HIV-1", JOURNAL OF VIROLOGY, vol. 92, no. 24, 15 December 2018 (2018-12-15), US, XP055961063, ISSN: 0022-538X, Retrieved from the Internet <URL:https://www.ncbi.nlm.nih.gov/pmc/articles/PMC6258936/pdf/e00709-18.pdf> [retrieved on 20220914], DOI: 10.1128/JVI.00709-18
- WEIDNER JESSICA M. ET AL: "Interferon-Induced Cell Membrane Proteins, IFITM3 and Tetherin, Inhibit Vesicular Stomatitis Virus Infection via Distinct Mechanisms", JOURNAL OF VIROLOGY, vol. 84, no. 24, 15 December 2010 (2010-12-15), US, pages 12646 - 12657, XP055961152, ISSN: 0022-538X, Retrieved from the Internet <URL:https://journals.asm.org/doi/pdf/10.1128/JVI.01328-10> [retrieved on 20220914], DOI: 10.1128/JVI.01328-10
- ZURCHER T ET AL: "Mouse Mx2 protein inhibits vesicular stomatitis virus but not influenza virus", VIROLOGY, ELSEVIER, AMSTERDAM, NL, vol. 187, no. 2, 1 April 1992 (1992-04-01), pages 796 - 800, XP026462299, ISSN: 0042-6822, [retrieved on 19920401], DOI: 10.1016/0042-6822(92)90481-4
- SASAKI KEISUKE ET AL: "A single nucleotide polymorphism of porcineMX2gene provides antiviral activity against vesicular stomatitis virus", IMMUNOGENETICS, SPRINGER VERLAG, BERLIN, DE, vol. 66, no. 1, 15 November 2013 (2013-11-15), pages 25 - 32, XP035332500, ISSN: 0093-7711, [retrieved on 20131115], DOI: 10.1007/S00251-013-0745-2
- HACHIM MAHMOOD YASEEN ET AL: "Interferon-Induced Transmembrane Protein (IFITM3) Is Upregulated Explicitly in SARS-CoV-2 Infected Lung Epithelial Cells", FRONTIERS IN IMMUNOLOGY, vol. 11, 10 June 2020 (2020-06-10), pages 1372, XP055961121, DOI: 10.3389/fimmu.2020.01372

## Description

### CROSS-REFERENCE TO RELATED APPLICATIONS

This claims benefit of priority from U.S. Provisional Patent Application No. 63/208,827, filed June 9, 2021.

### GOVERNMENT RIGHTS STATEMENT

This invention was made with Government support under grant number R01AI127372 awarded by the National Institutes of Health (NIH). The Government has certain rights in the invention.

### SEQUENCE LISTING

The instant application contains a Sequence Listing, created on June 3, 2022; the file, in ASCII format, is designated H2375420.txt and is 19.2 KB in size.

### 1. BACKGROUND

There is an urgent need to develop therapeutics to treat COVID-19 and diagnostics to detect severe acute respiratory syndrome coronavirus-2 (SARS-CoV-2). As of June 2, 2021, more than 172,199,076 people globally have tested positive for SARS-CoV-2. In addition, as of June 2, 2021, globally more than 3,696,794 people have died from COVID-19.

Type-I-interferons (IFN-Is) are a group of closely related cytokines that initiate the potent antiviral defenses of the innate immune system. During infection, viral nucleic acid and debris form inflamed cells stimulate a network of innate immune receptors on the cell surface, in the endosome, and in the cytoplasm that alert the cell to presence of a pathogen and activate the IFN system. Capable of signaling as both an autocrine and paracrine messenger, IFN-I cytokine signals through a classical JAK-STAT pathway to induce an antiviral network of IFN-stimulated genes (ISGs). However, precise control of the IFN-I response is crucial to prevent inordinate inflammation and unnecessary damage to the host.

To maintain a measured response, a subset of IFN-I stimulated genes (ISGs) encodes components of the systems' negative regulatory machinery. Among the most important are IFN stimulated gene 15 (ISG15) and ubiquitin specific peptidase 18 (USP18), which function together to displace JAK1 from the IFN-α receptor (IFNAR) to shut down the pathway. Specifically, USP18 is itself the negative regulator that displaces JAK1 from IFNAR, while ISG15 acts to protect USP18 from proteasome-mediated degradation. Therefore, a deficiency for either ISG15 or USP18 cripples the negative regulation of the IFN-I response, which manifests as persistent expression of ISGs. In the case of USP18 deficiency, the levels of persistent ISG expression are too high, and, unfortunately, these individuals die perinatally without immediate detection and therapeutic JAK1 inhibition. ISG15 deficiency, on the other hand, produces a milder form of persistent ISG expression that is not only survivable, but safe and potentially advantageous, as ISG15-null individuals have been documented into their late 20s with no lasting adverse effects. Schoggins ("Annual Review of Virology Interferon-Stimulated Genes: What Do They All Do?",Annu. Rev. Virol, (2019-07-05), 567-584) indicates that several antiviral Interferon Stimulated Genes have been identified by associating gene expression with suppression of viral infection, followed by functional testing and reports the antiviral activity of interferon-stimulated genes like IFITM1, IFITM2, MX1, MX2, IFIT3, IFI6, RSAD2 (viperin), BST2 (tetherin), targeting different steps of the viral replication cycle. Schoggins et al ("Interferon-stimulated genes and their antiviral effector functions", CURRENT OPINION IN VIROLOGY, vol. 1, no. 6, (2011-12-01), 519-525) identifies Interferon Stimulated Genes including MX1, IFI6, IFI27, IFIT1, IFIT3.

### 2. SUMMARY

The invention is set out in the appended set of claims. In some embodiments, provided herein is a composition comprising the following: (i) a polynucleotide encoding MX2, (ii) a polynucleotide encoding IFIT3, (iii) a polynucleotide encoding IFITM1, (iv) a polynucleotide encoding IFI27, (v) a polynucleotide encoding IFIT1, (vi) a polynucleotide encoding BST2, (vii) a polynucleotide encoding IFI6, (viii) a polynucleotide encoding RSAD2, (ix) a polynucleotide encoding MX1, and (x) a polynucleotide encoding IFI30.

The nucleic acid sequences may be in any vector (*e*.*g*., a plasmid or a viral vector). The nucleic acid sequences described herein may be in multiple different vectors or a single vector. In some embodiments, the nucleic acid sequences are chemically modified mRNA or nucleoside-modified mRNA (modRNA). *See*, *e*.*g*., Sultana et al., 2021, Methods Mol. Biol. 2158:281-294 for methods for synthesizing modRNA; *see* also PMID: 32078387 and PMID: 33347328. In certain embodiments, the nucleic acid sequence(s) (*e*.*g*., modRNA) is delivered using a commercial transfection agent, protamine, a liposome (e.g., a protamine liposome or a cationic polymer liposome), a polysaccharide particle, a cationic nanoemulsion, a cationic lipid nanoparticle, a cationic lipid, cholesterol PEG nanoparticle, a dendrimer nanoparticle or other nanoparticle delivery system.

In some embodiments, provided herein is a composition comprising a recombinant virus comprising the following: (i) a polynucleotide encoding MX2, (ii) a polynucleotide encoding IFIT3, (iii) a polynucleotide encoding IFITM1, (iv) a polynucleotide encoding IFI27, (v) a polynucleotide encoding IFIT1, (vi) a polynucleotide encoding BST2, (vii) a polynucleotide encoding IFI6, (viii) a polynucleotide encoding RSAD2, (ix) a polynucleotide encoding MX1, and (x) a polynucleotide encoding IFI30.

In another embodiment, provided herein is a composition comprising 10 recombinant viruses, wherein each recombinant virus comprises a different polynucleotide, and wherein (i) one recombinant virus comprises a polynucleotide encoding MX2, (ii) a second recombinant virus comprises a polynucleotide encoding IFIT3, (iii) a third recombinant virus comprises a polynucleotide encoding IFITM1, (iv) a fourth recombinant virus comprises a polynucleotide encoding IFI27, (v) a fifth recombinant virus comprises a polynucleotide encoding IFIT1, (vi) a sixth recombinant virus comprises a polynucleotide encoding BST2, (vii) a seventh recombinant virus comprises a polynucleotide encoding IFI6, (viii) an eighth recombinant virus comprises a polynucleotide encoding RSAD2, (ix) a ninth recombinant virus comprises a polynucleotide encoding MX1, and (x) a tenth recombinant virus comprises a polynucleotide encoding IFI30. In a specific embodiment, the recombinant viruses do not encode the same ISG protein.

In some embodiments, provided herein are polynucleotides for use in a method for treating or preventing a virus infection, wherein the method comprises administering an effective amount of the following to a human subject in need thereof: (i) a polynucleotide encoding MX2, (ii) a polynucleotide encoding IFIT3, (iii) a polynucleotide encoding IFITM1, (iv) a polynucleotide encoding IFI27, (v) a polynucleotide encoding IFIT1, (vi) a polynucleotide encoding BST2, (vii) a polynucleotide encoding IFI6, (viii) a polynucleotide encoding RSAD2, (ix) a polynucleotide encoding MX1, and (x) a polynucleotide encoding IFI30.

In certain embodiments, the methods are for treating a virus infection. In some embodiments, the methods are for preventing a virus infection. The nucleic acid sequences may be administered to the subject by any vector (*e*.*g*., a plasmid or a viral vector) known to one of skill in the art or described herein. Multiple different vectors or a single vector may be used to administer the nucleic acid sequences described herein. In some embodiments, the nucleic acid sequences are chemically modified mRNA or nucleoside-modified mRNA (modRNA). In certain embodiments, the nucleic acid sequence(s) (*e*.*g*., modRNA) is delivered using a commercial transfection agent, protamine, a liposome (e.g., a protamine liposome or a cationic polymer liposome), a polysaccharide particle, a cationic nanoemulsion, a cationic lipid nanoparticle, a cationic lipid, cholesterol PEG nanoparticle, a dendrimer nanoparticle or other nanoparticle delivery system.

In certain embodiments, provided herein is a recombinant virus for use in a method for treating or preventing a virus infection, the method comprising administering an effective amount of recombinant virus to a human subject in need thereof, wherein the recombinant virus comprises a nucleotide sequence, e.g. a transgene(s), encoding the following: MX2, IFIT3, IFITM1, IFI27, IFIT1, BST2 IFI6, RSAD2, MX1, and IFI30. In certain embodiments, the methods are for treating a virus infection. In some embodiments, the methods are for preventing a virus infection.

In another embodiment, provided herein is ten recombinant viruses for use in a method for treating or preventing a virus infection, comprising administering an effective amount of 10 recombinant viruses to a human subject in need thereof, wherein each recombinant virus comprises a different polynucleotide, and wherein (i) a first recombinant virus comprises a polynucleotide encoding MX2, (ii) a second recombinant virus comprises a polynucleotide encoding IFIT3, (iii) a third recombinant virus comprises a polynucleotide encoding IFITM1, (iv) a fourth recombinant virus comprises a polynucleotide encoding IFI27, (v) a fifth recombinant virus comprises a polynucleotide encoding IFIT1, (vi) a sixth recombinant virus comprises a polynucleotide encoding BST2, (vii) a seventh recombinant virus comprises a polynucleotide encoding IFI6, (viii) an eighth recombinant virus comprises a polynucleotide encoding RSAD2, (ix) a ninth recombinant virus comprises a polynucleotide encoding MX1, and (x) a tenth recombinant virus comprises a polynucleotide encoding IFI30. In a specific embodiment, the recombinant viruses do not encode the same ISG protein. In certain embodiments, the methods are for treating a virus infection. In some embodiments, the methods are for preventing a virus infection.

Also provided herein is a lipid nanoparticle for use in a method for treating or preventing a virus infection, the method comprising administering an effective amount of a lipid nanoparticle to a human subject in need thereof, wherein the lipid nanoparticle comprises the following: a nucleotide sequence encoding MX2, a nucleotide sequence encoding IFIT3, a nucleotide sequence encoding IFITM1, a nucleotide sequence encoding IFI27, a nucleotide sequence encoding IFIT1, a nucleotide sequence encoding BST2, a nucleotide sequence encoding IFI6, a nucleotide sequence encoding RSAD2, a nucleotide sequence encoding MX1, and a nucleotide sequence encoding IFI30.

In some embodiments, a polynucleotide or nucleotide sequence encoding IFIH1 may also be included.

In certain embodiments, the virus infection is a coronavirus, optionally SARS-CoV-2, an influenza virus, optionally influenza A, vesicular stomatitis virus (VSV), a flavivirus, optionally Zika, Sendai virus, a herpes simplex virus 1 (HSV-1), or a Rift valley fever virus infection.

Also provided herein is a pharmaceutical composition comprising the polynucleotides, the recombinant virus or ten recombinant viruses, or the lipid nanoparticle of the present invention, and a pharmaceutically acceptable carrier. In an embodiment, the polynucleotides are modRNA. In another embodiment, the pharmaceutical composition comprising the polynucleotides of the present invention further comprises a lipid nanoparticle.

In another embodiment, provided herein is a composition for use in a method for treating a virus infection, comprising administering a composition described herein. In another embodiment, provided herein is a composition for use in a method for preventing a virus infection, comprising administering a composition described herein.

The virus infection may be an RNA virus or a DNA virus. In a specific embodiment, the virus infection is an RNA virus infection. In certain embodiments, the RNA virus is a coronavirus, an influenza virus (e.g., an influenza A virus or influenza B virus), a vesicular stomatitis virus (VSV) or a Sendai virus infection. In an embodiment, the virus infection is a coronavirus infection. In an example, the virus infection is a SARS-CoV-2 infection. In another specific embodiment, the virus infection is a DNA virus. In certain embodiments, the virus infection is a herpes simplex virus 1 or Rift valley fever virus infection. In a specific embodiment, the virus infection is an infection by any virus described herein. In an embodiment, the virus infection may be infection with a flavivirus, such as Zika virus.

The subject treated in accordance with the methods described herein is a human subject.

### 3. DESCRIPTION OF THE FIGURES

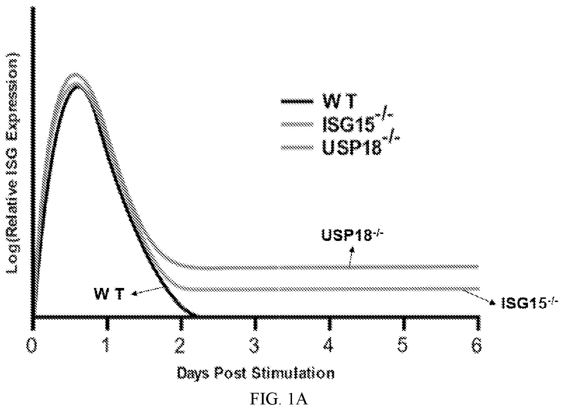

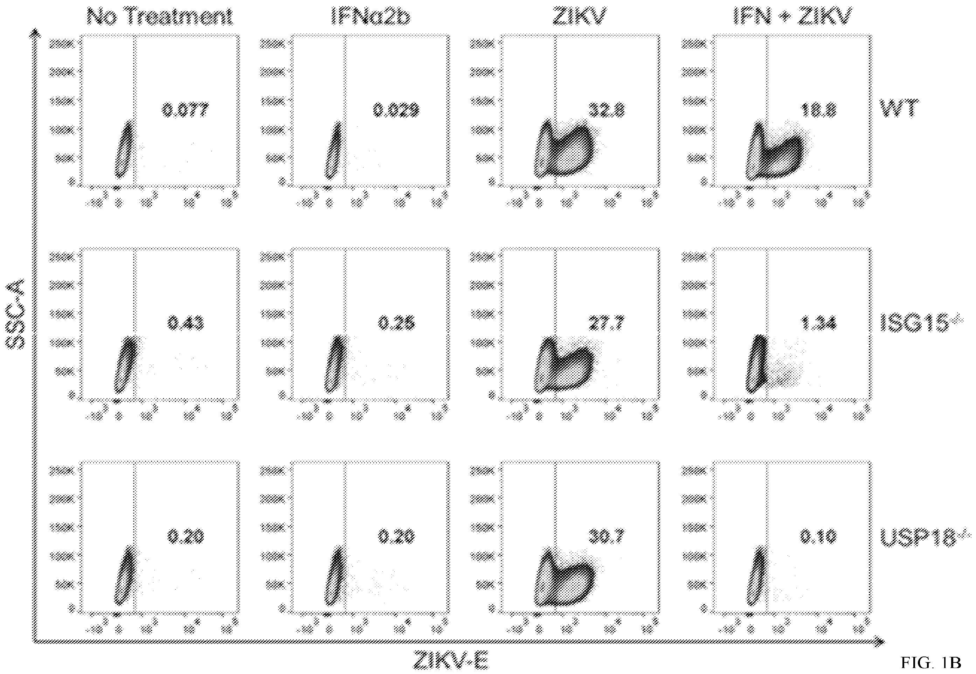

FIGs. 1A and 1B. Persistent ISG expression in ISG15- and USP18-deficient hTERTS produces broad-spectrum viral resistance. FIG. 1A shows cartoon representation of ISG expression kinetics detailing how without ISG15 or USP18 there is incomplete resolution of ISG transcription for as long as 6 days after stimulation. FIG. 1B shows overnight infection with ZIKV (MOI: 1) detected by E protein flow staining in prime/rested hTERTs.

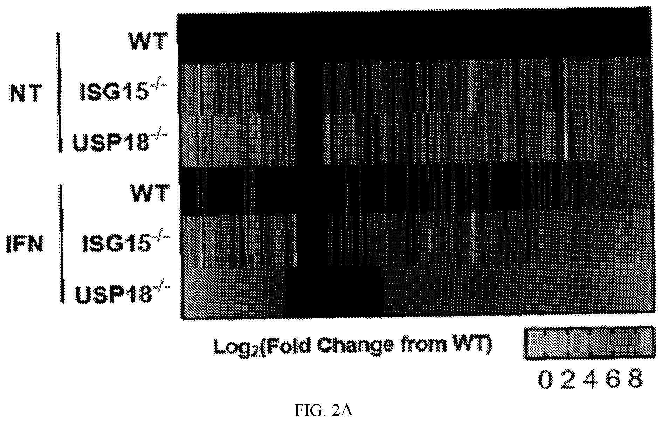

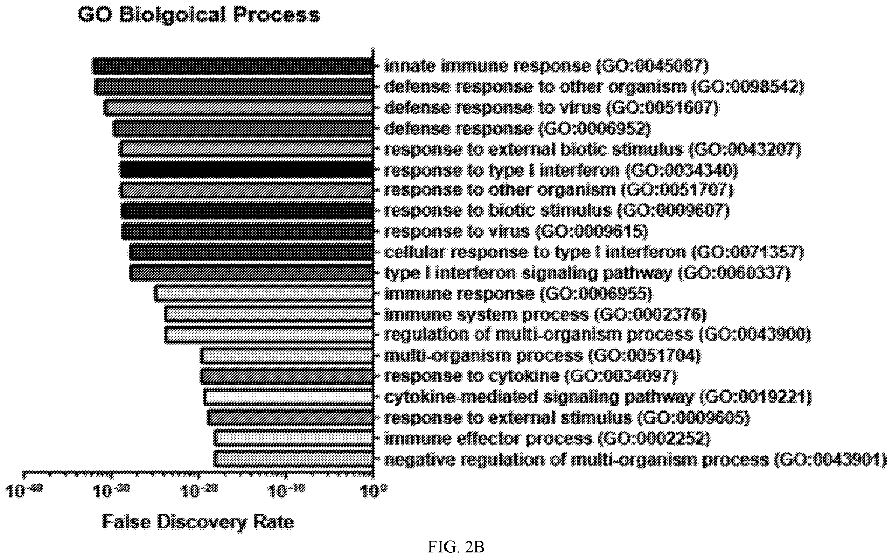

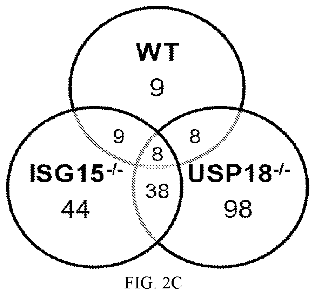

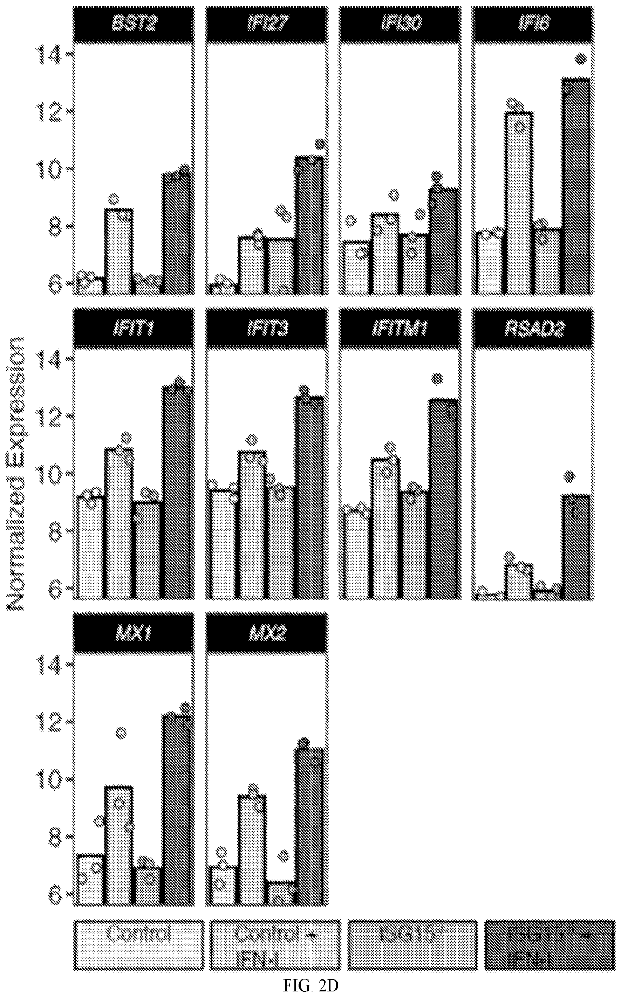

FIGs. 2A-2D. A small fraction of ISGs resist negative regulation in WT and are expressed at higher levels in ISG15-/- and USP18-/- hTERTs. Although most ISGs are returned to baseline following a prime/rest across genetic backgrounds, the few that remain are responsible for enhanced viral control in ISG15- and USP18-null cells. However, a select group of those ISGs remain elevated even in WT. FIG. 2A shows all canonical ISG expression levels in prime/rested hTERTs measured by RNAseq. FIG. 2B shows gene ontology (GO) analysis of the top 20 DEGs in IFN-I prime/rested USP18-/- hTERTs. FIG. 2C shows overlap of significantly differentially expressed ISGs. The significance cutoff was set at a Log2(fold change) > 1, q < 0.05. FIG. 2D shows a small subset of the differentially regulated ISGs in ISG15-/- cells that also remained elevated above baseline in prime-rested fibroblasts from healthy controls, albeit at lower levels as compared to unstimulated WT cells

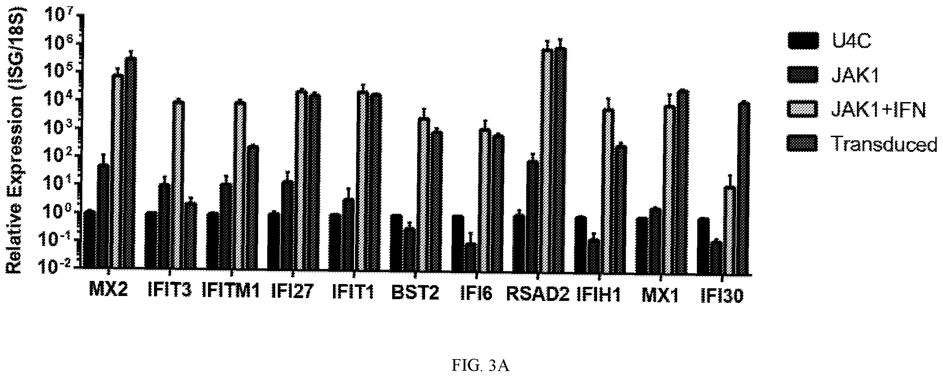

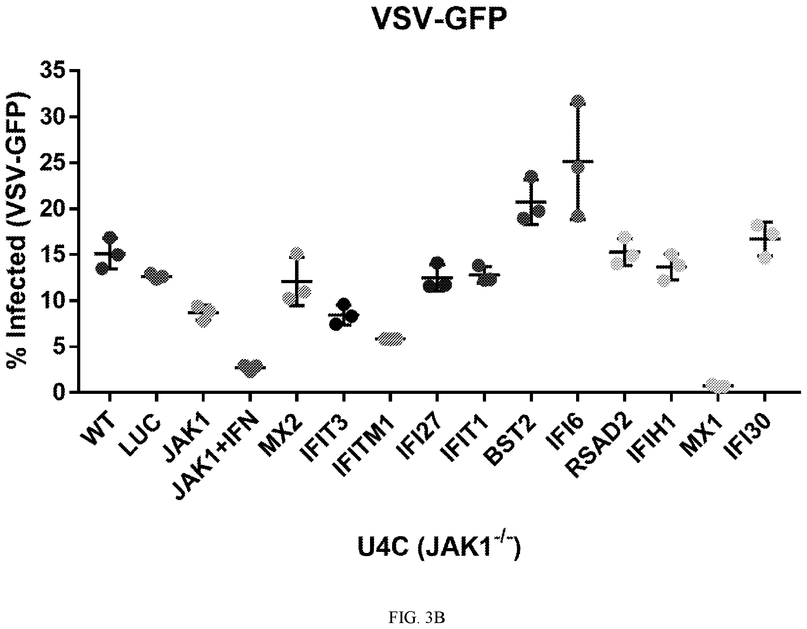

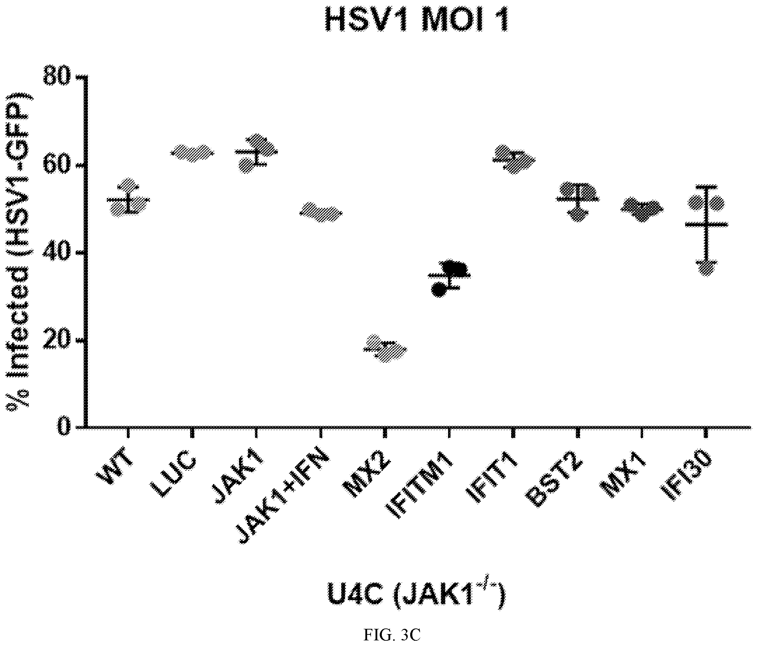

FIGs. 3A-3C. Select persistent ISGs expressed in isolation are restriction factors for specific viruses. All experiments were performed in U4Cs, a JAK1-deficient fibrosarcoma cell line. FIG. 3A shows confirmation of indicated ISG expression after lentiviral transduction by qPCR. 1 ISG per cell line. MX1 was included because of its well-defined anti-viral activity. The bar graphs appear in the following order U4C, JAK1, JAK1+IFN and Transduced. FIG. 3B shows cells from A were challenged with VSV, and FIG. 3C shows HSV1 for 24 hrs.

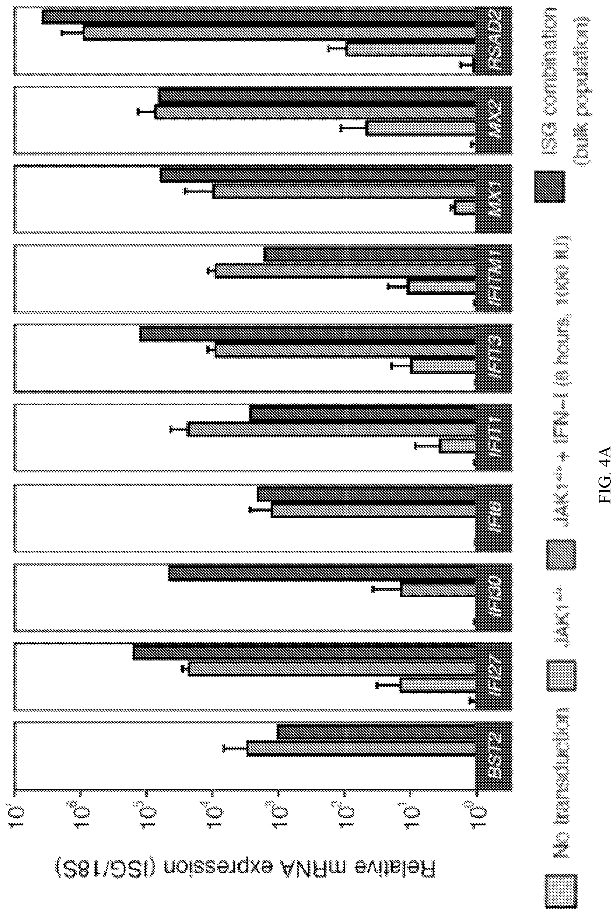

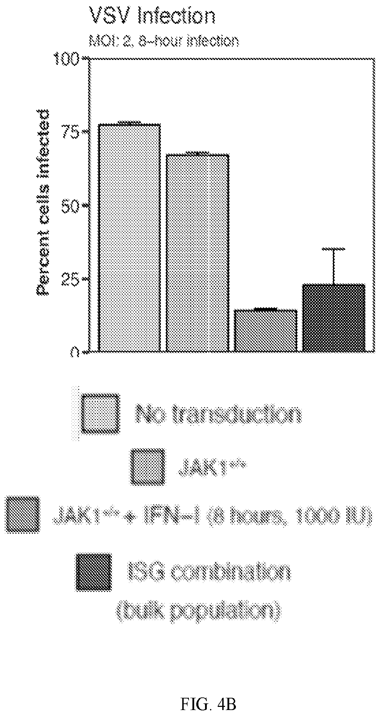

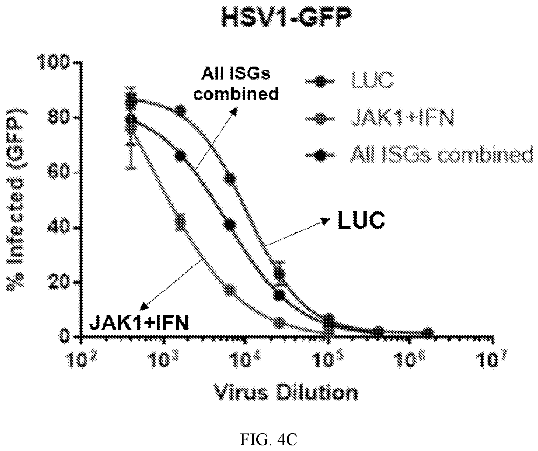

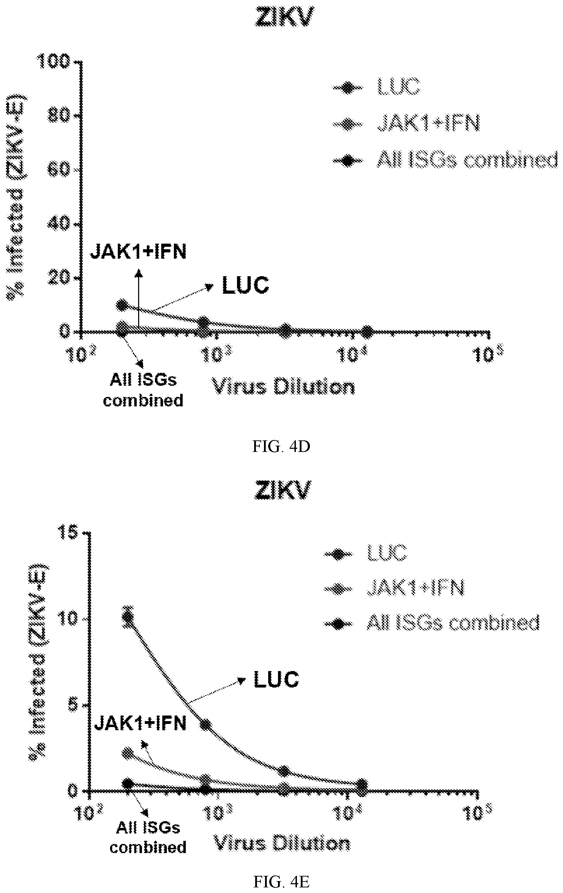

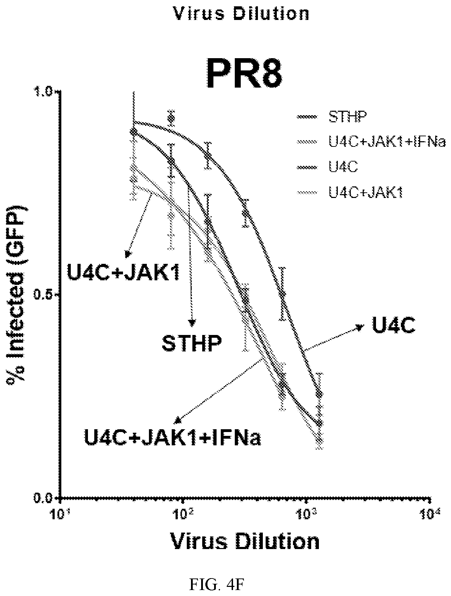

FIGs. 4A-4F. Combined expression of the most persistent ISGs in a single cell yields protection from a broad-spectrum of viral infections. All experiments were performed in U4Cs, a JAK1-deficient fibrosarcoma cell line. FIG. 4A shows confirmation of indicated ISG expression of the STHP-ISG population by qPCR. The bar graphs appear in the following order: U4C, U4C+JAK1, U4C+JAK1 + IFN, and U4C + STHP-ISG. FIG. 4B shows cells from A challenged with VSV, FIG. 4C shows HSV1, FIGs. 4D and 4E shows ZIKV, and FIG. 4F shows PR8 for 24 hrs, except for PR8, which was an 8 hr infection.

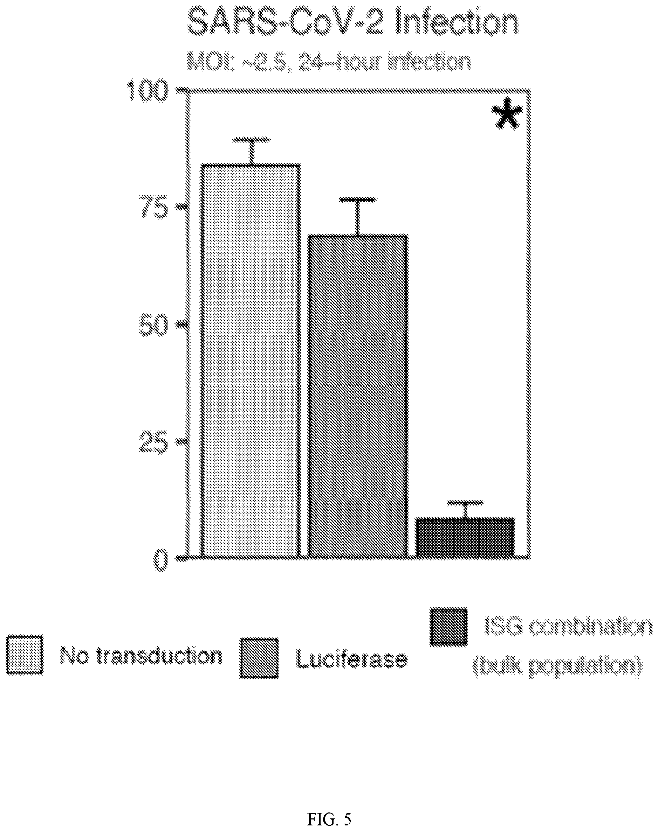

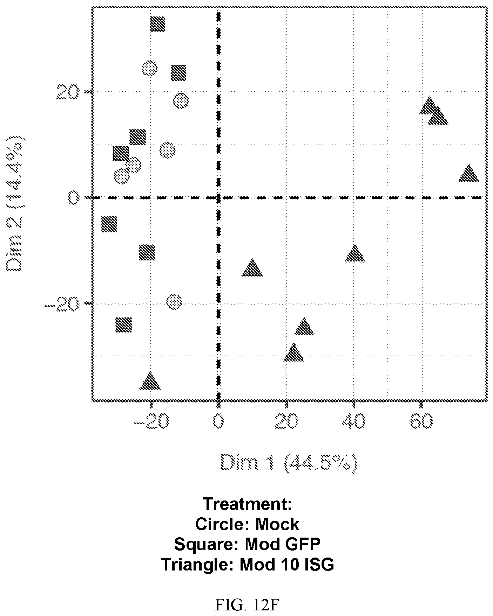

FIG. 5. Combination ISG modRNA transfection inhibits SARS-CoV-2 virus replication. FIG. 5 shows that the combination ISG modRNA transfection (cell line lentivirally engineered to express all 10 ISGs as illustrated in FIG. 12F) inhibits SARS-CoV-2 virus replication. U4C JAK1null-ACE2 expressing cells were plated at a density of 15,000 cells/well and allowed to settle in a 96 well cell culture plate. Cells were treated with modified RNAs in the following conditions: non-transfected (control), luciferase (transfection control), and the ISG combination (STHP-ISGs). STHP-ISGs consist of MX2, IFIT3, IFITM1, IFI27, IFIT1, BST2, IFI6, RSAD2, MX1, and IFI30 and were synthesized as previously described (Sultana et al., 2021, Methods Mol. Biol. 2158:281-294; PMID: 32857381). All treatments were carried out in triplicate. At 18 hours post-transfection, treated cells were challenged with MOI of about 2.5 SARS-CoV-2 virus (USA-WA1) for 24 hours. Infections were fixed and stained with primary polyclonal mouse anti-NP antibody (Clone 4G2, 1: 10,000 dilution) at 4° C overnight in the dark. Next, primary antibody was removed, cells were washed and incubated with rabbit anti-mouse Alexa Flour 647 (1:1,000 dilution) and DAPI (1:10,000 dilution) for 1 hour at room temperature in the dark. Cells were washed and suspended in 200 µL/well PBS and imaged using the Celigo Imaging Cytometer.

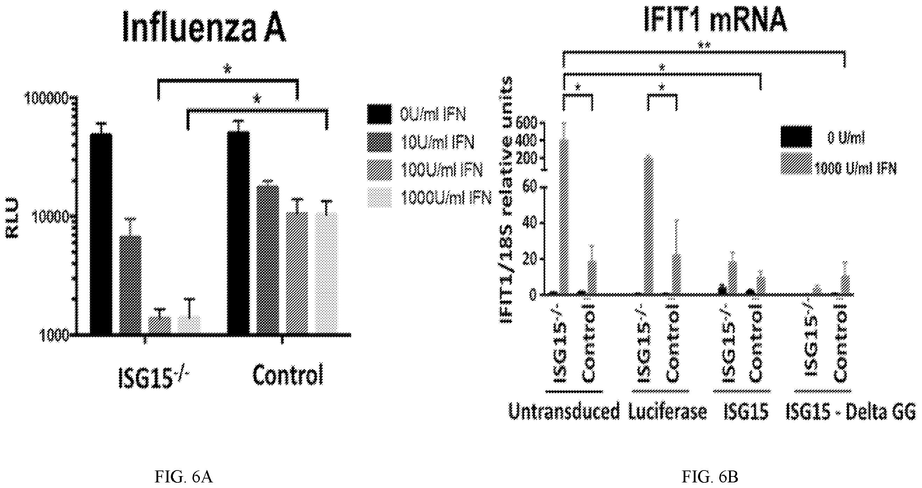

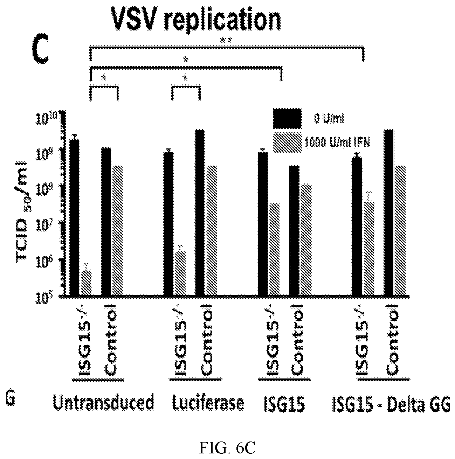

FIGs. 6A-6C. ISG15-deficient cells display enhanced control of IAV and VSV. hTert-immortalized fibroblasts from ISG15-deficient patients (n=3) or controls (n=2 or 3) were treated with 0,10,100 or 1000 IU (left to right) of IFN-I-α2b for 12 h, washed, and allowed to rest for 36 h before infection. FIG. 6A shows luciferase activity in cells infected with IAV PR8-gluc-PTV1 at an MOI of 10.0 for 24 h. Lysates were collected and assayed for luciferase activity. The results are shown in relative luminescence units. hTert-immortalized fibroblasts from ISG15-deficient patients (n=3) or controls (n=2), untransduced or stably transduced with luciferase, ISG15 or ISG15ΔGG, were mock-treated or primed with 1,000 IU ml-1 IFN-I-α2b for 12 h, washed, and allowed to rest for 36 h. FIG. 6B shows rlative IFIT1 mRNA levels 48 h post-priming. FIG. 6C shows TCID levels in fibroblasts infected with VSV at an MOI of 1.0, 48 h post-priming. Supernatants were collected 24 h post-infection and tittered by determining the TCID50 in duplicate.

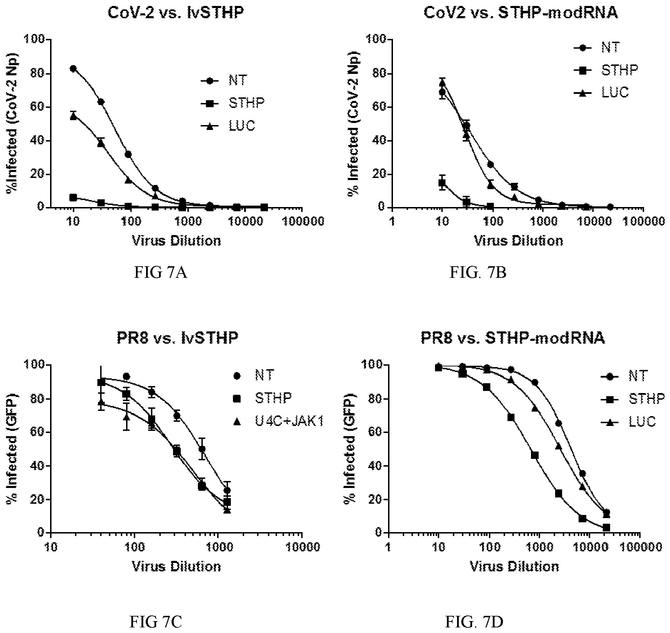

FIGs. 7A-7E. Drug validation in an *in vitro* assay. U4C (JAK1 null) cells were either mock transduced, or transduced with lentiviruses containing JAK1, luciferase (LUC) or 10 ISGs (STHP-ISG) FIG. 7A shows cells transduced with ACE2. Then they were challenged with SARS-CoV-2 (serially diluted) for 24h, followed by staining for NP of SARS-CoV-2. FIG. 7B shows ACE2 transduced U4C (JAK1 null) cells either mock transfected (NT), or transfected with modRNAs encoding either luciferase (LUC) or 10 ISGs (STHP) (transfection was validated using qPCR). Then they were challenged with SARS-CoV-2 (serially diluted) for 24h, followed by staining for NP of SARS-CoV-2. FIG. 7C shows U4C (JAK1 null) cells either mock transduced (NT), or transduced with lentiviruses containing JAK1 (U4C +JAK1) or 10 ISGs (STHP). Then they were challenged with Influenza A PR8 -GFP (serially diluted) FIG. 7D shows U4C (JAK1 null) cells either mock transfected (NT), or transfected with modRNAs encoding either luciferase (LUC) or 10 ISGs (STHP) (transfection was validated using qPCR). Then they were challenges with Influenza A PR8-GFP (serially diluted). STHP-ISGs consist of MX2, IFIT3, IFITM1, IFI27, IFIT1, BST2, IFI6, RSAD2, MX1, and IFI30 each individually introduced via lentiviral particles or modRNAs. ModRNAs were synthesized as previously described (Sultana N, Sharkar MTK, Hadas Y, Chepurko E, Zangi L. In Vitro Synthesis of Modified RNA for Cardiac Gene Therapy. Methods Mol Biol. 2021;2158:281-294.)

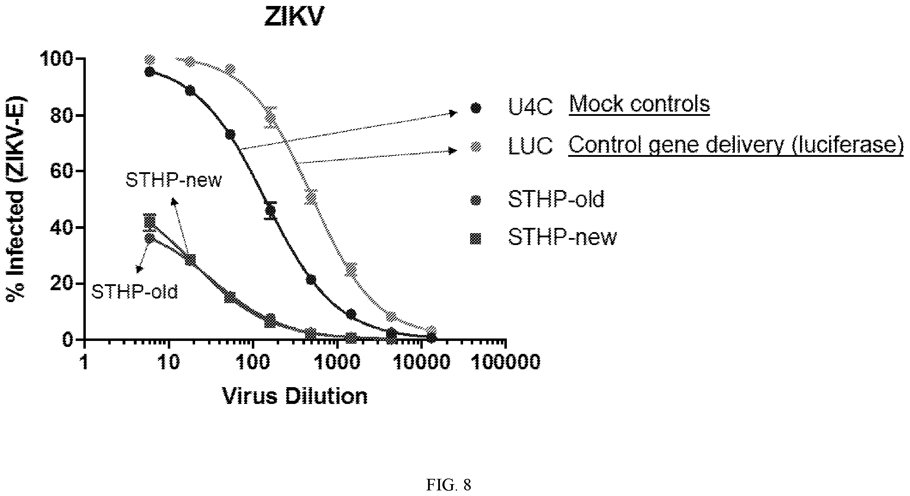

FIG. 8. modRNA delivery of combination of ISGs controls Zika virus replication. 10 ISGs - STHP (MX2, IFIT3, IFITM1, IFI27, IFIT1, BST2, IFI6, RSAD2, MX1, and IFI30) in two different batched (denoting STPH -old and STHP-new) or gene ancoding luciferase (negative control) were synthesized into modRNAs and transfected into U4C cells using RNAiMax transfection reagent or they were mock transfected and rested for 48h. These cell lines were then challenged with ZIKA virus for 24h, fixed and stained for E protein. Using microsopy the number of infected cells was determined.

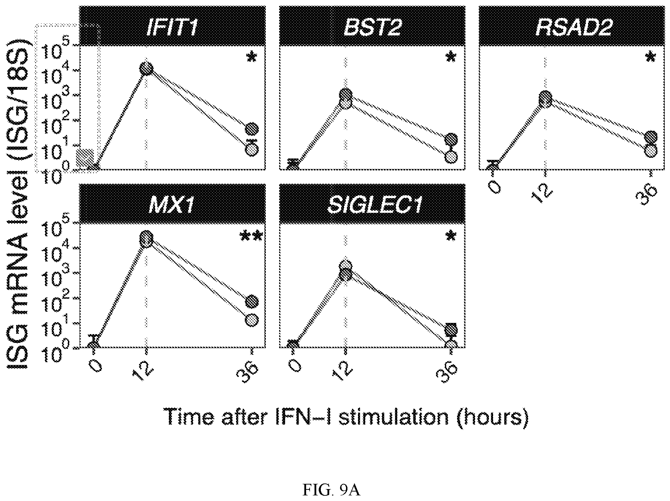

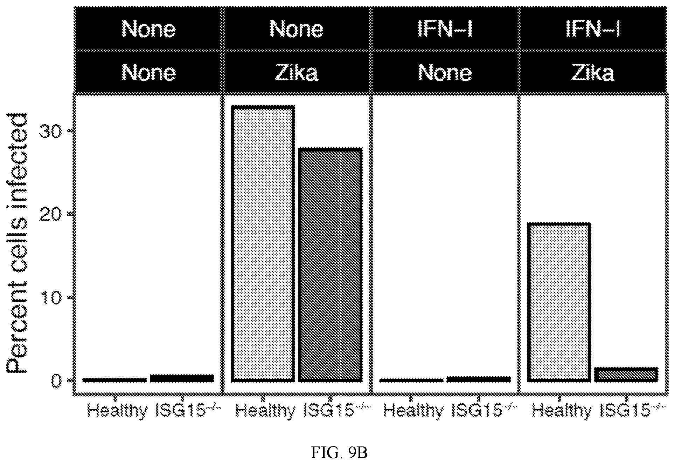

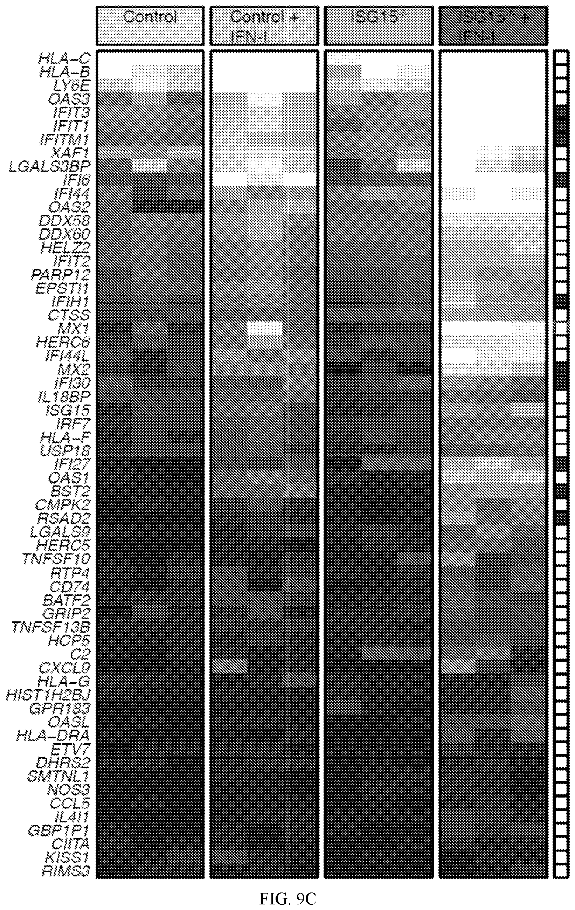

FIG 9A shows fibroblasts stimulated with IFN-I for 12 hours, then washed, then incubated for an additional 36 hours. 48 total hours after IFN-I exposure, USP18, stabilized by ISG15, has downregulated most ISG transcription in fibroblasts from healthy controls, a process that is incomplete in ISG15-/- individuals. Cells stimulated with IFN-I in this fashion (12 hour IFN-I stimulation, 36 hour rest) are referred to herein as "prime/rested." FIG. 9B shows overnight ZIKV challenge at an MOI of 1 in prime/rested fibroblasts was inhibited 14-fold in ISG15-/- compared to similarly treated healthy control cells. FIG. 9C shows RNA sequencing on IFN-I prime/rested fibroblasts from ISG15-/- and wild type individuals. Gene expression analyses identified a number ISGs that were differentially responsive between the two conditions.

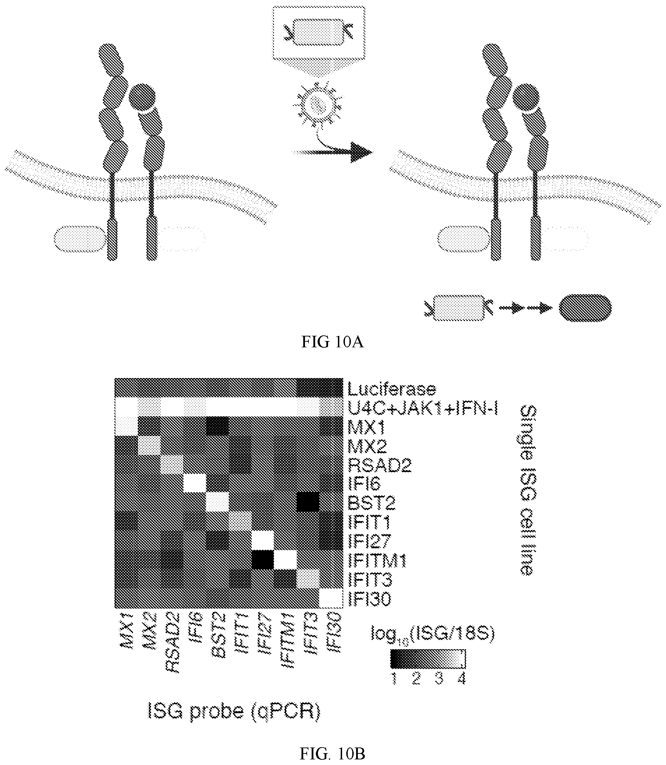

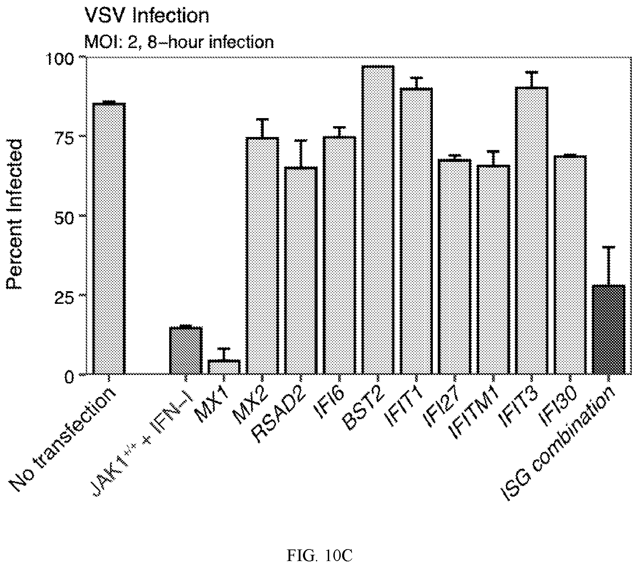

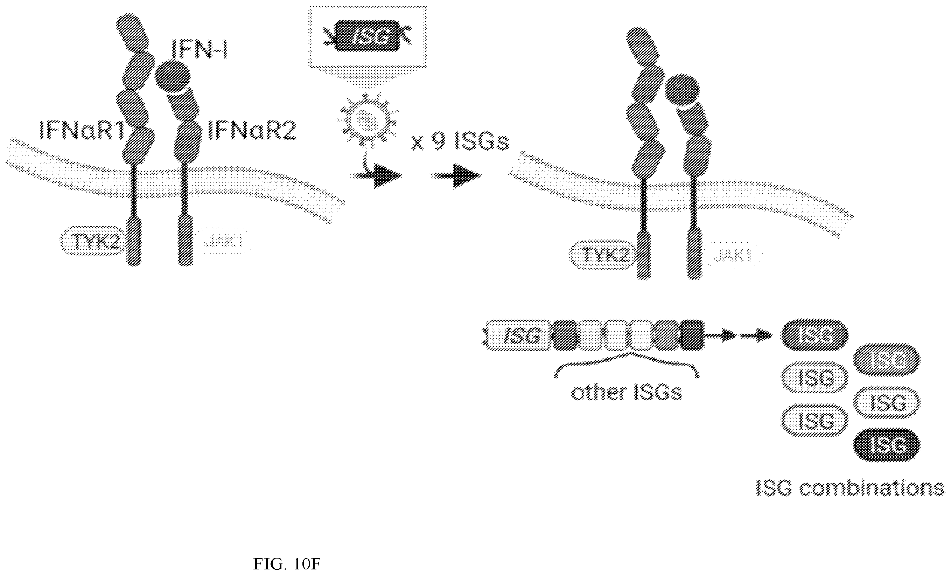

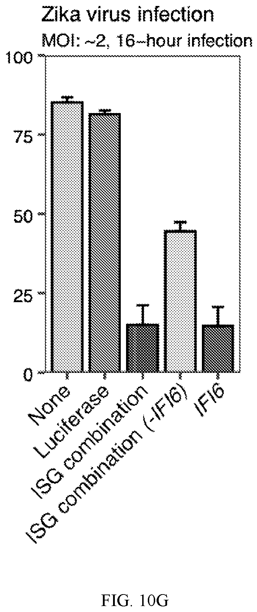

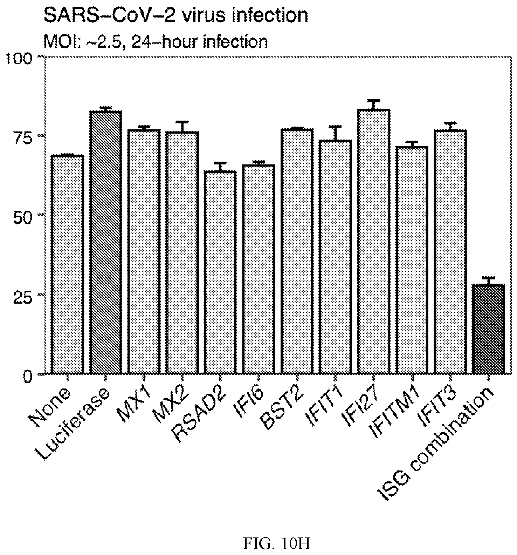

FIGs. 10A-10H. Antiviral potential of ISGs. FIG. 10A shows ISG transfected into JAK1-/-, IFN-I non-responsive fibrosarcoma (U4C) cell lines. FIG. 10B shows validation of select ISG expression in respective cell lines. FIGs. 10C-10G show antiviral potency of these ISGs against Vesicular Stomatitis Virus (VSV), Influenza A virus (IAV PR8), and Zika Virus (ZIKV PR2015). FIG. 10F illustrates a lentivirally or modRNA engineered U4C fibrosarcoma cell line that expresses all 10 of the ISG effectors persistently elevated in healthy and ISG15-/- prime-rested fibroblasts. FIGs. 10G and 10H show broad-spectrum restriction of viral infection by ISG combination transduced cells against Zika Virus (G) and SARS-COV-2 (H).

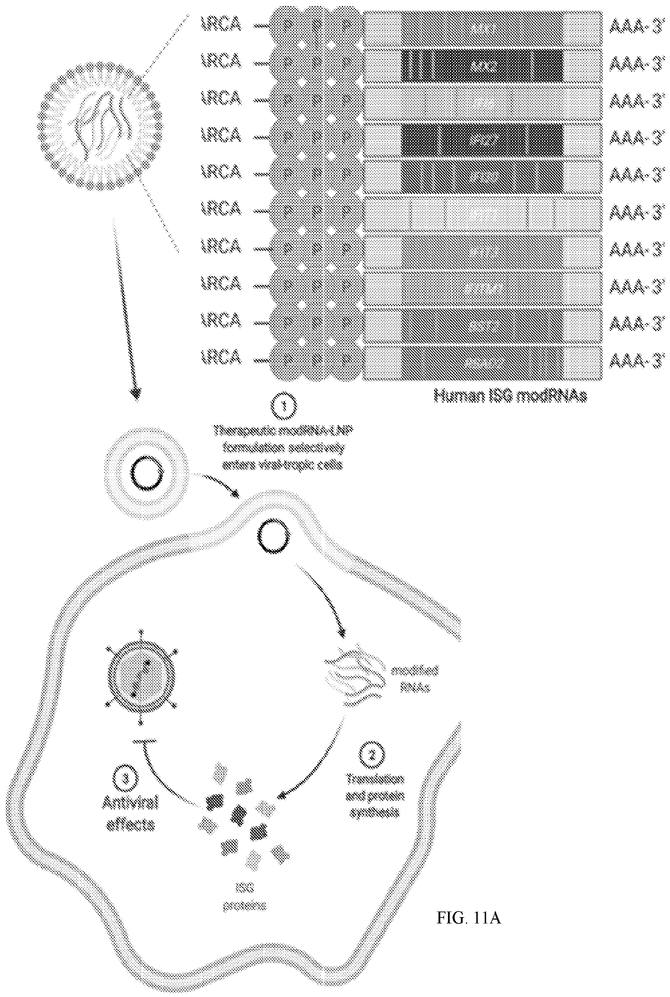

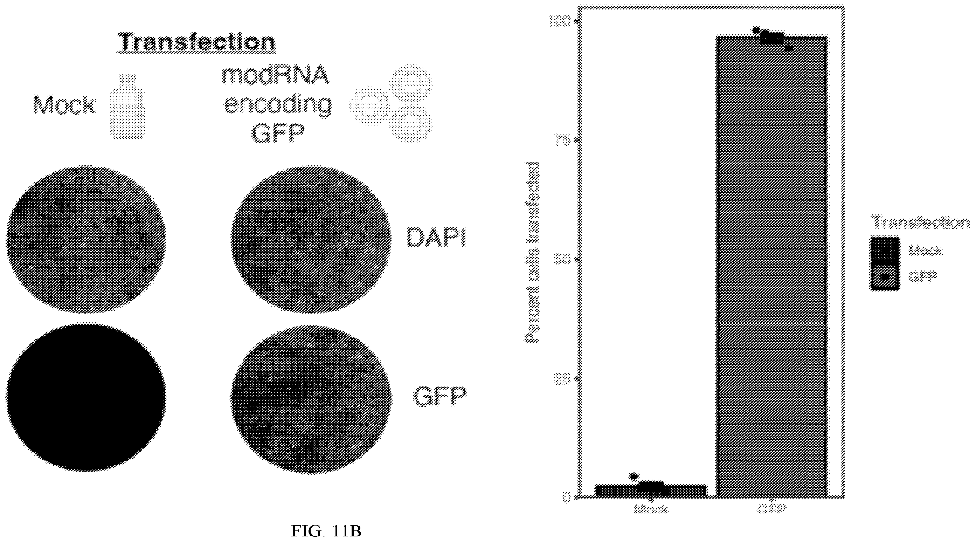

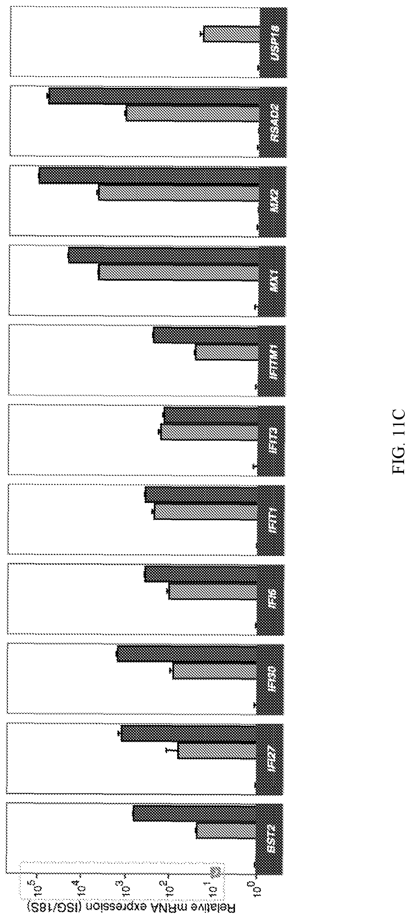

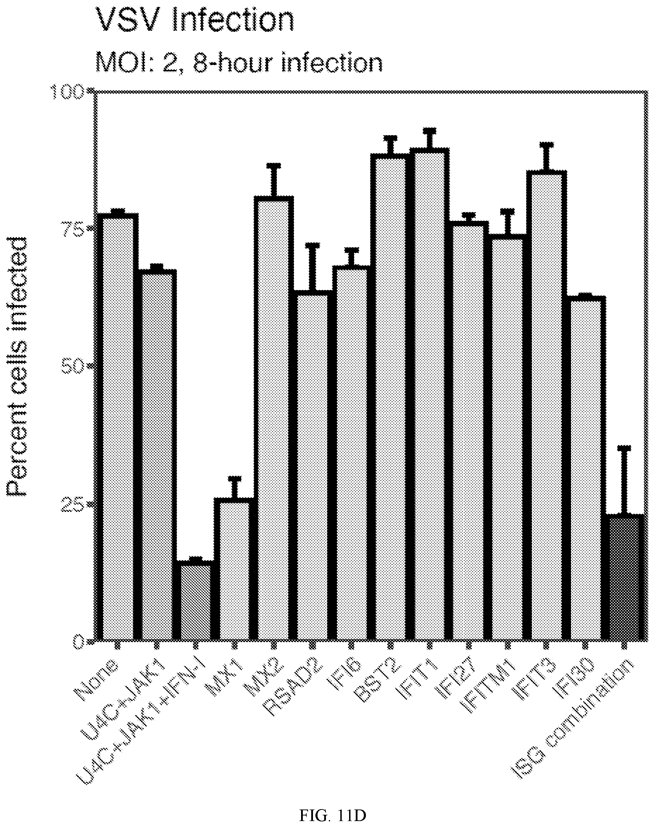

FIGs. 11A-11E. FIG. 11A illustrates U4Cs transfected with lipid nanoparticles (LIPOFECTAMINE^{™} RNAIMAX^{™}; LNPs) carrying synthesized modified RNAs (modRNA) with an ARCA cap and with pseudouridine sybstituted for uridine encoding each ISG (MX2, IFIT3, IFITM1, IFI27, IFIT1, BST2, IFI6, RSAD2, MX1, and IFI30). FIG. 11B shows modRNA levels of GFP in IFN-I non-responsive human cells, 16 hours post-transfection. FIG. 11C shows quantification of levels of expression of 10 ISGs, comparable to peak expression induced by IFN stimulation. FIG. 11D shows VSV infection of clls transfected with individual or 10 ISG.

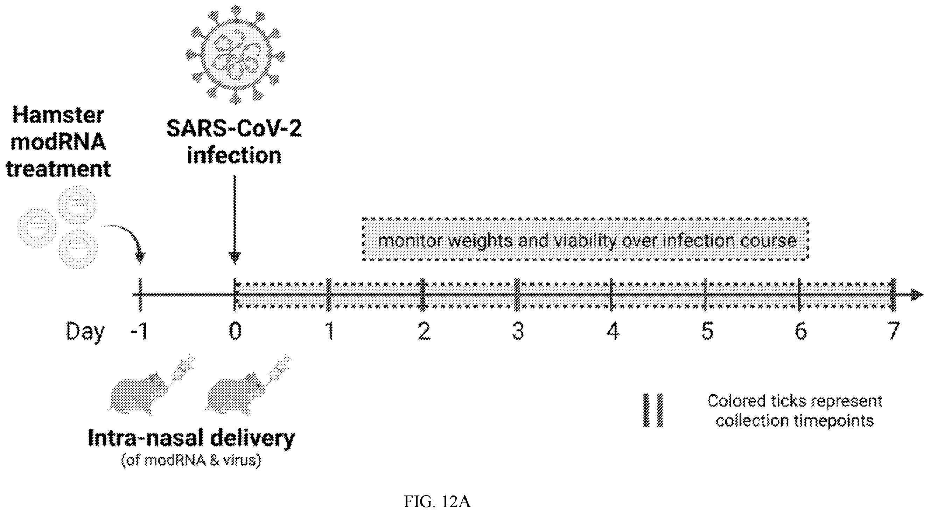

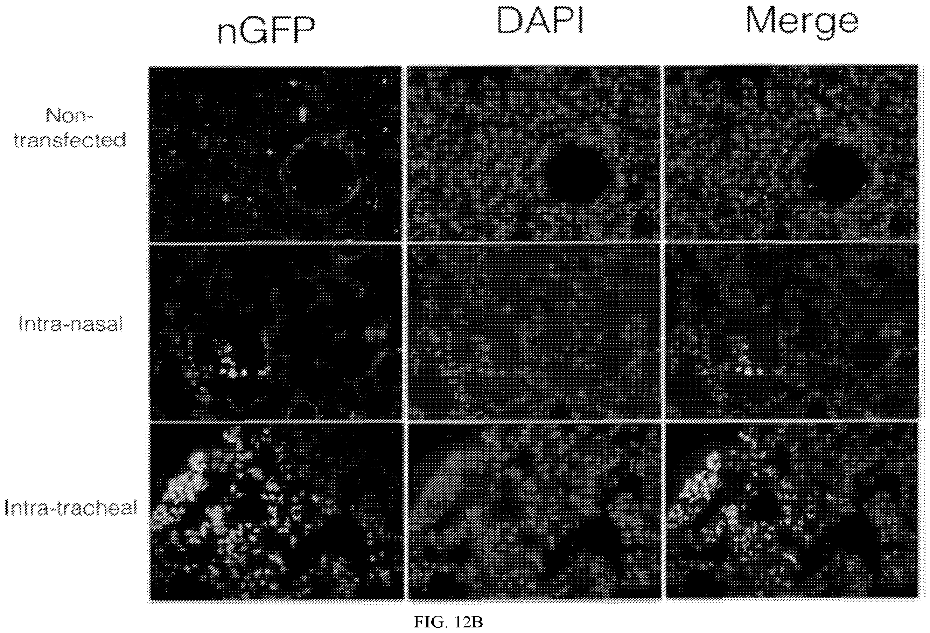

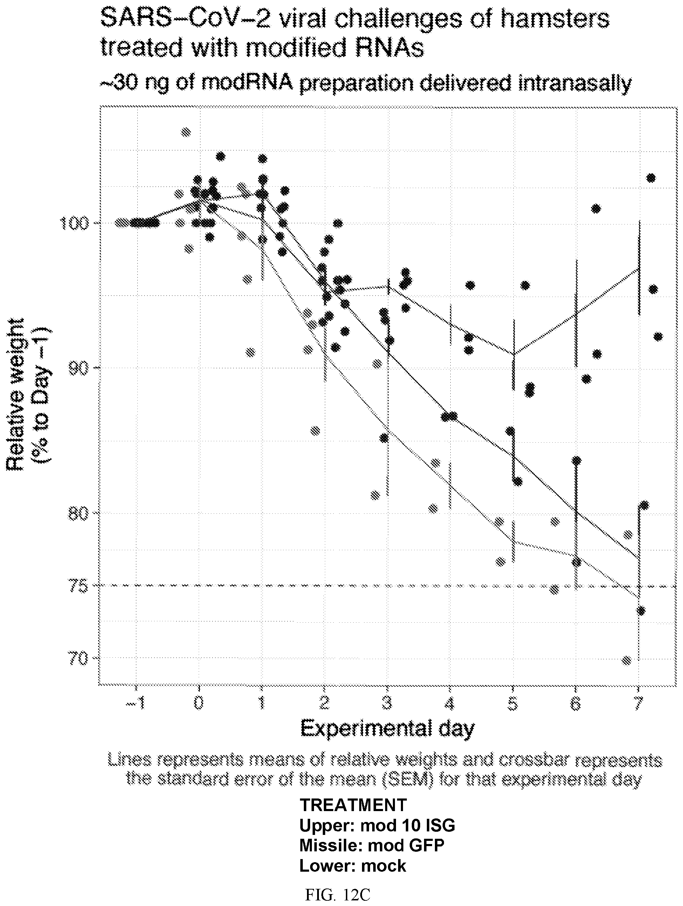

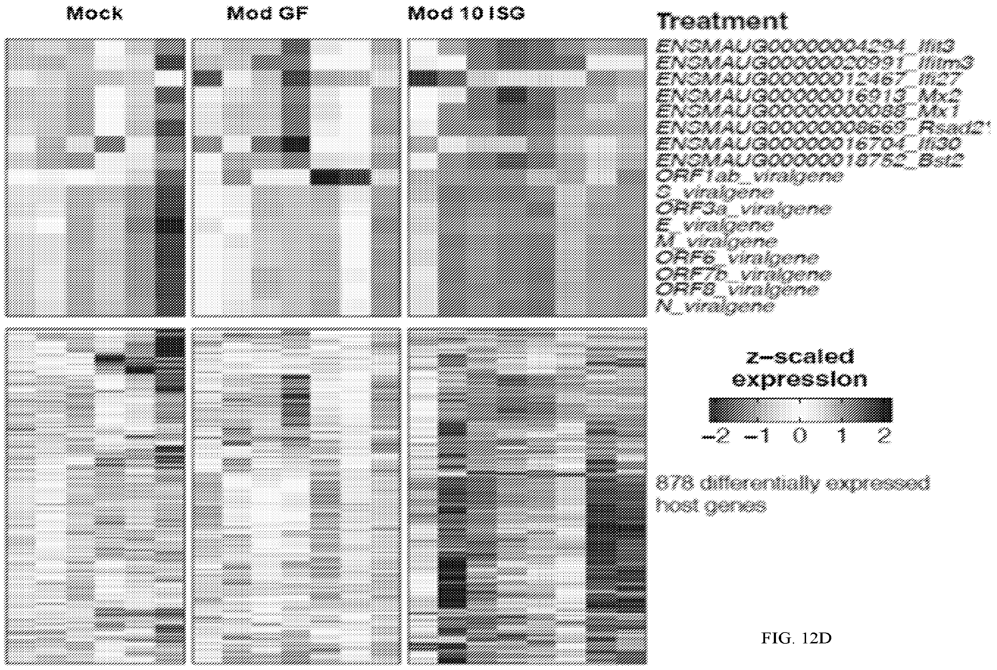

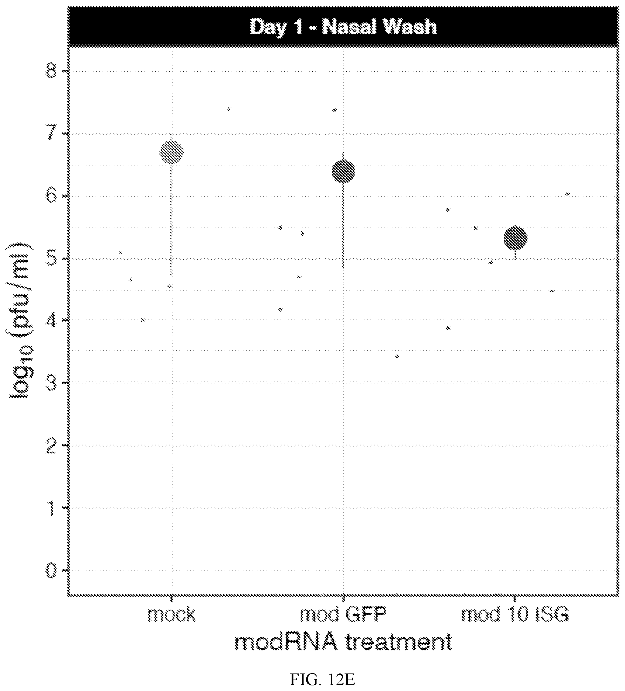

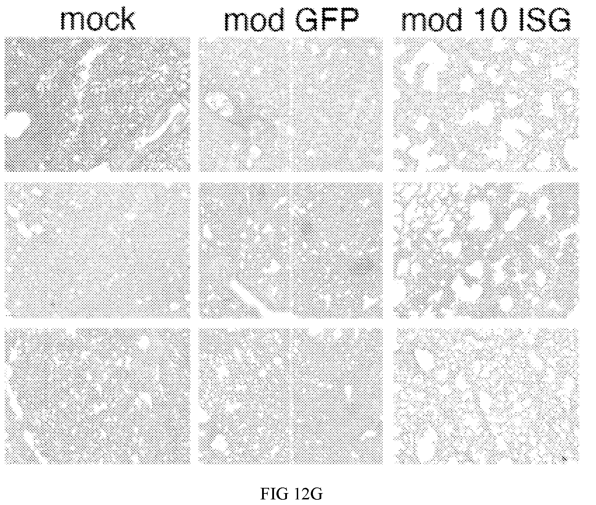

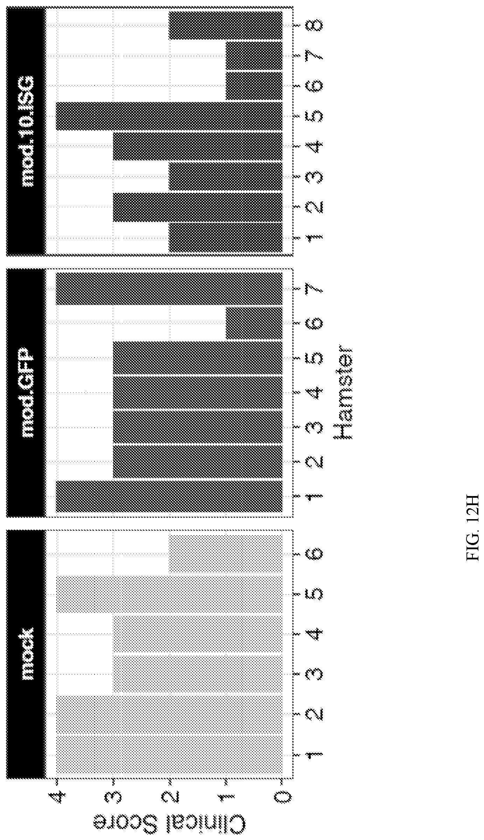

FIGs. 12A-12H. Restriction of viral infection in vive by ISCs. FIG. 12A illustrates study design for testing inhibition of viral infection using LNPs including modRNA for 10 ISGs (MX2, IFIT3, IFITM1, IFI27, IFIT1, BST2, IFI6, RSAD2, MX1, and IFI30). FIG. 12B shows lung expression of transgene following intranasal or intratracheal administration of LNP-modRNA encoding enhanced GFP (eGFP). FIG. 12C shows body weight of subjects administered a sub-lethal level of SARS-CoV-2 and treated intranasally with LNP containing modRNA for the 10 ISG, modRNA for a GFP, or control. FIGs. 12D and 12E show lung RNA seq (12D) and viral titer measurements in the nasal cavity at 1-day post-infection (12E). FIG. 12F shows PCA analyses of RNASeq from lungs of hamsters as indicated. FIGs 12G and 12H show photomicrographs (12G) and histopathology scoring (12H) of postmortem lung tissue.

### 4. DETAILED DESCRIPTION

### 4.1 NUCLEIC ACID SEQUENCES ENCODING PROTEINS, RECOMBINANT PROTEINS, AND VECTORS

Provided herein are nucleic acid sequences comprising nucleotide sequences encoding all of the following: MX2, IFIT3, IFITM1, IFI27, IFIT1, BST2, IFI6, RSAD2, MX1, or IFI30. In a specific embodiment, provided herein is a polynucleotide encoding: MX2, IFIT3, IFITM1, IFI27, IFIT1, BST2, IFI6, RSAD2, MX1, or IFI30. Any nucleotide sequence known to one of skill in the art may be encode: MX2, IFIT3, IFITM1, IFI27, IFIT1, BST2, IFI6, RSAD2, MX1, or IFI30. *See*, *e*.*g*., Sections 5 and 6, *infra*, for information regarding the expression of these sequences. In certain embodiments, a nucleotide sequence encoding MX2, IFIT3, IFITM1, IFI27, IFIT1, BST2, IFI6, RSAD2, MX1, or IFI30 comprises non-standard, naturally modified nucleosides (*e*.*g*., pseudouridine, 5-methylcytosine, 6-methyladenosine, etc.), synthetic nucleoside analogs, such as N1-methylpseudouridine, or a combination thereof. In a specific embodiment, a nucleotide sequence encoding MX2, IFIT3, IFITM1, IFI27, IFIT1, BST2, IFI6, RSAD2, MX1, or IFI30 is modRNA.

In a specific embodiment, provided herein are 10 nucleic acid sequences, wherein one nucleic acid sequence comprises a nucleotide sequence encoding MX2, a second nucleic acid sequence comprises a nucleotide sequence encoding IFIT3, a third nucleic acid sequence comprises a nucleotide sequence encoding IFITM1, a fourth nucleic acid sequence comprises a nucleotide sequence encoding IFI27, a fifth nucleic acid sequence comprises a nucleotide sequence encoding IFIT1, a sixth nucleic acid sequence comprises a nucleotide sequence encoding BST2, a seventh nucleic acid sequence comprises a nucleotide sequence encoding IFI6, an eighth nucleic acid sequence comprises a nucleotide sequence encoding RSAD2, a ninth nucleic acid sequence comprises a nucleotide sequence encoding MX1, and a tenth nucleic acid sequence comprises a nucleotide sequence encoding IFI30. In a specific embodiment, each nucleic acid sequence comprises non-standard, naturally modified nucleosides (*e*.*g*., pseudouridine, 5-methylcytosine, 6-methyladenosine, etc.), synthetic nucleoside analogs, such as N1-methylpseudouridine, or a combination thereof. In another specific embodiment, each nucleic acid sequence is modRNA.

Any codon optimization technique known to one of skill in the art may be used to codon optimize a nucleic acid sequence encoding a protein described herein. Methods of codon optimization are known in the art, *e*.*g*, the OptimumGene^{™} (GenScript^{®}) protocol and Genewiz^{®} protocol. *See* also U.S. Patent No. 8,326,547 for methods for codon optimization. In some embodiments, a nucleotide sequence(s) enoding all of the following: MX2, IFIT3, IFITM1, IFI27, IFIT1, BST2, IFI6, RSAD2, IFIH1, MX1, or IFI30 is codon optimized. In some embodiments, a nucleotide sequence(s) enoding all of the following: MX2, IFIT3, IFITM1, IFI27, IFIT1, BST2, IFI6, RSAD2, MX1, or IFI30 is codon optimized.

The term "MX2" includes any MX2 known to those of skill in the art. In a specific embodiment, the MX2 may be human, dog, cat, horse, pig, or cow MX2. In a specific embodiment, the MX2 is human MX2. GenBank^{™} accession number NP_002454 provides an exemplary human MX2 amino acid sequence.

The term "IFIT3" includes any IFIT3 known to those of skill in the art. In a specific embodiment, the IFIT3 may be human, dog, cat, horse, pig, or cow IFIT3. In a specific embodiment, the IFIT3 is human IFIT3. GenBank^{™} accession number NP_001026853.1 provides an exemplary human IFIT3 amino acid sequence.

The term "IFITM1" includes any IFITM1 known to those of skill in the art. In a specific embodiment, the IFITM1 may be human, dog, cat, horse, pig, or cow IFITM1. In a specific embodiment, the IFITM1 is human IFITM1. GenBank^{™} accession numbers NP_003632.4 and AAH00897.1 provide exemplary human IFITM1 amino acid sequences.

The term "IFI27" includes any IFI27 known to those of skill in the art. In a specific embodiment, the IFI27 may be human, dog, cat, horse, pig, or cow IFI27. In a specific embodiment, the IFI27 is human IFI27. GenBank^{™} accession number AAH15492.1 provides an exemplary human IFI27 amino acid sequence.

The term "IFIT1" includes any IFIT1 known to those of skill in the art. In a specific embodiment, the IFIT1 may be human, dog, cat, horse, pig, or cow IFIT1. In a specific embodiment, the IFIT1 is human IFIT1. GenBank^{™} accession numbers NP_001539.3 and NP_001257858 provide exemplary human IFIT1 amino acid sequences.

The term "BST2" includes any BST2 known to those of skill in the art. In a specific embodiment, the BST2 may be human, dog, cat, horse, pig, or cow BST2. In a specific embodiment, the BST2 is human BST2. GenBank^{™} accession numbers NP_004326.1 and AAH33873.1 provide exemplary human BST2 amino acid sequences.

The term "IF16" includes any IF16 known to those of skill in the art. In a specific embodiment, the IF16 may be human, dog, cat, horse, pig, or cow IF16. In a specific embodiment, the IF16 is human IF16. GenBank^{™} accession number AAH11601.1 provides an exemplary human IF16 amino acid sequence.

The term "RSAD2" includes any RSAD2 known to those of skill in the art. In a specific embodiment, the RSAD2 may be human, dog, cat, horse, pig, or cow RSAD2. In a specific embodiment, the RSAD2 is human RSAD2. GenBank^{™} accession number AAH17969.1 provides an exemplary human RSAD2 amino acid sequence.

The term "IFIH1" includes any IFIH1 known to those of skill in the art. In a specific embodiment, the IFIH1 may be human, dog, cat, horse, pig, or cow IFIH1. In a specific embodiment, the IFIH1 is human IFIH1. GenBank^{™} accession number AAH46208.1 provides an exemplary human IFIH1 amino acid sequence.

The term "MX1" includes any MX1 known to those of skill in the art. In a specific embodiment, the MX1 may be human, dog, cat, horse, pig, or cow MX1. In a specific embodiment, the MX1 is human MX1. GenBank^{™} accession number CAB90556.1 provides an exemplary human MX1 amino acid sequence.

The term "IFI30" includes any IFI30 known to those of skill in the art. In a specific embodiment, the IFI30 may be human, dog, cat, horse, pig, or cow IFI30. In a specific embodiment, the IFI30 is human IFI30. GenBank^{™} accession numbers NP_006323.2 and AAH31020.1 provide exemplary human IFI30 amino acid sequences.

In a specific embodiment, provided herein is a polynucleotide encoded by the amino acid sequence for MX2 (e.g., human MX2). The amino acid sequence for MX2 may comprise the amino acid sequence found at GenBank^{™} accession number NP_002454. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number NP_002454. In some embodiments, provided herein is a nucleotide sequence encoded by an amino acid sequence that is at least 90%, at least 95%, or at least 98% identical to the amino acid sequence found at GenBank^{™} accession number NP_002454. In a specific embodiment, provided herein is a nucleotide sequence encoded by the amino acid sequence found at GenBank^{™} accession number NP_002454 with 1, 2, 3, 4, 5, 6, 7, 8, 9 or 10 amino acid substitutions (e.g., conservative amino acid substitutions). In another specific embodiment, provided herein is a nucleic acid sequence comprising the nucleotide sequence of SEQ ID NO: 1. In certain embodiments, provided herein is a polynucleotide at least 75%, at least 80%, at least 85%, at least 90%, at least 95%, or at least 98% identical to SEQ ID NO:1. In some embodiments, provided herein is a polynucleotide at least 75%, at least 80%, at least 85%, at least 90%, at least 95%, or at least 98% identical to SEQ ID NO: 1. In a specific embodiment, provided herein is a polynucleotide of SEQ ID NO:1 with 1, 2, 3, 4, 5, 6, 7, 8, 9 or 10 nucleotide substitutions. In particular embodiments, the nucleotide sequence encodes an MX2 protein, which retains one, two, or more, or all of the functions of MX2 protein (e.g., human MX2 protein). For example, the MX2 protein may retain its role in regulating nucleocytoplasmic transport, cell-cycle progression, or both.

In a specific embodiment, provided herein is a polynucleotide encoded by the amino acid sequence for IFIT3 (*e*.*g*., human IFIT3). The amino acid sequence for IFIT3 may comprise the amino acid sequence found at GenBank^{™} accession number NP_001026853.1. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number NP_001026853.1. In some embodiments, provided herein is a nucleotide sequence encoded by an amino acid sequence that is at least 90%, at least 95%, or at least 98% identical to the amino acid sequence found at GenBank^{™} accession number NP_001026853.1. In a specific embodiment, provided herein is a nucleotide sequence encoded by the amino acid sequence found at GenBank^{™} accession number NP_001026853.1 with 1, 2, 3, 4, 5, 6, 7, 8, 9 or 10 amino acid substitutions (e.g., conservative amino acid substitutions). In a specific embodiment, provided herein is a nucleic acid sequence comprising the nucleotide sequence of SEQ ID NO:2. In certain embodiments, provided herein is a polynucleotide at least 75%, at least 80%, at least 85%, at least 90%, at least 95%, or at least 98% identical to SEQ ID NO:2. In some embodiments, provided herein is a polynucleotide at least 90%, at least 95%, or at least 98% identical to SEQ ID NO:2. In a specific embodiment, provided herein is a polynucleotide of SEQ ID NO:2 with 1, 2, 3, 4, 5, 6, 7, 8, 9 or 10 nucleotide substitutions. In particular embodiments, the nucleotide sequence encodes an IFIT3 protein, which retains one, two, or more, or all of the functions of IFIT3 protein (e.g., human IFIT3 protein). For example, the IFIT3 protein may retain its role as an inhibitor of cellular as well as viral processes, cell migration, proliferation, signaling, and viral replication.

In a specific embodiment, provided herein is a polynucleotide encoded by the amino acid sequence for IFITM1 (e.g., human IFITM1). The amino acid sequence for IFITM1 may comprise the amino acid sequence found at GenBank^{™} accession number NP_003632.4 or AAH00897.1. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number NP_003632.4 or AAH00897.1. In some embodiments, provided herein is a nucleotide sequence encoded by an amino acid sequence that is at least 90%, at least 95%, or at least 98% identical to the amino acid sequence found at GenBank^{™} accession number NP_003632.4 or AAH00897.1. In a specific embodiment, provided herein is a nucleotide sequence encoded by the amino acid sequence found at GenBank^{™} accession number NP_003632.4 or AAH00897.1 with 1, 2, 3, 4, 5, 6, 7, 8, 9 or 10 amino acid substitutions (e.g., conservative amino acid substitutions). In another specific embodiment, provided herein is a nucleic acid sequence comprising the nucleotide sequence of SEQ ID NO:3. In certain embodiments, provided herein is a polynucleotide at least 75%, at least 80%, at least 85%, at least 90%, at least 95%, or at least 98% identical to SEQ ID NO:3. In some embodiments, provided herein is a polynucleotide at least 90%, at least 95%, or at least 98% identical to SEQ ID NO:3. In a specific embodiment, provided herein is a polynucleotide of SEQ ID NO:3 with 1, 2, 3, 4, 5, 6, 7, 8, 9 or 10 nucleotide substitutions. In particular embodiments, the nucleotide sequence encodes an IFITM1 protein, which retains one, two, or more, or all of the functions of IFITM1 protein (e.g., human IFITM1 protein). For example, the IFITM1 protein may retain its role inhibiting the ERK activation or arresting cell growth in G1 phase in a p53-dependent manner, a positive regulator of osteoblast differentiation, or a combination therof.

In a specific embodiment, provided herein is a polynucleotide encoded by the amino acid sequence for IFI27 (e.g., human IFI27). The amino acid sequence for IFI27 may comprise the amino acid sequence found at GenBank^{™} accession number AAH15492.1. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number AAH15492.1. In some embodiments, provided herein is a nucleotide sequence encoded by an amino acid sequence that is at least 90%, at least 95%, or at least 98% identical to the amino acid sequence found at GenBank^{™} accession number AAH15492.1. In a specific embodiment, provided herein is a nucleotide sequence encoded by the amino acid sequence found at GenBank^{™} accession number AAH15492.1 with 1, 2, 3, 4, 5, 6, 7, 8, 9 or 10 amino acid substitutions (e.g., conservative amino acid substitutions). In another specific embodiment, provided herein is a nucleic acid sequence comprising the nucleotide sequence of SEQ ID NO:4. In certain embodiments, provided herein is a polynucleotide at least 75%, at least 80%, at least 85%, at least 90%, at least 95%, or at least 98% identical to SEQ ID NO:4. In some embodiments, provided herein is a polynucleotide at least 90%, at least 95%, or at least 98% identical to SEQ ID NO:4. In a specific embodiment, provided herein is a polynucleotide of SEQ ID NO:4 with 1, 2, 3, 4, 5, 6, 7, 8, 9 or 10 nucleotide substitutions. In particular embodiments, the nucleotide sequence encodes an IFI27 protein, which retains one, two, or more, or all of the functions of IFI27 protein (e.g., human IFI27 protein). For example, the IFI27 protein may retain its role as an adapter protein involved in different biological processes (PubMed:22427340, PubMed:27194766), as part of signaling pathways that lead to apoptosis, or a combination thereof.

In a specific embodiment, provided herein is a polynucleotide encoded by the amino acid sequence for IFIT1 (e.g., human IFIT1). The amino acid sequence for IFIT1 may comprise the amino acid sequence found at GenBank^{™} accession number NP_001539.3 or NP_001257858. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number NP_001539.3 or NP_001257858. In some embodiments, provided herein is a nucleotide sequence encoded by an amino acid sequence that is at least 90%, at least 95%, or at least 98% identical to the amino acid sequence found at GenBank^{™} accession number NP_001539.3 or NP_001257858. In a specific embodiment, provided herein is a nucleotide sequence encoded by the amino acid sequence found at GenBank^{™} accession number NP_001539.3 or NP_001257858 with 1, 2, 3, 4, 5, 6, 7, 8, 9 or 10 amino acid substitutions (e.g., conservative amino acid substitutions). In another specific embodiment, provided herein is a nucleic acid sequence comprising the nucleotide sequence of SEQ ID NO:5. In certain embodiments, provided herein is a polynucleotide at least 75%, at least 80%, at least 85%, at least 90%, at least 95%, or at least 98% identical to SEQ ID NO:5. In some embodiments, provided herein is a polynucleotide at least 90%, at least 95%, or at least 98% identical to SEQ ID NO:5. In a specific embodiment, provided herein is a polynucleotide of SEQ ID NO:5 with 1, 2, 3, 4, 5, 6, 7, 8, 9 or 10 nucleotide substitutions. In particular embodiments, the nucleotide sequence encodes an IFIT1 protein, which retains one, two, or more, or all of the functions of IFIT1 protein (e.g., human IFIT1 protein). For example, the IFIT1 protein may retain its role acting as a sensor of viral single-stranded RNAs and inhibiting expression of viral messenger RNAs.

In a specific embodiment, provided herein is a polynucleotide encoded by the amino acid sequence for BST2 (e.g., human BST2). The amino acid sequence for BST2 may comprise the amino acid sequence found at GenBank^{™} accession number NP_004326.1 or AAH33873.1. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number NP_004326.1 or AAH33873.1. In some embodiments, provided herein is a nucleotide sequence encoded by an amino acid sequence that is at least 90%, at least 95%, or at least 98% identical to the amino acid sequence found at GenBank^{™} accession number NP_004326.1 or AAH33873.1. In a specific embodiment, provided herein is a nucleotide sequence encoded by the amino acid sequence found at GenBank^{™} accession number NP_004326.1 or AAH33873.1 with 1, 2, 3, 4, 5, 6, 7, 8, 9 or 10 amino acid substitutions (e.g., conservative amino acid substitutions). In another specific embodiment, provided herein is a nucleic acid sequence comprising the nucleotide sequence of SEQ ID NO:6. In certain embodiments, provided herein is a polynucleotide at least 75%, at least 80%, at least 85%, at least 90%, at least 95%, or at least 98% identical to SEQ ID NO:6. In some embodiments, provided herein is a polynucleotide at least 90%, at least 95%, or at least 98% identical to SEQ ID NO:6. In a specific embodiment, provided herein is a polynucleotide of SEQ ID NO:6 with 1, 2, 3, 4, 5, 6, 7, 8, 9 or 10 nucleotide substitutions. In particular embodiments, the nucleotide sequence encodes a BST2 protein, which retains one, two, or more, or all of the functions of BST2 protein (e.g., human BST2 protein). For example, the BST2 protein may retain its role in the organization of the subapical actin cytoskeleton in polarized epithelial cells.

In a specific embodiment, provided herein is a polynucleotide encoded by the amino acid sequence for IFI6 (*e*.*g*., human IFI6). The amino acid sequence for IFI6 may comprise the amino acid sequence found at GenBank^{™} accession number AAH11601.1. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number AAH11601.1. In some embodiments, provided herein is a nucleotide sequence encoded by an amino acid sequence that is at least 90%, at least 95%, or at least 98% identical to the amino acid sequence found at GenBank^{™} accession number AAH11601.1. In a specific embodiment, provided herein is a nucleotide sequence encoded by the amino acid sequence found at GenBank^{™} accession number AAH11601.1 with 1, 2, 3, 4, 5, 6, 7, 8, 9 or 10 amino acid substitutions (e.g., conservative amino acid substitutions). In another specific embodiment, provided herein is a nucleic acid sequence comprising the nucleotide sequence of SEQ ID NO:7. In certain embodiments, provided herein is a polynucleotide at least 75%, at least 80%, at least 85%, at least 90%, at least 95%, or at least 98% identical to SEQ ID NO:7. In some embodiments, provided herein is a polynucleotide at least 90%, at least 95%, or at least 98% identical to SEQ ID NO:7. In a specific embodiment, provided herein is a polynucleotide of SEQ ID NO:7 with 1, 2, 3, 4, 5, 6, 7, 8, 9 or 10 nucleotide substitutions. In particular embodiments, the nucleotide sequence encodes a IFI6 protein, which retains one, two, or more, or all of the functions of IFI6 protein (e.g., human IFI6 protein). For example, the IFI6 protein may retain its role in the regulation of apoptosis.

In a specific embodiment, provided herein is a polynucleotide encoded by the amino acid sequence for RSAD2 (e.g., human RSAD2). The amino acid sequence for RSAD2 may comprise the amino acid sequence found at GenBank^{™} accession number AAH17969.1. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number AAH17969.1. In some embodiments, provided herein is a nucleotide sequence encoded by an amino acid sequence that is at least 90%, at least 95%, or at least 98% identical to the amino acid sequence found at GenBank^{™} accession number AAH17969.1. In a specific embodiment, provided herein is a nucleotide sequence encoded by the amino acid sequence found at GenBank^{™} accession number AAH17969.1 with 1, 2, 3, 4, 5, 6, 7, 8, 9 or 10 amino acid substitutions (e.g., conservative amino acid substitutions). In another specific embodiment, provided herein is a nucleic acid sequence comprising the nucleotide sequence of SEQ ID NO:8. In certain embodiments, provided herein is a polynucleotide at least 75%, at least 80%, at least 85%, at least 90%, at least 95%, or at least 98% identical to SEQ ID NO:8. In some embodiments, provided herein is a polynucleotide at least 90%, at least 95%, or at least 98% identical to SEQ ID NO:8. In a specific embodiment, provided herein is a polynucleotide of SEQ ID NO:8 with 1, 2, 3, 4, 5, 6, 7, 8, 9 or 10 nucleotide substitutions. In particular embodiments, the nucleotide sequence encodes a RSAD2 protein, which retains one, two, or more, or all of the functions of RSAD2 protein (e.g., human RSAD2 protein). For example, the RSAD2 protein may retain its role in cellular antiviral response and innate immune signaling.

In a specific embodiment, provided herein is a polynucleotide encoded by the amino acid sequence for IFIH1 (e.g., human IFIH1). The amino acid sequence for IFIH1 may comprise the amino acid sequence found at GenBank^{™} accession number AAH46208.1. In certain embodiments, provided herein is a nucleotide sequence encoded by an amino acid sequence that is at least 75%, at least 80%, at least 85%, at least 90%, at least 95%, or at least 98% identical to the amino acid sequence found at GenBank^{™} accession number AAH46208.1. In some embodiments, provided herein is a nucleotide sequence encoded by an amino acid sequence that is at least 90%, at least 95%, or at least 98% identical to the amino acid sequence found at GenBank^{™} accession number AAH46208.1. In a specific embodiment, provided herein is a nucleotide sequence encoded by the amino acid sequence found at GenBank^{™} accession number AAH17969.1 with 1, 2, 3, 4, 5, 6, 7, 8, 9 or 10 amino acid substitutions (e.g., conservative amino acid substitutions). In a specific embodiment, provided herein is a nucleic acid sequence comprising the nucleotide sequence of SEQ ID NO:9. In certain embodiments, provided herein is a polynucleotide at least 75%, at least 80%, at least 85%, at least 90%, at least 95%, or at least 98% identical to SEQ ID NO:9. In some embodiments, provided herein is a polynucleotide at least 90%, at least 95%, or at least 98% identical to SEQ ID NO:9. In a specific embodiment, provided herein is a polynucleotide of SEQ ID NO:9 with 1, 2, 3, 4, 5, 6, 7, 8, 9 or 10 nucleotide substitutions. In particular embodiments, the nucleotide sequence encodes a IFIH1 protein, which retains one, two, or more, or all of the functions of IFIH1 protein (e.g., human IFIH1 protein). For example, the IFIH1 protein may retain its role in sensing viral infection and in the activation of a cascade of antiviral responses including the induction of type I interferons and proinflammatory cytokines.

In a specific embodiment, provided herein is a polynucleotide encoded by the amino acid sequence for MX1 (e.g., human MX1). The amino acid sequence for MX1 may comprise the amino acid sequence found at GenBank^{™} accession number CAB90556.1. In certain embodiments, provided herein is a nucleotide sequence encoded by an amino acid sequence that is at least 75%, at least 80%, at least 85%, at least 90%, at least 95%, or at least 98% identical to found at GenBank^{™} accession number CAB90556.1. In some embodiments, provided herein is a nucleotide sequence encoded by an amino acid sequence that is at least 90%, at least 95%, or at least 98% identical to found at GenBank^{™} accession number CAB90556.1. In a specific embodiment, provided herein is a nucleic acid sequence comprising the nucleotide sequence of SEQ ID NO: 10. In certain embodiments, provided herein is a polynucleotide at least 75%, at least 80%, at least 85%, at least 90%, at least 95%, or at least 98% identical to SEQ ID NO:10. In some embodiments, provided herein is a polynucleotide at least 90%, at least 95%, or at least 98% identical to SEQ ID NO:10. In a specific embodiment, provided herein is a polynucleotide of SEQ ID NO:10 with 1, 2, 3, 4, 5, 6, 7, 8, 9 or 10 nucleotide substitutions. In particular embodiments, the nucleotide sequence encodes a MX1 protein, which retains one, two, or more, or all of the functions of MX1 protein (e.g., human MX1 protein). For example, the MX1 protein may retain its antiviral activity.

In a specific embodiment, provided herein is a polynucleotide encoded by the amino acid sequence for IFI30 (e.g., human IFI30). The amino acid sequence for IFI30 may comprise the amino acid sequence found at GenBank^{™} accession number CAB90556.1. In certain embodiments, provided herein is a nucleotide sequence encoded by an amino acid sequence that is at least 75%, at least 80%, at least 85%, at least 90%, at least 95%, or at least 98% identical to found at GenBank^{™} accession number CAB90556.1. In some embodiments, provided herein is a nucleotide sequence encoded by an amino acid sequence that is at least 90%, at least 95%, or at least 98% identical to found at GenBank^{™} accession number CAB90556.1. In a specific embodiment, provided herein is a nucleic acid sequence comprising the nucleotide sequence of SEQ ID NO:11. In certain embodiments, provided herein is a polynucleotide at least 75%, at least 80%, at least 85%, at least 90%, at least 95%, or at least 98% identical to SEQ ID NO:11. In some embodiments, provided herein is a polynucleotide at least 90%, at least 95%, or at least 98% identical to SEQ ID NO:11. In a specific embodiment, provided herein is a polynucleotide of SEQ ID NO:11 with 1, 2, 3, 4, 5, 6, 7, 8, 9 or 10 nucleotide substitutions. In particular embodiments, the nucleotide sequence encodes a IFI30 protein, which retains one, two, or more, or all of the functions of IFI30 protein (e.g., human IFI30 protein). For example, the IFI30 protein may retain its ability to facilitate the complete unfolding of proteins destined for lysosomal degradation or its role in antigen processing.

In a specific embodiment, provided herein is a recombinant protein comprising the amino acid sequence for MX2 (e.g., human MX2). The amino acid sequence for MX2 may comprise the amino acid sequence found at GenBank^{™} accession number NP_002454. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number NP_002454. In some embodiments, provided herein is a recombinant protein comprising an amino acid sequence that is at least 90%, at least 95%, or at least 98% identical to the amino acid sequence found at GenBank^{™} accession number NP_002454. In a specific embodiment, provided herein is a recombinant protein comprising the amino acid sequence found at GenBank^{™} accession number NP_002454 with 1, 2, 3, 4, 5, 6, 7, 8, 9 or 10 amino acid substitutions (e.g., conservative amino acid substitutions). In another specific embodiment, provided herein is a recombinant protein comprising an amino acid sequence encoded by the nucleotide sequence of SEQ ID NO:1. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO: 1. In some embodiments, provided herein is a recombinant protein comprising an amino acid sequence encoded by a nucleotide sequence at least 75%, at least 80%, at least 85%, at least 90%, at least 95%, or at least 98% identical to SEQ ID NO: 1. In a specific embodiment, provided herein is a recombinant protein comprising an amino acid sequence encoded by the nucleotide sequence of SEQ ID NO:1 with 1, 2, 3, 4, 5, 6, 7, 8, 9 or 10 nucleotide substitutions. In particular embodiments, the MX2 protein retains one, two, or more, or all of the functions of MX2 protein (e.g., human MX2 protein). For example, the MX2 protein may retain its role in regulating nucleocytoplasmic transport, cell-cycle progression, or both.

In a specific embodiment, provided herein is a recombinant protein comprising the amino acid sequence for IFIT3 (e.g., human IFIT3). The amino acid sequence for IFIT3 may comprise the amino acid sequence found at GenBank^{™} accession number NP_001026853.1. The amino acid sequence for IFIT3 may comprise the amino acid sequence found at GenBank^{™} accession number NP_001026853.1. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number NP_001026853.1. In some embodiments, provided herein is a recombinant protein comprising an amino acid sequence that is at least 90%, at least 95%, or at least 98% identical to the amino acid sequence found at GenBank^{™} accession number NP_001026853.1. In a specific embodiment, provided herein is a recombinant protein comprising the amino acid sequence found at GenBank^{™} accession number NP_001026853.1 with 1, 2, 3, 4, 5, 6, 7, 8, 9 or 10 amino acid substitutions. In a specific embodiment, provided herein is a recombinant protein comprising an amino acid sequence encoded by the nucleotide sequence of SEQ ID NO:2. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:2. In some embodiments, provided herein is a recombinant protein comprising an amino acid sequence encoded by a nucleotide sequence at least 90%, at least 95%, or at least 98% identical to SEQ ID NO:2. In a specific embodiment, provided herein is a recombinant protein comprising an amino acid sequence encoded by the nucleotide sequence of SEQ ID NO:2 with 1, 2, 3, 4, 5, 6, 7, 8, 9 or 10 nucleotide substitutions. In particular embodiments, the IFIT3 protein retains one, two, or more, or all of the functions of IFIT3 protein (e.g., human IFIT3 protein). For example, the IFIT3 protein may retain its role as an inhibitor of cellular as well as viral processes, cell migration, proliferation, signaling, and viral replication.

In a specific embodiment, provided herein is a recombinant protein comprising the amino acid sequence for IFITM1 (e.g., human IFITM1). The amino acid sequence for IFITM1 may comprise the amino acid sequence found at GenBank^{™} accession number NP_003632.4 or AAH00897.1. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number NP_003632.4 or AAH00897.1. In some embodiments, provided herein is a recombinant protein comprising an amino acid sequence that is at least 90%, at least 95%, or at least 98% identical to the amino acid sequence found at GenBank^{™} accession number NP_003632.4 or AAH00897.1. In a specific embodiment, provided herein is a recombinant protein comprising the amino acid sequence found at GenBank^{™} accession number NP_003632.4 or AAH00897.1 with 1, 2, 3, 4, 5, 6, 7, 8, 9 or 10 amino acid substitutions. In another specific embodiment, provided herein is a recombinant protein comprising an amino acid sequence encoded by the nucleotide sequence of SEQ ID NO:3. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:3. In some embodiments, provided herein is a recombinant protein comprising an amino acid sequence encoded by a nucleotide sequence at least 90%, at least 95%, or at least 98% identical to SEQ ID NO:3. In a specific embodiment, provided herein is recombinant protein comprising an amino acid sequence encoded by the nucleotide sequence of SEQ ID NO:3 with 1, 2, 3, 4, 5, 6, 7, 8, 9 or 10 nucleotide substitutions. In particular embodiments, the IFITM1protein retains one, two, or more, or all of the functions of IFITM1 protein (e.g., human IFITM1 protein). For example, the IFITM1 protein may retain its role inhibiting the ERK activation or arresting cell growth in G1 phase in a p53-dependent manner, a positive regulator of osteoblast differentiation, or a combination thereof.

In a specific embodiment, provided herein is a recombinant protein comprising the amino acid sequence for IFI27 (e.g., human IFI27). The amino acid sequence for IFI27 may comprise the amino acid sequence found at GenBank^{™} accession number AAH15492.1. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number AAH15492.1. In some embodiments, provided herein is a recombinant protein comprising an amino acid sequence that is at least 90%, at least 95%, or at least 98% identical to the amino acid sequence found at GenBank^{™} accession number AAH15492.1. In a specific embodiment, provided herein is a recombinant protein comprising the amino acid sequence found at GenBank^{™} accession number AAH15492.1 with 1, 2, 3, 4, 5, 6, 7, 8, 9 or 10 amino acid substitutions. In another specific embodiment, provided herein is a recombinant protein comprising an amino acid sequence encoded by the nucleotide sequence of SEQ ID NO:4. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:4. In some embodiments, provided herein is a recombinant protein comprising an amino acid sequence encoded by a nucleotide sequence at least 90%, at least 95%, or at least 98% identical to SEQ ID NO:4. In a specific embodiment, provided herein is a recombinant protein comprising an amino acid sequence encoded by the nucleotide sequence of SEQ ID NO:4 with 1, 2, 3, 4, 5, 6, 7, 8, 9 or 10 nucleotide substitutions. In particular embodiments, the IFI27 protein retains one, two, or more, or all of the functions of IFI27 protein (e.g., human IFI27 protein). For example, the IFI27 protein may retain its role as an adapter protein involved in different biological processes (PubMed:22427340, PubMed:27194766), as part of signaling pathways that lead to apoptosis, or a combination thereof.

In a specific embodiment, provided herein is a recombinant protein comprising the amino acid sequence for IFIT1 (e.g., human IFIT1). The amino acid sequence for IFIT1 may comprise the amino acid sequence found at GenBank^{™} accession number NP_001539.3 or NP_001257858. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number NP_001539.3 or NP_001257858. In some embodiments, provided herein is a recombinant protein comprising an amino acid sequence that is at least 90%, at least 95%, or at least 98% identical to the amino acid sequence found at GenBank^{™} accession number NP_001539.3 or NP _001257858. In a specific embodiment, provided herein is a recombinant protein comprising the amino acid sequence found at GenBank^{™} accession number NP_001539.3 or NP_001257858 with 1, 2, 3, 4, 5, 6, 7, 8, 9 or 10 amino acid substitutions. In another specific embodiment, provided herein is a recombinant protein comprising an amino acid sequence encoded by the nucleotide sequence of SEQ ID NO:5. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:5. In some embodiments, provided herein is a recombinant protein comprising an amino acid sequence encoded by a nucleotide sequence at least 90%, at least 95%, or at least 98% identical to SEQ ID NO:5. In a specific embodiment, provided herein is a recombinant protein comprising an amino acid sequence encoded by the nucleotide sequence of SEQ ID NO:5 with 1, 2, 3, 4, 5, 6, 7, 8, 9 or 10 nucleotide substitutions. In particular embodiments, the IFIT1 protein retains one, two, or more, or all of the functions of IFIT1 protein (e.g., human IFIT1 protein). For example, the IFIT1 protein may retain its role acting as a sensor of viral single-stranded RNAs and inhibiting expression of viral messenger RNAs.

In a specific embodiment, provided herein is a recombinant protein comprising the amino acid sequence for BST2 (e.g., human BST2). The amino acid sequence for BST2 may comprise the amino acid sequence found at GenBank^{™} accession number NP_004326.1 or AAH33873.1. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number NP_004326.1 or AAH33873.1. In some embodiments, provided herein is a recombinant protein comprising an amino acid sequence that is at least 90%, at least 95%, or at least 98% identical to the amino acid sequence found at GenBank^{™} accession number NP_004326.1 or AAH33873.1. In a specific embodiment, provided herein is a recombinant protein comprising the amino acid sequence found at GenBank^{™} accession number NP_004326.1 or AAH33873.1 with 1, 2, 3, 4, 5, 6, 7, 8, 9 or 10 amino acid substitutions. In another specific embodiment, provided herein is a recombinant protein comprising an amino acid sequence encoded by the nucleotide sequence of SEQ ID NO:6. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:6. In some embodiments, provided herein is a recombinant protein comprising an amino acid sequence encoded by a nucleotide sequence at least 90%, at least 95%, or at least 98% identical to SEQ ID NO:6. In a specific embodiment, provided herein is a recombinant protein comprising an amino acid sequence encoded by the nucleotide sequence of SEQ ID NO:6 with 1, 2, 3, 4, 5, 6, 7, 8, 9 or 10 nucleotide substitutions. In particular embodiments, the BST2 protein retains one, two, or more, or all of the functions of BST2 protein (e.g., human BST2 protein). For example, the BST2 protein may retain its role in the organization of the subapical actin cytoskeleton in polarized epithelial cells.

In a specific embodiment, provided herein is a recombinant protein comprising the amino acid sequence for IFI6 (e.g., human IFI6). The amino acid sequence for IFI6 may comprise the amino acid sequence found at GenBank^{™} accession number AAH11601.1. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number AAH11601.1. In some embodiments, provided herein is a recombinant protein comprising an amino acid sequence that is at least 90%, at least 95%, or at least 98% identical to the amino acid sequence found at GenBank^{™} accession number AAH11601.1. In a specific embodiment, provided herein is a recombinant protein comprising the amino acid sequence found at GenBank^{™} accession number AAH11601.1 with 1, 2, 3, 4, 5, 6, 7, 8, 9 or 10 amino acid substitutions. In another specific embodiment, provided herein is a recombinant protein comprising an amino acid sequence encoded by the nucleotide sequence of SEQ ID NO:7. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:7. In some embodiments, provided herein is a recombinant protein comprising an amino acid sequence encoded by a nucleotide sequence at least 90%, at least 95%, or at least 98% identical to SEQ ID NO:7. In a specific embodiment, provided herein is a recombinant protein comprising an amino acid sequence encoded by the nucleotide sequence of SEQ ID NO:7 with 1, 2, 3, 4, 5, 6, 7, 8, 9 or 10 nucleotide substitutions. In particular embodiments, the IFI6 protein retains one, two, or more, or all of the functions of IFI6 protein (e.g., human IFI6 protein). For example, the IFI6 protein may retain its role in the regulation of apoptosis.

In a specific embodiment, provided herein is a recombinant protein comprising the amino acid sequence for RSAD2 (e.g., human RSAD2). The amino acid sequence for RSAD2 may comprise the amino acid sequence found at GenBank^{™} accession number AAH17969.1. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number AAH17969.1. In some embodiments, provided herein is a recombinant protein comprising an amino acid sequence that is at least 90%, at least 95%, or at least 98% identical to the amino acid sequence found at GenBank^{™} accession number AAH17969.1. In a specific embodiment, provided herein is a recombinant protein comprising the amino acid sequence found at GenBank^{™} accession number AAH17969.1 with 1, 2, 3, 4, 5, 6, 7, 8, 9 or 10 amino acid substitutions. In another specific embodiment, provided herein is a recombinant protein comprising an amino acid sequence encoded by the nucleotide sequence of SEQ ID NO:8. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:8. In some embodiments, provided herein is a recombinant protein comprising an amino acid sequence encoded by a nucleotide sequence at least 90%, at least 95%, or at least 98% identical to SEQ ID NO:8. In a specific embodiment, provided herein is a recombinant protein comprising an amino acid sequence encoded by the nucleotide sequence of SEQ ID NO:8 with 1, 2, 3, 4, 5, 6, 7, 8, 9 or 10 nucleotide substitutions. In particular embodiments, the RSAD2 protein retains one, two, or more, or all of the functions of RSAD2 protein (e.g., human RSAD2 protein). For example, the RSAD2 protein may retain its role in cellular antiviral response and innate immune signaling.

In a specific embodiment, provided herein is a recombinant protein comprising the amino acid sequence for IFIH1 (e.g., human IFIH1). The amino acid sequence for IFIH1 may comprise the amino acid sequence found at GenBank^{™} accession number AAH46208.1. In certain embodiments, provided herein is a recombinant protein comprising an amino acid sequence that is at least 75%, at least 80%, at least 85%, at least 90%, at least 95%, or at least 98% identical to the amino acid sequence found at GenBank^{™} accession number AAH46208.1. In some embodiments, provided herein is a recombinant protein comprising an amino acid sequence that is at least 90%, at least 95%, or at least 98% identical to the amino acid sequence found at GenBank^{™} accession number AAH46208.1. In a specific embodiment, provided herein is a recombinant protein comprising the amino acid sequence found at GenBank^{™} accession number AAH17969.1 with 1, 2, 3, 4, 5, 6, 7, 8, 9 or 10 amino acid substitutions. In a specific embodiment, provided herein is a recombinant protein comprising an amino acid sequence encoded by the nucleotide sequence of SEQ ID NO:9. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:9. In some embodiments, provided herein is a recombinant protein comprising an amino acid sequence encoded by a nucleotide sequence at least 90%, at least 95%, or at least 98% identical to SEQ ID NO:9. In a specific embodiment, provided herein is a recombinant protein comprising an amino acid sequence encoded by the nucleotide sequence of SEQ ID NO:9 with 1, 2, 3, 4, 5, 6, 7, 8, 9 or 10 nucleotide substitutions. In particular embodiments, the IFIH1 protein retains one, two, or more, or all of the functions of IFIH1 protein (e.g., human IFIH1 protein). For example, the IFIH1 protein may retain its role in sensing viral infection and in the activation of a cascade of antiviral responses including the induction of type I interferons and proinflammatory cytokines.

In a specific embodiment, provided herein is a recombinant protein comprising the amino acid sequence for MX1 (e.g., human MX1). The amino acid sequence for MX1 may comprise the amino acid sequence found at GenBank^{™} accession number CAB90556.1. In certain embodiments, provided herein is a recombinant protein comprising an amino acid sequence that is at least 75%, at least 80%, at least 85%, at least 90%, at least 95%, or at least 98% identical to found at GenBank^{™} accession number CAB90556.1. In some embodiments, provided herein is a recombinant protein comprising an amino acid sequence that is at least 90%, at least 95%, or at least 98% identical to found at GenBank^{™} accession number CAB90556.1. In a specific embodiment, provided herein is a recombinant protein comprising an amino acid sequence encoded by the nucleotide sequence of SEQ ID NO:10. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO: 10. In some embodiments, provided herein is a recombinant protein comprising an amino acid sequence encoded by a nucleotide sequence at least 90%, at least 95%, or at least 98% identical to SEQ ID NO:10. In a specific embodiment, provided herein is a recombinant protein comprising an amino acid sequence encoded by the nucleotide sequence of SEQ ID NO:10 with 1, 2, 3, 4, 5, 6, 7, 8, 9 or 10 nucleotide substitutions. In particular embodiments, the MX1 protein retains one, two, or more, or all of the functions of MX1 protein (e.g., human MX1 protein). For example, the MX1 protein may retain its antiviral activity.

In a specific embodiment, provided herein is a recombinant protein comprising the amino acid sequence for IFI30 (e.g., human IFI30). The amino acid sequence for IFI30 may comprise the amino acid sequence found at GenBank^{™} accession number CAB90556.1. In certain embodiments, provided herein is a recombinant protein comprising an amino acid sequence that is at least 75%, at least 80%, at least 85%, at least 90%, at least 95%, or at least 98% identical to found at GenBank^{™} accession number CAB90556.1. In some embodiments, provided herein is a recombinant protein comprising an amino acid sequence that is at least 90%, at least 95%, or at least 98% identical to found at GenBank^{™} accession number CAB90556.1. In a specific embodiment, provided herein is a recombinant protein comprising an amino acid sequence encoded by the nucleotide sequence of SEQ ID NO:11. In certain embodiments, provided herein is a recombinant protein comprising an amino acid sequence encoded by a nucleotide sequence at least 75%, at least 80%, at least 85%, at least 90%, at least 95%, or at least 98% identical to SEQ ID NO:11. In some embodiments, provided herein is a recombinant protein comprising an amino acid sequence encoded by a nucleotide sequence at least 90%, at least 95%, or at least 98% identical to SEQ ID NO:11. In a specific embodiment, provided herein is a recombinant protein comprising an amino acid sequence encoded by the nucleotide sequence of SEQ ID NO:11 with 1, 2, 3, 4, 5, 6, 7, 8, 9 or 10 nucleotide substitutions. In particular embodiments, the IFI30 protein retains one, two, or more, or all of the functions of IFI30 protein (e.g., human IFI30 protein). For example, the IFI30 protein may retain its ability to facilitate the complete unfolding of proteins destined for lysosomal degradation or its role in antigen processing.

In specific embodiments, a protein described herein is modified by post-translational processing such as signal peptide cleavage, disulfide bond formation, glycosylation (e.g., N-linked glycosylation), protease cleavage and lipid modification (e.g., S-palmitoylation). In some embodiments, a protein described herein includes a signal sequence. In some embodiments, a protein described herein is the immature form. In other embodiments, a protein described herein is the mature form. A nucleic acid or nucleotide sequence may encode an immature or mature form of a protein described herein. In certain embodiments, a protein described herein is an isoform. A nucleic acid or nucleotide sequence may encode an isoform of a protein described herein.

In certain embodiments, an "isolated" nucleic acid sequence or nucleotide sequence refers to a nucleic acid molecule which is separated from other nucleic acid molecules which are present in the natural source of the nucleic acid. In other words, the isolated nucleic acid sequence can comprise heterologous nucleic acids that are not associated with it in nature. In other embodiments, an "isolated" nucleic acid sequence, such as a cDNA or RNA sequence, can be substantially free of other cellular material, or culture medium when produced by recombinant techniques, or substantially free of chemical precursors or other chemicals when chemically synthesized. The term "substantially free of cellular material" includes preparations of nucleic acid sequences in which the nucleic acid sequence is separated from cellular components of the cells from which it is isolated or recombinantly produced. Thus, in certain embodiments, a nucleic acid sequence that is substantially free of cellular material includes preparations of nucleic acid sequence having less than about 30%, 20%, 10%, or 5% (by dry weight) of other nucleic acids. The term "substantially free of culture medium," in certain embodiments, includes preparations of nucleic acid sequence in which the culture medium represents less than about 50%, 20%, 10%, or 5% of the volume of the preparation. In a specific embodiment, a nucleic acid sequence or nucleotide sequence described herein is isolated.

As used herein, the terms "nucleic acid" and "nucleotide" are intended to include DNA molecules (*e*.*g*., cDNA or genomic DNA) and RNA molecules (*e*.*g*., mRNA) and analogs of the DNA or RNA generated using nucleotide analogs. A nucleic acid sequence or nucleotide sequence can be single-stranded or double-stranded. In one embodiment, a nucleic acid sequence or a nucleotide sequence is a DNA sequence. In another embodiment, a nucleic acid sequence or a nucleotide sequence is an RNA sequence (*e*.*g*., mRNA sequence or modRNA). In certain embodiments, a nucleic acid sequence or nucleotide sequence is a mRNA sequence comprising non-standard, naturally modified nucleosides, synthetic nucleoside analogs, or a combination thereof. For example, mRNA nucleosides may be replaced by non-standard, naturally modified nucleosides in eukaryotes (*e*.*g*., pseudouridine, 5-methylcytosine, 6-methyladenosine), or by synthetic nucleoside analogs, such as N1-methylpseudouridine.

Modified RNA, or modRNA, is a synthetic modified RNA that can be used for expression of a gene of interest. Chemical modifications to a ribonucleotide included in modRNA may stabilize an RNA molecule, blunt an immune response, or enhance transcription. Additionally, unlike delivery of protein agents directly to a cell, which can activate the immune system, the delivery of modRNA can be achieved without immune impact. For example, substitution of uridine and cytidine with pseudouridine or N1-methylpseudouridine and 5-methylcytidine, respectively, drastically reduces the immune response elicited from exogenous RNA without such substitutions. Stability and translational efficiency from an RNA molecule may also be increased by including a 3'-O-Me-m7G(5')ppp(5')G Anti Reverse Cap Analog (ARCA) at the 5' end of the RNA molecule.

modRNA may encompass an RNA molecule with at least uridine substituted with pseudouridine. modRNA may encompass an RNA molecule with at least cytidine substituted with 5-methylcytidine. modRNA may encompass an RNA molecule including the modified nucleoside 5-methylcytidine (5mC). modRNA may encompass an RNA molecule including the modified nucleoside 2-Thiouridine-5'-Triphosphate (2-thio ψU). modRNA may encompass an RNA molecule with at least the modified nucleoside1-Methylpseudouridine-5'-Triphosphate (1-mψU). modRNA may encompass an RNA molecule with at least the modified nucleoside N1-methyl-pseudouridine (N1mΨ) substituted for uridine. modRNA may encompass an RNA molecule wherein at least 5' triphosphates are removed. modRNA may encompass an RNA molecule wherein at least a 3'-O-Me-m7G(5')ppp(5')G Anti Reverse Cap Analog (ARCA) cap or C₃₂H₄₃N₁₅O₂₄P₄ CleanCap Reagent AG is included in a 5' untranslated regions of the RNA molecule.

modRNAs may be prepared by in vitro transcription. modRNA may be in vitro transcribed, e.g., from a linear DNA template using one or more reagents selected from a cap analog, guanosine triphosphate, adenosine triphosphate, cytidine triphosphate, uridine triphosphate, and derivatives thereof. A cap analog may be selected from Anti-Reverse Cap Analog (ARCA) 3'-O-Me-m7G(5')ppp(5')G, standard cap analog m7G(5')ppp(5')G, unmethylated cap analog G(5')ppp(5')G, methylated cap analog for A+1 sites m7G(5')ppp(5')A, and unmethylated cap analog for A+1 sites G(5')ppp(5')A. In certain examples, a cap analog is Anti-Reverse Cap Analog (ARCA) 3'-O-Me-m7G(5')ppp(5')G. According to some examples, modRNA may be in vitro transcribed from a plasmid template using one or more reagents selected from 3'-O-Me-m7G(5')ppp(5')G, guanosine triphosphate, adenosine triphosphate, cytidine triphosphate, N1-methylpseudouridine-5-triphosphate, and any one or more of the aforementioned examples of modRNA, or others, without limitation and in any combination.

Additional suitable modifications to a modRNA or mRNA molecule are well known in the art (*see*, *e*.*g*., U.S. Patent No. 8,278,036 to Kariko et al.; U.S. Patent No. 10,086,043 to Chien et al.; U.S. Patent Application Publication No. 2019/0203226 to Zangi et al.; and U.S. Patent Application Publication No. 2018/0353618 to Burkhardt et al.). In some embodiments, the nucleoside that is modified in the modRNA is a uridine (U), a cytidine (C), an adenine (A), or guanine (G). The modified nucleoside can be, for example, m⁵C (5-methylcytidine), m⁶A (N⁶-methyladenosine), s²U (2-thiouridien), ψ (pseudouridine), or Um (2-O-methyluridine). Some exemplary chemical modifications of nucleosides in the modRNA molecule may further include, for example and without limitation, pyridine-4-one ribonucleoside, 5-aza-uridine, 2-thio-5-aza uridine, 2-thiouridine, 4-thio pseudouridine, 2-thio pseudouridine, 5-hydroxyuridine, 3-methyluridine, 5-carboxymethyl uridine, 1-carboxymethyl pseudouridine, 5-propynyl uridine, 1-propynyl pseudouridine, 5-taurinomethyluridine, 1-taurinomethyl pseudouridine, 5-taurinomethyl-2-thio uridine, 1-taurinomethyl-4-thio uridine, 5-methyl uridine, 1-methyl pseudouridine, 4-thio-1-methyl pseudouridine, 2-thio-1-methyl pseudouridine, 1-methyl-1-deaza pseudouridine, 2-thio-1-methyl-1-deaza pseudouridine, dihydrouridine, dihydropseudouridine, 2-thio dihydrouridine, 2-thio dihydropseudouridine, 2-methoxyuridine, 2-methoxy-4-thio uridine, 4-methoxy pseudouridine, 4-methoxy-2-thio pseudouridine, 5-aza cytidine, pseudoisocytidine, 3-methyl cytidine, N4-acetylcytidine, 5-formylcytidine, N4-methylcytidine, 5-hydroxymethylcytidine, 1-methyl pseudoisocytidine, pyrrolo-cytidine, pyrrolo-pseudoisocytidine, 2-thio cytidine, 2-thio-5-methyl cytidine, 4-thio pseudoisocytidine, 4-thio-1-methyl pseudoisocytidine, 4-thio-1-methyl-1-deaza pseudoisocytidine, 1-methyl-1-deaza pseudoisocytidine, zebularine, 5-aza zebularine, 5-methyl zebularine, 5-aza-2-thio zebularine, 2-thio zebularine, 2-methoxy cytidine, 2-methoxy-5-methyl cytidine, 4-methoxy pseudoisocytidine, 4-methoxy-1-methyl pseudoisocytidine, 2-aminopurine, 2,6-diaminopurine, 7-deaza adenine, 7-deaza-8-aza adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N⁶-methyladenosine, N⁶-isopentenyladenosine, N⁶-(cis-hydroxyisopentenyl) adenosine, 2-methylthio-N⁶-(cis-hydroxyisopentenyl) adenosine, N⁶-glycinylcarbamoyladenosine, N⁶-threonylcarbamoyladenosine, 2-methylthio-N⁶-threonyl carbamoyladenosine, N⁶,N⁶-dimethyladenosine, 7-methyladenine, 2-methylthio adenine, 2-methoxy adenine, inosine, 1-methyl inosine, wyosine, wybutosine, 7-deaza guanosine, 7-deaza-8-aza guanosine, 6-thio guanosine, 6-thio-7-deaza guanosine, 6-thio-7-deaza-8-aza guanosine, 7-methyl guanosine, 6-thio-7-methyl guanosine, 7-methylinosine, 6-methoxy guanosine, 1-methylguanosine, N²-methylguanosine, N²,N²-dimethylguanosine, 8-oxo guanosine, 7-methyl-8-oxo guanosine, 1-methyl-6-thio guanosine, N²-methyl-6-thio guanosine, or N²,N²-dimethyl-6-thio guanosine.

In an example, modifications made to the modRNA are independently selected from 5-methylcytosine, pseudouridine, and 1-methylpseudouridine.

In some embodiments, the modRNA comprises a modified uracil selected from the group consisting of pseudouridine (ψ), pyridine-4-one ribonucleoside, 5-aza uridine, 6-aza uridine, 2-thio-5-aza uridine, 2-thio uridine (s2U), 4-thio uridine (s4U), 4-thio pseudouridine, 2-thio pseudouridine, 5-hydroxy uridine (ho⁵U), 5-aminoallyl uridine, 5-halo uridine (e.g., 5-iodom uridine or 5-bromo uridine), 3-methyl uridine (m³U), 5-methoxy uridine (mo⁵U), uridine 5-oxyacetic acid (cmo⁵U), uridine 5-oxyacetic acid methyl ester (mcmo⁵U), 5-carboxymethyl uridine (cm⁵U), 1-carboxymethyl pseudouridine, 5-carboxyhydroxymethyl uridine (chm⁵U), 5-carboxyhydroxym ethyl uridine methyl ester (mchm⁵U), 5-methoxycarbonylmethyl uridine (mcm⁵U), 5-methoxycarbonylmethyl-2-thio uridine (mcm⁵s2U), 5-aminomethyl-2-thio uridine (nm⁵s2U), 5-methylaminomethyl uridine (mnm⁵U), 5-methylaminomethyl-2-thio uridine (mnm⁵s2U), 5-methylaminomethyl-2-seleno uridine (mnm⁵se²U), 5-carbamoylmethyl uridine (ncm⁵U), 5-carboxymethylaminomethyl uridine (cmnm⁵U), 5-carboxymethylaminomethyl-2-thio uridine (cmnm⁵s2U), 5-propynyl uridine, 1-propynyl pseudouridine, 5-taurinomethyl uridine (τcm⁵U), 1-taurinomethyl pseudouridine, 5-taurinomethyl-2-thio uridine (^{™5}s2U), 1-taurinomethyl-4-thio pseudouridine, 5-methyl uridine (m⁵U, e.g., having the nucleobase deoxythymine), 1-methyl pseudouridine (m¹ψ), 5-methyl-2-thio uridine (m⁵s2U), 1-methyl-4-thio pseudouridine (m₁s⁴ψ), 4-thio-1-methyl pseudouridine, 3-methyl pseudouridine (m³ψ), 2-thio-1-methyl pseudouridine, 1-methyl-1-deaza pseudouridine, 2-thio-1-methyl-1-deaza pseudouridine, dihydrouridine (D), dihydropseudouridine, 5,6-dihydrouridine, 5-methyl dihydrouridine (m⁵D), 2-thio dihydrouridine, 2-thio dihydropseudouridine, 2-methoxy uridine, 2-methoxy-4-thio uridine, 4-methoxy pseudouridine, 4-methoxy-2-thio pseudouridine, N¹-methyl pseudouridine, 3-(3-amino-3-carboxypropyl) uridine (acp³U), 1-methyl-3-(3-amino-3-carboxypropyl) pseudouridine (acp³ψ), 5-(isopentenylaminomethyl) uridine (inm⁵U), 5-(isopentenylaminomethyl)-2-thio uridine (inm⁵s2U), α-thio uridine, 2'-O-methyl uridine (Um), 5,2'-O-dimethyl uridine (m⁵Um), 2'-O-methyl pseudouridine (ψm), 2-thio-2'-O-methyl uridine (s2Um), 5-methoxycarbonylmethyl-2'-O-methyl uridine (mcm⁵Um), 5-carbamoylmethyl-2'-O-methyl uridine (ncm⁵Um), 5-carboxymethylaminomethyl-2'-O-methyl uridine (cmnm⁵Um), 3,2'-O-dimethyl uridine (m³Um), 5-(isopentenylaminomethyl)-2'-O-methyl uridine (inm⁵Um), 1-thio uridine, deoxythymidine, 2'-F-ara uridine, 2'-F uridine, 2'-OH-ara uridine, 5-(2-carbomethoxyvinyl) uridine, and 5-3-(1-E-propenylamino) uridine.

In some embodiments, the modRNA comprises a modified cytosine selected from the group consisting of 5-aza cytidine, 6-aza cytidine, pseudoisocytidine, 3-methyl cytidine (m³C), N⁴-acetyl cytidine (act), 5-formyl cytidine (f⁵C), N⁴-methyl cytidine (m⁴C), 5-methyl cytidine (m⁵C), 5-halo cytidine (*e*.*g*., 5-iodo cytidine), 5-hydroxymethyl cytidine (hm⁵C), 1-methyl pseudoisocytidine, pyrrolo-cytidine, pyrrolo-pseudoisocytidine, 2-thio cytidine (s2C), 2-thio-5-methyl cytidine, 4-thio pseudoisocytidine, 4-thio-1-methyl pseudoisocytidine, 4-thio-1-methyl-1-deaza pseudoisocytidine, 1-methyl-1-deaza pseudoisocytidine, zebularine, 5-aza zebularine, 5-methyl zebularine, 5-aza-2-thio zebularine, 2-thio zebularine, 2-methoxy cytidine, 2-methoxy-5-methyl cytidine, 4-methoxy pseudoisocytidine, 4-methoxy-1-methyl pseudoisocytidine, lysidine (k²C), alpha-thio cytidine, 2'-O-methyl cytidine (Cm), 5,2'-O-dimethyl cytidine (m⁵Cm), N⁴-acetyl-2'-O-methyl cytidine (ac⁴Cm), N⁴,2'-O-dimethyl cytidine (m⁴Cm), 5-formyl-2'-O-methyl cytidine (f⁵Cm), N⁴,N⁴,2'-O-trimethyl cytidine (m⁴₂Cm), 1-thio cytidine, 2'-F-ara cytidine, 2'-F cytidine, and 2'-OH-ara cytidine.

In some embodiments, the modRNA comprises a modified adenine selected from the group consisting of 2-amino purine, 2,6-diamino purine, 2-amino-6-halo purine (e.g., 2-amino-6-chloro purine), 6-halo purine (*e*.*g*., 6-chloro purine), 2-amino-6-methyl purine, 8-azido adenosine, 7-deaza adenine, 7-deaza-8-aza adenine, 7-deaza-2-amino purine, 7-deaza-8-aza-2-amino purine, 7-deaza-2,6-diamino purine, 7-deaza-8-aza-2,6-diamino purine, 1-methyl adenosine (m¹A), 2-methyl adenine (m²A), N⁶-methyl adenosine (m⁶A), 2-methylthio-N⁶-methyl adenosine (ms²m⁶A), N⁶-isopentenyl adenosine (i⁶A), 2-methylthio-N⁶-isopentenyl adenosine (ms²i⁶A), N⁶-(cis-hydroxyisopentenyl) adenosine (io⁶A), 2-methylthio-N⁶-(cis-hydroxyisopentenyl) adenosine (ms²io⁶A), N⁶-glycinylcarbamoyl adenosine (g⁶A), N⁶-threonylcarbamoyl adenosine (t⁶A), N⁶-methyl-N⁶-threonylcarbamoyl adenosine (m⁶t⁶A), 2-methylthio-N⁶-threonylcarbamoyl adenosine (ms²g⁶A), N⁶,N⁶-dimethyl adenosine (m⁶₂A), N⁶-hydroxynorvalyIcarbamoyl adenosine (hn⁶A), 2-methylthio-N⁶-hydroxynorvalylcarbamoyl adenosine (ms²hn⁶A), N⁶-acetyl adenosine (ac⁶A), 7-methyl adenine, 2-methylthio adenine, 2-methoxy adenine, alpha-thio adenosine, 2'-O-methyl adenosine (Am), N⁶,2'-O-dimethyl adenosine (m⁶Am) N⁶,N⁶,2'-O-trimethyl adenosine (m⁶₂Am), 1,2'-O-dimethyl adenosine (m¹Am), 2'-O-ribosyl adenosine (phosphate) (Ar(p)), 2-amino-N⁶-methyl purine, 1-thio adenosine, 8-azido adenosine, 2'-F-ara adenosine, 2'-F adenosine, 2'-OH-ara adenosine, and N⁶-(19-amino-pentaoxanonadecyl) adenosine.

In some embodiments, the modRNA comprises a modified guanine selected from the group consisting of inosine (I), 1-methyl inosine (m¹I), wyosine (imG), methylwyosine (mimG), 4-demethyl wyosine (imG-14), isowyosine (imG2), wybutosine (yW), peroxywybutosine (o₂yW), hydroxywybutosine (OHyW), undermodified hydroxywybutosine (OHyWy), 7-deaza guanosine, queuosine (Q), epoxyqueuosine (oQ), galactosyl queuosine (galQ), mannosyl queuosine (manQ), 7-cyano-7-deaza guanosine (preQ₀), 7-aminomethyl-7-deaza guanosine (preQ₁), archaeosine (G⁺), 7-deaza-8-aza guanosine, 6-thio guanosine, 6-thio-7-deaza guanosine, 6-thio-7-deaza-8-aza guanosine, 7-methyl guanosine (m⁷G), 6-thio-7-methyl guanosine, 7-methyl inosine, 6-methoxy guanosine, 1-methyl guanosine (m¹G), N²-methyl-guanosine (m²G), N²,N²-dimethyl guanosine (m²₂G), N^{2,7}-dimethyl guanosine (m^{2,7}G), N², N^{2,7}-dimethyl guanosine (m^{2,2,7}G), 8-oxo guanosine, 7-methyl-8-oxo guanosine, 1-methio guanosine, N²-methyl-6-thio guanosine, N²,N²-dimethyl-6-thio guanosine, alpha-thio guanosine, 2'-O-methyl guanosine (Gm), N²-methyl-2'-O-methyl guanosine (m²Gm), N²,N²-dimethyl-2'-O-methyl guanosine (m²₂Gm), 1-methyl-2'-O-methyl guanosine (m¹Gm), N^{2,7}-dimethyl-2'-O-methyl guanosine (m^{2,7}Gm), 2'-O-methyl inosine (1m), 1,2'-O-dimethyl inosine (m¹Im), 2'-O-ribosyl guanosine (phosphate) (Gr(p)), 1-thio guanosine, O⁶-methyl guanosine, 2'-F-ara guanosine, and 2'-F guanosine.

modRNA may include, for example, a non-natural or modified nucleotide. The non-natural or modified nucleotide may include, for example, a backbone modification, sugar modification, or base modification. The non-natural or modified nucleotide may include, for example, a base modification. In some embodiments, the base modification is selected from the group consisting of 2-amino-6-chloropurine riboside 5' triphosphate, 2-aminoadenosine 5' triphosphate, 2-thiocytidine 5' triphosphate, 2-thiouridine 5' triphosphate, 4-thiouridine 5' triphosphate, 5-aminoallylcytidine 5' triphosphate, 5-aminoallyluridine 5' triphosphate, 5-bromocytidine 5' triphosphate, 5-bromouridine 5' triphosphate, 5-iodocytidine 5' triphosphate, 5-iodouridine 5' triphosphate, 5-methylcytidine 5' triphosphate, 5-methyluridine 5' triphosphate, 6-azacytidine 5' triphosphate, 6-azauridine 5' triphosphate, 6-chloropurine riboside 5'-triphosphate, 7-deazaadenosine 5' triphosphate, 7-deazaguanosine 5' triphosphate, 8-azaadenosine 5' triphosphate, 8-azidoadenosine 5' triphosphate, benzimidazole riboside 5' triphosphate, N¹-methyladenosine 5' triphosphate, N¹-methylguanosine 5' triphosphate, N⁶-methyladenosine 5' triphosphate, O⁶-methylguanosine 5' triphosphate, N¹-methyl-pseudouridine 5' triphosphate, puromycin 5'-triphosphate, and xanthosine 5' triphosphate. Thus, according to some embodiments, the modRNA comprises N¹-methyl-pseudouridine 5' triphosphate.

In another aspect, provided herein are vectors (e.g., plasmids or viral vectors) comprising a nucleic acid sequence(s) comprising a nucleotide sequence(s) encoding a recombinant protein(s) described herein. In a specific embodiment, provided herein is a mammalian vector comprising a nucleotide sequence(s) encoding a recombinant protein(s) described herein. In another specific embodiment, provided herein is a baculovirus vector comprising a nucleotide sequence(s) encoding a recombinant protein(s) described herein. In another specific embodiment, provided herein is a vector comprising a nucleotide sequence(s) described herein. In another specific embodiment, provided herein is a viral vector comprising a nucleotide sequence(s) described herein. *See* Section 5 or 6, *infra*, for an example of a recombinant viral vector.

Techniques known to one of skill in the art may be used to produce a protein described herein. For example, standard methods in molecular biology are described Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA). Standard methods also appear in Ausbel, et al. (2001) Current Protocols in Molecular Biology, Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4). In a specific embodiment, a protein described herein is recombinantly produced.

Methods for protein purification including immunoprecipitation, chromatography, electrophoresis, centrifugation, and crystallization are described in the art (e.g., Coligan, et al. (2000) Current Protocols in Protein Science, Vol. 1, John Wiley and Sons, Inc., New York). Chemical analysis, chemical modification, post-translational modification, production of fusion proteins, glycosylation of proteins are described in the art (see, *e*.*g*., Coligan, et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391). In a specific embodiment, a protein described herein is isolated.

In a specific embodiment, a protein is isolated when substantially free of contaminating materials from the natural source, *e.g.*, soil particles, minerals, chemicals from the environment, and/or cellular materials from the natural source, such as but not limited to cell debris, cell wall materials, membranes, organelles, the bulk of the nucleic acids, carbohydrates, proteins, and/or lipids present in cells. In a specific embodiment, a protein that is isolated includes preparations of a polypeptide having less than about 30%, 20%, 10%, 5%, 2%, or 1% (by dry weight) of cellular materials and/or contaminating materials. In some embodiments, a chemically synthesized polypeptide is isolated when substantially free of chemical precursors or other chemicals which are involved in the syntheses of the polypeptide. The term "substantially free of chemical precursors or other chemicals" includes, in certain embodiments, preparations in which the amino acid sequence is separated from chemical precursors or other chemicals which are involved in the synthesis of the amino acid sequence. In specific embodiments, such preparations of the amino acid sequence have less than about 50%, 30%, 20%, 10%, 5% (by dry weight) of chemical precursors or compounds other than the amino acid sequence of interest.

### 4.2 VIRAL VECTORS

In one aspect, provided herein is a recombinant virus comprising a genome, wherein the genome comprises a transgene(s) comprising a nucleotide sequence(s) encoding all of the following: MX2, IFIT3, IFITM1, IFI27, IFIT1, BST2, IFI6, RSAD2, MX1, and IFI30. In some embodiments, provided herein is a recombinant virus comprising a genome, wherein the genome comprises a polynucleotide(s) encoding all of the following: MX2, IFIT3, IFITM1, IFI27, IFIT1, BST2, IFI6, RSAD2, IFIH1, MX1, and IFI30. In some embodiments, provided herein is a recombinant virus comprising a genome, wherein the genome comprises a polynucleotide(s) encoding all of the following: MX2, IFIT3, IFITM1, IFI27, IFIT1, BST2, IFI6, RSAD2, MX1, and IFI30. In some embodiment, a single nucleotide sequence comprises nucleic acid sequences encoding the proteins and the nucleotide sequence includes nucleic acid sequence encoding cleavage sites (e.g., a 2A cleavage site) which separates to the proteins.

In some embodiments, provided herein is a recombinant virus comprising a genome, wherein the genome comprises a transgene, wherein the transgene comprises a nucleotide sequence(s) encoding all of the following: (i) MX2, (ii) IFIT3, (iii) IFITM1, (iv) IFI27, (v) IFIT1, (vi) BST2, (vii) IFI6, (viii) RSAD2, (ix) IFIH1, (x) MX1, and (xi) IFI30. In some embodiments, a single nucleotide sequence comprises nucleic acid sequences encoding all of the proteins (i) to (xi) and the nucleotide sequence encodes a cleavage site(s) (e.g., a 2A cleavage site) which separates to the two or more proteins.

In some embodiments, provided herein is a recombinant virus comprising a genome, wherein the genome comprises two transgenes, wherein one transgene comprises a nucleotide sequence(s) encoding 1, 2, 3, 4, 5, or more of all the following: (i) MX2, (ii) IFIT3, (iii) IFITM1, (iv) IFI27, (v) IFIT1, (vi) BST2, (vii) IFI6, (viii) RSAD2, (ix) MX1, and (x) IFI30, and the other transgene comprises a nucleotide sequence(s) encoding 1, 2, 3, 4, 5, or more of all (i) to (x). In certain embodiments, a nucleotide sequence encoding a cleavage site (e.g. a 2A cleavage site) is included in a transgene encoding a single polypeptide, which may be cleaved to produce two or more of the proteins described herein. In a specific embodiment, both transgenes do not encode the same proteins of (i) to (x).

In certain embodiments, provided herein is a recombinant virus comprising a genome, wherein the genome comprises three transgenes, wherein one transgene comprises a nucleotide sequence(s) encoding 1, 2, 3, 4, 5, or more of all the following: (i) MX2, (ii) IFIT3, (iii) IFITM1, (iv) IFI27, (v) IFIT1, (vi) BST2, (vii) IFI6, (viii) RSAD2, (ix) MX1, and (x) IFI30, a second transgene comprises a nucleotide sequence(s) encoding 1, 2, 3, 4, 5, or more of all (i) to (x), and the third transgene comprises a nucleotide sequence(s) encoding 1, 2, 3, 4, 5, or more of all (i) to (x). In a specific embodiment, the three transgenes do not encode the same proteins of (i) to (x).

In certain embodiments, provided herein is a recombinant virus comprising a genome, wherein the genome comprises four transgenes, wherein one transgene comprises a nucleotide sequence(s) encoding 1, 2, 3, 4, 5, or more of all the following: (i) MX2, (ii) IFIT3, (iii) IFITM1, (iv) IFI27, (v) IFIT1, (vi) BST2, (vii) IFI6, (viii) RSAD2, (ix) MX1, and (x) IFI30, a second transgene comprises a nucleotide sequence(s) encoding 1, 2, 3, 4, 5, or more of all (i) to (x), the third transgene comprises a nucleotide sequence(s) encoding 1, 2, 3, 4, 5, or more of all (i) to (x), and the fourth transgene comprises a nucleotide sequence(s) encoding 1, 2, 3, 4, 5, or more of all (i) to (x). In a specific embodiment, the four transgenes do not encode the same proteins of (i) to (x).

In certain embodiments, provided herein is a recombinant virus comprising a genome, wherein the genome comprises five transgenes, wherein one transgene comprises a nucleotide sequence(s) encoding 1, 2, 3, 4, 5, or more of all the following: (i) MX2, (ii) IFIT3, (iii) IFITM1, (iv) IFI27, (v) IFIT1, (vi) BST2, (vii) IFI6, (viii) RSAD2, (ix) MX1, and (x) IFI30, a second transgene comprises a nucleotide sequence(s) encoding 1, 2, 3, 4, 5, or more of all (i) to (x), the third transgene comprises a nucleotide sequence(s) encoding 1, 2, 3, 4, 5, or more of all (i) to (x), the fourth transgene comprises a nucleotide sequence(s) encoding 1, 2, 3, 4, 5, or more of all (i) to (x), and the fifth transgene comprises a nucleotide sequence(s) encoding 1, 2, 3, 4, 5, or more of all (i) to (x). In a specific embodiment, the five transgenes do not encode the same proteins of (i) to (x).

In some embodiments, the virus that is a genetically engineered is an influenza virus, measles virus, poliovirus, avian paramyxovirus (*e*.*g*., Newcastle disease virus or other avian paramyxovirus serotype), vaccinia virus, poxvirus, picornavirus, alphavirus, retrovirus, rhabdovirus, reovirus, adenovirus, lentivirus, adeno-associated virus (AAV; such as, *e*.*g*., AAV1-AAV9), herpes simplex virus, or vesicular stomatitis virus. In a specific embodiment, such viruses are attenuated. In one embodiment, the virus that is a genetically engineered is an attenuated adenovirus. In specific embodiments, the virus is attenuated such that it only undergoes a limited number of rounds of replication (*e*.*g*., 1, 2, or 3 rounds of replication) *in vitro* or in a subject (*e*.*g*., a human subject). In specific embodiments, the virus is attenuated such that it only undergoes a limited number of rounds of replication (*e*.*g*., 1, 2, or 3 rounds of replication) in a subject (*e*.*g*., a human subject) and does not cause symptoms of a disease associated with the virus in the subject. Techniques known to one of skill in the art or described herein may be used to genetically engineer a recombinant virus described herein. Methods for engineering a recombinant virus to encode a transgene and express a heterologous protein encoded by the transgene are known to one skilled in the art, such as, *e*.*g*., insertion of the transgene into a restriction site that has been engineered into the viral genome, inclusion appropriate signals in the transgene for recognition by the virus, and inclusion of a valid Kozak sequence (*e*.*g*., to improve eukaryotic ribosomal translation).

In specific embodiments, a polynucleotide encoding a protein described herein (*e*.*g*., MX2, IFIT3, IFITM1, IFI27, IFIT1, BST2, IFI6, RSAD2, IFIH1, MX1, or IFI30) is codon optimized. Any codon optimization technique known to one of skill in the art may be used to codon optimize a nucleic acid sequence encoding a protein described herein. Methods of codon optimization are known in the art, *e*.*g*, the OptimumGene^{™} (GenScript^{®}) protocol and Genewiz^{®} protocol. *See* also U.S. Patent No. 8,326,547 for methods for codon optimization.

As an exemplary method for codon optimization, each codon in the open frame of the nucleic acid sequence encoding a protein described herein is replaced by the codon most frequently used in mammalian proteins. This may be done using a web-based program (www.encorbio.com/protocols/Codon.htm) that uses the Codon Usage Database, maintained by the Department of Plant Gene Research in Kazusa, Japan. This nucleic acid sequence optimized for mammalian expression may be inspected for: (1) the presence of stretches of 5xA or more that may act as transcription terminators; (2) the presence of restriction sites that may interfere with subcloning; and (3) compliance with the rule of six for viruses that follow the rule of six. Following inspection, (1) stretches of 5xA or more that may act as transcription terminators may be replaced by synonymous mutations; (2) restriction sites that may interfere with subcloning may be replaced by synonymous mutations; (3) a virus's regulatory signals, such as, e.g., gene end, intergenic and gene start sequences, and Kozak sequences for optimal protein expression may be added; and (4) nucleotides may be added in the non-coding region to ensure compliance with the rule of six for viruses, that follow the rule of six. Synonymous mutations are typically nucleotide changes that do not change the amino acid encoded. For example, in the case of a stretch of 6 As (AAAAAA), which sequence encodes Lys-Lys, a synonymous sequence would be AAGAAG, which sequence also encodes Lys-Lys.

### 4.3 PROPAGATION OF VIRUSES

A recombinant virus described herein can be propagated in any substrate that allows the virus to grow to titers that permit the uses of the viruses described herein. A recombinant virus described herein may be propagated in cells (*e*.*g*., avian cells, chicken cells, etc.) that are susceptible to infection by the viruses, embryonated eggs (*e.*g., chicken eggs or quail eggs) or animals (*e*.*g*., birds). Such methods are well-known to those skilled in the art. For example, a recombinant virus described herein may be propagated in a cell line, *e*.*g*., cancer cell lines such as HeLa cells, MCF7 cells, B16-F10 cells, CT26 cells, TC-1 cells, THP-1 cells, U87 cells, DU145 cells, Lncap cells, and T47D cells. In certain embodiments, the cells or cell lines (*e*.*g*., cancer cells or cancer cell lines) are obtained and/or derived from a human(s). In another embodiment, a recombinant virus described herein is propagated in chicken cells or embryonated eggs. Representative chicken cells include, but are not limited to, chicken embryo fibroblasts and chicken embryo kidney cells. In a specific embodiment, a recombinant virus described is propagated in IFN-deficient cells (*e*.*g*., IFN-deficient cell lines). In a specific embodiment, a recombinant virus described herein is propagated as described in Section 5 or 6, infra.

A recombinant virus described herein may be propagated in embryonated eggs, *e*.*g*., from 6 to 14 days old, 6 to 12 days old, 6 to 10 days old, 6 to 9 days old, 6 to 8 days old, 8 days old, 9 days old, 10 days old, 8 to 10 days old, 12 days old, or 10 to 12 days old. Young or immature embryonated eggs can be used to propagate a recombinant virus described herein,. Immature embryonated eggs encompass eggs which are less than ten day old eggs, *e.g.*, eggs 6 to 9 days old or 6 to 8 days old that are IFN-deficient. Immature embryonated eggs also encompass eggs which artificially mimic immature eggs up to, but less than ten day old, as a result of alterations to the growth conditions, *e*.*g*., changes in incubation temperatures; treating with drugs; or any other alteration which results in an egg with a retarded development, such that the IFN system is not fully developed as compared with ten to twelve day old eggs. In another specific embodiment, a recombinant virus described herein may be propagated in 10 day old embryonated chicken eggs. For a detailed discussion on the growth and propagation viruses, *see*, *e*.*g*., U.S. Patent No. 6,852,522 and U.S. Patent No. 7,494,808.

In a specific embodiment, provided herein is a method for propagation of a recombinant virus described herein, the method comprising culturing a substrate (*e*.*g*., a cell line or embryonated egg) infected with the virus. In another specific embodiment, provided herein is a method for propagating a recombinant virus described herein, the method comprising: (a) culturing a substrate (*e*.*g*., a cell line or embryonated egg) infected with the virus; and (b) isolating or purifying the virus from the substrate. In certain embodiments, these methods involve infecting the substrate with the recombinant virus prior to culturing the substrate.

For virus isolation, a recombinant virus described herein can be removed from embryonated eggs or cell culture and separated from cellular components, typically by well-known clarification procedures, *e*.*g*., such as centrifugation, depth filtration, and microfiltration, and may be further purified as desired using procedures well known to those skilled in the art, *e*.*g*., tangential flow filtration (TFF), density gradient centrifugation, differential extraction, or chromatography. In a specific embodiment, a recombinant virus described herein is isolated.

### 4.4 COMPOSITIONS

Provided herein is a composition comprising a recombinant virus described herein. In a specific embodiment, provided herein is a composition comprising 10 recombinant viruses described herein.

In another embodiment, provided herein is a composition comprising the following: (i) a polynucleotide encoding MX2, (ii) a polynucleotide encoding IFIT3, (iii) a polynucleotide encoding IFITM1, (iv) a polynucleotide encoding IFI27, (v) a polynucleotide encoding IFIT1, (vi) a polynucleotide encoding BST2, (vii) a polynucleotide encoding IFI6, (viii) a polynucleotide encoding RSAD2, (ix) a polynucleotide encoding MX1, and (x) a polynucleotide encoding IFI30. In certain embodiments, the nucleic acid sequence or nucleotide sequence is modRNA.

A nucleic acid sequence(s) described herein may be administered using a gene therapy technique known to one of skill in the art or described herein. In a specific embodiment, a nucleic acid sequence(s) described herein may be administered, e.g., as an mRNA using techniques known to one of skill in the art, including, as described in, *e.g.*, U.S. Patent Application Publication No. 2016/0158354 and Richner et al., 2017, Cell 168: 1114 for examples of such formulations for mRNA delivery. In certain embodiments, a nucleic acid sequence or nucleotide sequence described herein is associated with, encapsulated in, or attached to protamine, a liposome (e.g., a protamine liposome or a cationic polymer liposome), a polysaccharide particle, a cationic nanoemulsion, a cationic lipid nanoparticle, a cationic lipid, cholesterol PEG nanoparticle, a dendrimer nanoparticle or other nanoparticle delivery system. In some embodiments, a recombinant virus or other vector described herein is associated with, encapsulated in, or attached to protamine, a liposome (e.g., a protamine liposome or a cationic polymer liposome), a polysaccharide particle, a cationic nanoemulsion, a cationic lipid nanoparticle, a cationic lipid, cholesterol PEG nanoparticle, a dendrimer nanoparticle or other nanoparticle delivery system. In certain embodiments, a recombinant protein(s) described herein is associated with, encapsulated in, or attached to protamine, a liposome (e.g., a protamine liposome or a cationic polymer liposome), a polysaccharide particle, a cationic nanoemulsion, a cationic lipid nanoparticle, a cationic lipid, cholesterol PEG nanoparticle, a dendrimer nanoparticle or other nanoparticle delivery system. The term "nanoparticle" may refer generally to particles with a diameter of from about 1 nm to about 100 nm, or to about 500 nm, or to about 1,000 nm.

The compositions may be used in methods for treating or preventing a viral infection. In a specific embodiment, the compositions may be used to treat or prevent an RNA virus infection. In another specific embodiment, the compositions may be used to treat or prevent a DNA virus infection . In certain embodiments, the compositions may be used to treat or prevent an influenza virus (*e*.*g*., influenza A virus), rift valley fever virus, Vesicular Stomatitis virus, human cytomegalovirus, herpes simplex (*e*.*g*., HSV-1), HIV, Zika virus, or Nipah virus infection. In some embodiments, the compositions may further comprise another prophylactic or therapeutic agent (*e*.*g*., an antibiotic, antifungal or another agent described herein).

In a specific embodiment, a composition described herein is a pharmaceutical composition. The pharmaceutical compositions provided herein can be in any form that allows for the composition to be administered to a subject. In a specific embodiment, the pharmaceutical compositions are suitable for veterinary administration, human administration or both. The pharmaceutical compositions may comprise a pharmaceutically acceptable carrier. As used herein, the term "pharmaceutically acceptable" means approved by a regulatory agency of the Federal or a state government or listed in the U.S. Pharmacopeia or other generally recognized pharmacopeias for use in animals, and more particularly in humans. The term "carrier" refers to a diluent, adjuvant, excipient, or vehicle with which the pharmaceutical composition is administered. Saline solutions and aqueous dextrose and glycerol solutions can also be employed as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Examples of suitable pharmaceutical carriers are described in "Remington's Pharmaceutical Sciences" by E.W. Martin. The formulation should suit the mode of administration.

A pharmaceutical composition may be formulated for any route of administration to a subject. Specific examples of routes of administration include oral, intransal, transdermal, intradermal, parenteral, and mucosal. In a specific embodiment, the composition is formulated for oral administration. In another specific embodiment, the composition is formulated for intramuscular, subcutaneous or intravenous administration. Parenteral administration, characterized by either subcutaneous, intramuscular or intravenous injection, is also contemplated herein. Injectables can be prepared in conventional forms, either as liquid solutions or suspensions, solid forms suitable for solution or suspension in liquid prior to injection, or as emulsions. The injectables, solutions and emulsions also contain one or more excipients. Suitable excipients are, for example, water, saline, dextrose, glycerol or ethanol.

### 4.5 PROPHYLACTIC AND THERAPEUTIC USES

In one aspect, provided herein are polynucleotides, a recombinant virus(es), or a lipid nanoparticle for use in methods for treating or preventing a virus infection, comprising administering a nucleic acid sequence(s), a recombinant virus(es), or a lipid nanoparticle described herein to a subject in need thereof. In a specific embodiment, provided herein are polynucleotides, a recombinant virus(es), or a lipid nanoparticle for use in a method for treating or preventing a virus infection in a subject, comprising administering to the subject an effective amount of a nucleic acid sequence(s), a recombinant virus(es), or a lipid nanoparticle described herein. In another specific embodiment, provided herein are polynucleotides, a recombinant virus(es), or a lipid nanoparticle for use in a method for treating or preventing a virus infection in a subject, comprising administering to the subject a pharmaceutical composition comprising an effective amount of a nucleic acid sequence(s), a recombinant virus(es), or a lipid nanoparticle described herein. In another specific embodiment, provided herein are polynucleotides, a recombinant virus(es), or a lipid nanoparticle for use in a method for treating or preventing a virus infection, comprising administering to the subject an effective amount of a nucleic acid sequence(s), a recombinant virus(es), or a lipid nanoparticle described herein and another therapy, such as known to one of skill in the art or described herein. In another specific embodiment, provided herein are polynucleotides, a recombinant virus(es), or a lipid nanoparticle for use in a method for treating or preventing a virus infection in a subject, comprising administering to the subject a pharmaceutical composition comprising an effective amount of a nucleic acid sequence(s), a recombinant virus(es), or a lipid nanoparticle described herein, and another therapy, such as known to one of skill in the art or described herein (*see*, *e*.*g*., Section 4.5.2). In certain embodiments, multiple recombinant viruses are administered to a subject. In some embodiments, the viruses each encode a different protein described herein. **In** certain embodiments, multiple compositions are administered to a subject. In specific embodiments, the compositions each comprise a different recombinant virus, or a different nucleic acid sequence described herein.

The virus infection may be an RNA virus infection, a DNA virus infection, or an ambisense virus infection. The virus infection may be caused by a virus with a single or double stranded RNA or DNA genome. In a specific embodiment, the virus infection is an RNA virus infection. The RNA virus may be single stranded or double-stranded, positive or negative sense, and segmented or non-segmented. In some embodiments, the RNA virus is a single-stranded, positive sense RNA virus. In other embodiments, the RNA virus is a single-stranded, negative sense segmented or non-segmented virus. In a specific embodiment, the RNA virus infection is a coronavirus (*e*.*g*., SARS-CoV-1 or SARS-CoV-2) infection, an influenza virus (e.g., an influena A virus or an influenza B virus) infection, a heptatitis C virus infection, a vesicular stomatitis virus (VSV) infection, a flavivirus (e.g., Zika virus) infection, or a Sendai virus infection. The virus infection may be caused by an ambisense RNA virus. In a specific embodiment, the virus infection is Rift Valley Fever Virus infection. The virus infection may be caused by a double-stranded DNA virus. For example, the virus may be herpes simplex virus-1 (HSV-1). In certain embodiments, the virus infection is a Zika virus infection, a VSV infection, an influenza virus (e.g., an influena A virus or an influenza B virus) infection, a Sendai virus infection, an HSV-1 infection, or a SARS-CoV-2 infection. In some embodiments, the virus infection is not an influenza virus infection. In cetain embodiment, the virus infection is not an influenza A virus infection. In some embodiments, the virus infection is not a PR8 infection. In a specific embodiment, the virus infection is SARS-CoV-2 infection. In certain embodiments, the virus infection is an influenza virus (*e.g.*, influenza A virus), rift valley fever virus, Vesicular Stomatitis virus, human cytomegalovirus, herpes simplex (*e*.*g*., HSV-1), HIV, Zika virus, or Nipah virus infection.

In a specific embodiment, a recombinant virus is one described herein or known in the art. For example, the recombinant virus may be one described in Section 4.2, 5 or 6.

The ability of a specific combination of nucleotide sequences encoding MX2, IFIT3, IFITM1, IFI27, IFIT1, BST2, IFI6, RSAD2, MX1, and IFI30, recombinant viruses comprising a transgene(s) comprising a nucleotide sequence encoding MX2, IFIT3, IFITM1, IFI27, IFIT1, BST2, IFI6, RSAD2, MX1, and IF30, or MX2, IFIT3, IFITM1, IFI27, IFIT1, BST2, IFI6, RSAD2, MX1, and IFI30 proteins to inhibit replication of a virus may be assessed in an assay described in Section 5 or 6, *infra.*

In a particular embodiment, the administration of an effective amount of a nucleic acid sequence(s), a recombinant virus(es), or a lipid nanoparticle described herein to the subject inhibits or reduces in the progression of a virus infection. In another embodiment, the administration of an effective amount of a nucleic acid sequence(s), a recombinant virus(es), or a lipid nanoparticle described herein to the subject inhibits or reduces onset, development and/or severity of a symptom (*e*.*g*., fever, myalgia, cough, difficulty breathing, tiredness) of a virus infection. In another embodiment, the administration of an effective amount of a nucleic acid sequence(s), a recombinant virus(es), or a lipid nanoparticle described herein to the subject inhibits or reduces duration of a virus infection, or a symptom associated therewith. In another embodiment, the administration of an effective amount of a nucleic acid sequence(s), a recombinant virus(es), or a lipid nanoparticle described herein to the subject reduces organ failure associated with a virus infection. In another embodiment, the administration of an effective amount of a nucleic acid sequence(s), a recombinant virus(es), or a lipid nanoparticle described herein to the subject reduces the hospitalization of the subject. In another embodiment, the administration of an effective amount of a nucleic acid sequence(s), a recombinant virus(es), or a lipid nanoparticle described herein to the subject reduces the length of hospitalization of the subject. In another embodiment, the administration of an effective amount of a nucleic acid sequence(s), a recombinant virus(es), or a lipid nanoparticle described herein to the subject increases the overall survival of subjects with a virus infection. In another embodiment, the administration of an effective amount of a nucleic acid sequence(s), a recombinant virus(es), or a lipid nanoparticle described herein to the subject prevents the onset or progression of a secondary infection associated with virus infection.

In a specific embodiment, administration of a nucleic acid sequence(s), a recombinant virus(es), or a lipid nanoparticle described herein to a subject reduces the incidence of hospitalization by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the incidence of hospitalization in the absence of administration of a nucleic acid sequence(s), a recombinant virus(es), or a lipid nanoparticle described herein.

In a specific embodiment, administration of a nucleic acid sequence(s), a recombinant virus(es), or a lipid nanoparticle described herein to a subject reduces mortality by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the mortality in the absence of administration of a nucleic acid sequence(s), a recombinant virus(es), or a lipid nanoparticle described herein.

In certain embodiments, the administration of an effective amount of a nucleic acid sequence(s), a recombinant virus(es), or a lipid nanoparticle described herein to a subject results in one, two, three, four, five, or more of the following effects: (i) reduction or amelioration in the severity of a virus infection; (ii) reduction in the duration of a virus infection; (iii) prevention of the progression of a virus infection; (iv) regression of a virus infection; (v) prevention of the development or onset of a symptom of a virus infection; (vi) reduction in organ failure associated with a virus infection; (vii) reduction in the hospitalization of a subject; (viii) reduction in the hospitalization length; (ix) an increase in the survival of a subject with a virus infection; (x) reduction in virus titer; (xi) the reduction in the number of symptoms associated with a virus infection; (xiii) inhibition of replication of the virus; (xiv) enhancement, improvement, supplementation, complementation, or augmentation of the prophylactic or therapeutic effect(s) of another therapy; (xii) prevention of the onset or progression of a secondary infection associated with a virus infection; and/or (xiii) prevention of the onset or diminution of disease severity of bacterial infection occurring secondary to a virus infection.

In a particular embodiment, the administration of an effective amount of a nucleic acid sequence(s), a recombinant virus(es), or a lipid nanoparticle described herein to the subject inhibits or reduces in the progression of COVID-19. In another embodiment, the administration of an effective amount of a nucleic acid sequence(s), a recombinant virus(es), or a lipid nanoparticle described herein to the subject inhibits or reduces onset, development and/or severity of a symptom (*e*.*g*., fever, myalgia, cough, headache, sore throat, nausea, diarrhea, difficulty breathing, lack of smell, lack of taste, tiredness) of COVID-19. In another embodiment, the administration of an effective amount of a nucleic acid sequence(s), a recombinant virus(es), or a lipid nanoparticle described herein to the subject inhibits or reduces duration of COVID-19 or a symptom associated therewith. In another embodiment, the administration of an effective amount of a nucleic acid sequence(s), a recombinant virus(es), or a lipid nanoparticle described herein to the subject reduces organ failure associated with COVID-19. In another embodiment, the administration of an effective amount of a nucleic acid sequence(s), a recombinant virus(es), or a lipid nanoparticle described herein to the subject reduces the hospitalization of the subject. In another embodiment, the administration of an effective amount of a nucleic acid sequence(s), a recombinant virus(es), or a lipid nanoparticle described herein to the subject reduces the length of hospitalization of the subject. In another embodiment, the administration of an effective amount of a nucleic acid sequence(s), a recombinant virus(es), or a lipid nanoparticle described herein to the subject increases the overall survival of subjects with COVID-19. In another embodiment, the administration of an effective amount of a nucleic acid sequence(s), a recombinant virus(es), or a lipid nanoparticle described herein to the subject prevents the onset or progression of a secondary infection associated with SARS-CoV-2 infection.

In a specific embodiment, administration of a nucleic acid sequence(s), a recombinant virus(es), or a lipid nanoparticle described herein to a subject reduces the incidence of hospitalization by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the incidence of hospitalization in the absence of administration of a nucleic acid sequence(s), a recombinant virus(es), or a lipid nanoparticle described herein.

In a specific embodiment, administration of a nucleic acid sequence(s), a recombinant virus(es), or a lipid nanoparticle described herein to a subject reduces mortality by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the mortality in the absence of administration of a nucleic acid sequence(s), a recombinant virus(es), or a lipid nanoparticle described herein.

In certain embodiments, the administration of an effective amount of a nucleic acid sequence(s), a recombinant virus(es), or a lipid nanoparticle described herein to a subject results in one, two, three, four, five, or more of the following effects: (i) reduction or amelioration in the severity of a SARS-CoV-2 infection, COVID-19 or a symptom associated therewith; (ii) reduction in the duration of a SARS-CoV-2 infection, COVID-19 or a symptom associated therewith; (iii) prevention of the progression of a SARS-CoV-2 infection, COVID-19 or a symptom associated therewith; (iv) regression of a SARS-CoV-2 infection, COVID-19 or a symptom associated therewith; (v) prevention of the development or onset of a symptom of a SARS-CoV-2 infection or COVID-19; (vi) reduction in organ failure associated with a SARS-CoV-2 infection or COVID-19; (vii) reduction in the hospitalization of a subject; (viii) reduction in the hospitalization length; (ix) an increase in the survival of a subject with a SARS-CoV-2 infection or COVID-19; (x) reduction in SARS-CoV-2 titer; (xi) the reduction in the number of symptoms associated with a SARS-CoV-2 infection or COVID-19; (xxiii) enhancement, improvement, supplementation, complementation, or augmentation of the prophylactic or therapeutic effect(s) of another therapy; (xii) prevention of the onset or progression of a secondary infection associated with a SARS-CoV-2 infection; and/or (xiii) prevention of the onset or diminution of disease severity of bacterial pneumonias occurring secondary to a SARS-CoV-2 infection.

In a specific embodiment, administration of a nucleic acid sequence(s), a recombinant virus(es), or a lipid nanoparticle described herein to a subject reduces the number of and/or the frequency of symptoms of in the subject (exemplary symptoms of a SARS-CoV-2 include, but are not limited to, body aches (especially joints, muscle, and throat), fever, nausea, headaches, fatigue, sore throat, lack of smell, lack of taste, and difficulty breathing). In another specific embodiment, administration of a nucleic acid sequence(s), a recombinant virus(es), or a lipid nanoparticle described herein to a subject reduces the progression of a SARS-CoV-2 infection or COVID-19 using the WHO ordinal scale. In another specific embodiment, administration of a nucleic acid sequence(s), a recombinant virus(es), or a lipid nanoparticle described herein to a subject reduces the need for invasive mechanical ventilation. In another specific embodiment, administration of a nucleic acid sequence(s), a recombinant virus(es), or a lipid nanoparticle described herein to a subject reduces the need to provide oxygen supplementation to the subject. In another specific embodiment, administration of a nucleic acid sequence(s), a recombinant virus(es), or a lipid nanoparticle described herein to a subject reduces the mortality caused by a SARS-CoV-2 infection or COVID-19.

A nucleic acid sequence(s), a recombinant virus(es), or a lipid nanoparticle described herein may be administered alone or in combination with another/other type of therapy known in the art. *See*, *e*.*g*., Section 4.5.2 for other therapies.

In specific embodiment, a nucleic acid sequence(s), a recombinant virus(es), or a lipid nanoparticle described herein may be used as any line of therapy, including, but not limited to, a first, second, third, fourth and/or fifth line of therapy.

### 4.5.1 ROUTES OF ADMINISTRATION AND DOSAGE

A nucleic acid sequence(s), a recombinant virus(es), or a lipid nanoparticle described herein may be delivered to a subject by a variety of routes. These include, but are not limited to, oral, intradermal, intramuscular, intraperitoneal, transdermal, intravenous, intranasal, intratracheal, and subcutaneous routes. In a specific embodiment, a route known to one of skill in the art is used to administer a nucleic acid sequence(s), a recombinant virus(es), or a lipid nanoparticle described herein.

The amount of a recombinant virus for administration are generally about 10², 5 x 10², 10³, 5 x 10³, 10⁴, 5 x 10⁴, 10⁵, 5 x 10⁵, 10⁶, 5 x 10⁶, 10⁷, 5 x 10⁷, 10⁸, 5 x 10⁸, 1 x 10⁹, 5 x 10⁹, 1 x 10¹⁰, 5 x 10¹⁰, 1 x 10¹¹, 5 x 10¹¹ or 10¹² pfu, and most preferably about 10⁴ to about 10¹², 10⁶ to 10¹², 10⁸ to 10¹², 10⁹ to 10¹², 10⁹ to 10¹¹ pfu, 10⁶ to 10¹⁰, or 10⁶ to 10⁸, and can be administered to a subject once, twice, three, four or more times with intervals as often as needed. Dosage ranges of nucleic acid sequences for administration may include 10 µg to 250 µg, and can be administered to a subject once, twice, three or more times with intervals as often as needed.

### 4.5.2 COMBINATION THERAPY

In various embodiments, a composition described herein may be administered to a subject in combination with one or more other therapies (*e*.*g*., antiviral or immunomodulatory therapies). In some embodiments, a pharmaceutical composition described herein may be administered to a subject in combination with one or more therapies. The one or more other therapies may be in the same composition or a different composition as a nucleic acid sequence(s), a recombinant virus(es), or a lipid nanoparticle described herein.

In some embodiments, the one or more other therapies are supportive measures, such as pain relievers, anti-fever medications, or therapies that alleviate or assist with breathing. Specific examples of supportive measures include humidification of the air by an ultrasonic nebulizer, aerolized racemic epinephrine, oral dexamethasone, intravenous fluids, intubation, fever reducers (*e*.*g*., ibuprofen or acetometaphin), and antibiotic and/or antifungal therapy (*i*.*e*., to prevent or treat secondary bacterial and/or fungal infections). In some embodiments, the one or more other therapies are antivirals.

In certain embodiments, the therapies are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In some embodiments, two or more therapies are administered concurrently. The two or more therapies can be administered in the same composition or a different composition. Further, the two or more therapies can be administered by the same route of administration or a different route of administration.

### 4.5.3 PATIENT POPULATIONS

As used herein, the terms "subject" and "patient" are used interchangeably to refer to an animal (*e*.*g*., birds, reptiles, and mammals, such as humans). In one embodiment, a patient treated in accordance with the methods provided herein is a patient suffering from or expected to suffer from a virus (*e*.*g*., RNA virus or DNA virus) infection. In another embodiment, a patient treated in accordance with the methods provided herein is a patient exposed to a virus (*e*.*g*., RNA virus or DNA virus) infection but not manifesting any symptoms of the infection. In another embodiment, a patient treated in accordance with the methods provided herein is a patient experiencing one or more symptoms of a virus (e.g., RNA virus or DNA virus) infection. In another embodiment, a patient treated in accordance with the methods provided herein is a patient diagnosed with a virus (e.g., RNA virus or DNA virus) infection. In some embodiments, a patient treated in accordance with the methods provided herein is a patient infected with a virus (e.g., RNA virus or DNA virus) that does not manifest any symptoms of the infection. In certain embodiments, a patient treated in accordance with the methods provided herein is a patient infected with a virus (e.g., RNA virus or DNA virus) that manifests mild symptoms of the infection. In some embodiments, a patient treated in accordance with the methods provided herein is a patient infected with a virus (e.g., RNA virus or DNA virus) that manifests moderate symptoms of the infection. In certain embodiments, a patient treated in accordance with the methods provided herein is a patient infected with a virus (e.g., RNA virus or DNA virus) that manifests moderate to severe symptoms of the infection.

In another embodiment, a patient treated in accordance with the methods provided herein is a patient suffering from or expected to suffer from a SARS-CoV-2 infection or COVID-19. In another embodiment, a patient treated in accordance with the methods provided herein is a patient exposed to a SARS-CoV-2 infection but not manifesting any symptoms of the infection or COVID-19. In another embodiment, a patient treated in accordance with the methods provided herein is a patient diagnosed with a SARS-CoV-2 infection or COVID-19. In some embodiments, a patient treated in accordance with the methods provided herein is a patient infected with a SARS-CoV-2 that does not manifest any symptoms of the infection or COVID-19. In certain embodiments, a patient treated in accordance with the methods provided herein is a patient infected with a SARS-CoV-2 that manifests mild symptoms of the infection or COVID-19. In some embodiments, a patient treated in accordance with the methods provided herein is a patient infected with a SARS-CoV-2 that manifests moderate symptoms of the infection or COVID-19. In certain embodiments, a patient treated in accordance with the methods provided herein is a patient infected with a SARS-CoV-2 that manifests moderate to severe symptoms of the infection or COVID-19.

In another embodiment, a patient treated in accordance with the methods provided herein is a patient experiencing one or more symptoms of COVID-19. Symptoms of COVID-19 include, but are not limited to, body aches (especially joints, muscle and throat), fever, nausea, headaches, fatigue, sore throat, lack of sell, lack of taste, diarrhea, and difficulty breathing. In another embodiment, a patient treated in accordance with the methods provided herein is a patient with COVID-19 who does not manifest symptoms of the disease that are severe enough to require hospitalization. In another embodiment, a patient treated in accordance with the methods provided herein is a patient with COVID-19 who manifests symptoms of the disease that are severe enough to require hospitalization.

A patient treated in accordance with the methods provided herein is a human. In certain embodiments, a patient treated in accordance with the methods provided herein is a human infant. In some embodiments, a patient treated in accordance with the methods provided herein is a human toddler. In certain embodiments, a patient treated in accordance with the methods provided herein is a human child. In other embodiments, a patient treated in accordance with the methods provided herein is a human adult. In some embodiments, a patient treated in accordance with the methods provided herein is an elderly human. In certain embodiments, a patient treated in accordance with the methods provided herein is patient that is pregnant. As used herein, the term "human adult" refers to a human that is 18 years or older. As used herein, the term "human child" refers to a human that is 1 year to 18 years old. As used herein, the term "human infant" refers to a newborn to 1 year old human. As used herein, the term "human toddler" refers to a human that is 1 years to 3 years old. As used herein, the term "elderly human" refers to a human that is 65 years old and older.

In some embodiments, a patient treated in accordance with the methods provided herein is a patient infected by a virus (*e.g*., an RNA virus, such as SARS-CoV-2 ) with a condition that increases susceptibility to virus (e.g., the RNA virus, such as SARS-CoV-2 ) complications or for which the virus (*e.g.*, the RNA virus, such as SARS-CoV-2 ) increases complications associated with the condition such as, *e.g.*, conditions that affect the lung, such as cystic fibrosis, asthma, chronic obstructive pulmonary disease, emphysema, or bacterial infections; cardiovascular disease; or diabetes. Other conditions that may increase virus (*e.g*., an RNA virus, such as SARS-CoV-2 ) complications include kidney disorders; blood disorders (including anemia or sickle cell disease); or weakened immune systems (including immunosuppression caused by medications, malignancies such as cancer, organ transplant, or HIV infection). In some embodiments, a patient treated in accordance with the methods provided herein is any subject with a virus (*e.g*., an RNA virus, such as SARS-CoV-2 ) infection who is immunocompromised or immunodeficient.

In certain embodiments, patients treated in accordance with the methods provided herein are patients already being treated with antibiotics, antivirals, antifungals, or other biological therapy/immunotherapy.

### 4.6 KITS

In another aspect, provided herein is a pharmaceutical pack or kit comprising one or more containers filled with a composition (*e.g.,* a pharmaceutical compositions) described herein. In a specific embodiment, provided herein is a pharmaceutical pack or kit comprising one or more containers filled with a nucleic acid sequence(s), a recombinant virus(es), or a lipid nanoparticle described herein. Optionally associated with such container(s) can be a notice in the form prescribed by a governmental agency regulating the manufacture, use or sale of pharmaceuticals or biological products, which notice reflects approval by the agency of manufacture, use or sale for human administration. The kits encompassed herein can be used in the above methods.

### 5. EXAMPLE 1: SYNDICATE OF TEN INTERFERON STIMULATED GENES ARE SUFFICIENT TO SERVE AS A BROAD SPECTRUM ANTIVIRAL

Type-I-interferons (IFN-Is) are cytokines with potent antiviral and inflammatory capacity. IFN-Is elicit this protection by driving the expression of over 500 IFN-I stimulated genes (ISGs) that together control infection. A few of these ISGs are tasked with negatively regulating the IFN-I response to prevent overt inflammation. The tail end of this negative regulation is executed by USP18, which acts directly on the IFN-I receptor, and ISG15 which potentiates USP18's activity, and absence of either leads to prolonged ISG expression. Identification of individuals with genetic deficiencies in ISG15 or USP18 define the safe range of persistent ISG expression. The breadth and magnitude of ISGs expressed in ISG15 deficiency are sufficient to increase broad-spectrum antiviral resistance.

Guided by ISG15-deficiency and the kinetics of ISG negative regulation in WT cells, a combination of 10 ISGs have been identified that recapitulate a substantial portion of the full antiviral potential of the IFN system. The ectopic expression of these 10 ISGs in an IFN-I-signaling-deficient cell line not only increased cellular resistance to Zika virus, Herpes Simplex virus 1, Vesicular Stomatitis virus but also to SARS-Cov2. A deliverable formulation of this 10 ISG syndicate may not only provide protection against the current SARS-CoV-2 viral pandemic, but many current and future viral pathogens.

### 5.1 METHODS

### Cell culture

HEK293T, U4C (JAK1^{-/-}), and hTERT-immortalized dermal fibroblasts from healthy donors, ISG15-, and USP18-deficient individuals were cultured in DMEM (Gibco) supplemented with 10% FBS (Invitrogen), GlutaMAX (350 ng/ml; Gibco), and penicillin/streptomycin (Gibco) (cDMEM). All cells were cultured at 37°C and 10% CO₂. All cell lines were tested for mycoplasma contamination with the MycoAlert PLUS Mycoplasma Detection Kit (Lonza) according to the manufacturer's instructions.

### Ectopic ISG expression

Lentiviral particles were generated by cotransfection of the ISG of interest in the pTRIP.CMV.IVSB.iPuro-2A-TagRFP-DEST vector, psPAX2, and pMD2 by Lipofectamine 2000 (Thermo Fisher Scientific) according to manufacturer's instructions into HEK293T cells. In particular, 3x10⁵ HEK293T cells were seeded on a 6-well tissue culture dish and incubated overnight until cells reach ~70% confluence (~1-2 days). Media (Dubecco's Modified Eagle Media; supplemented with 10% Fetal Bovine Serum, 1% Peniccilin/Streptomycin, 1% D-Glutamine) was changed 30 minutes before transfection. *ISG* lentiviral transfer vector (pTRIP) and packaging vectors, pMD2.G and psPAX2 were mixed in a 5:1:4 molar ratio (total DNA amount: ~2.5 µg) in a final volume of 100 µl of DMEM in an 1.5 mL eppendorf tube. 100 µl of Lipofectamine was added directly to the DNA mixture. Resulting mixture was gently vortexed and incubated at room temperature (25°C) for 5 min. Transfection mixture was added dropwise to HEK293Ts and incubated overnight (16 hours) at 37°C (5% CO₂) and replaced with fresh DMEM. Virus-containing medium was collected 48 and 72 hours after transfection. Supernatants were collected 48 and 72 hours post-transfection, purified through a 0.45 µm filter, and transferred to target cells with 0.4 µg/mL polybrene. Cells were selected with 0.4 µg/mL puromycin. To compensate for equivalent levels of ectopic expression, transfection and transduction experiments were matched with an irrelevant gene (Luciferase) in the same expression vector.

### IFN stimulation

All IFN-I stimulations were performed with IFN-α 2b (Intron-A) in cDMEM and followed the 48 hr prime/rest protocol. Cells were allowed at least 18 hrs to settle after seeding before being exposed to 1,000 U/mL INF-I for 12 hrs. The cells were then washed 3 times with PBS and incubated in cDMEM for an additional 36 hrs prior to sample collection and analysis.

### RNA isolation and quantitative PCR

RNA was extracted from U4C cells or hTERT-immortalized fibroblasts with the Qiagen RNeasy and reverse-transcribed using ABI High Capacity Reverse Transcription kit. The expression of ISGs, relative to the 18S housekeeper gene, was analyzed by TaqMan quantitative real-time PCR (TaqMan Universal Master Mix II with uracil-DNA glycosylases) on a Roche LightCycler 480 II. The relative levels of ISG expression were calculated by the ΔΔCT method.

### RNAseq

Prime/rested hTERTS from three different healthy donors, three ISG15-deficient individuals, two USP18-deficient individuals, and one CRISPR generated USP18-null line derived from one of the healthy donors were sent for 150 bp paired-end sequencing and analyzed with the RNA Express package (STAR aligner followed by DESeq2) in BaseSpace (Illumina).

### Viral challenges and handling of infectious samples

All infections were performed 48 hrs after IFN treatment (12 hr prime, 36 hr rest) in cDMEM at MOIs or dilutions from stock indicated in the figures. Percent infection was determined either with a GFP reporter or with immunofluorescent labeling of viral proteins combined with a DAPI stain to provide a cell count. The ZIKV Puerto Rican clinical isolate, PRVABC059, which was detected with the Anti-Flavivirus Group Antigen Antibody, clone D1-4G2-4-15 (Milipore Sigma), was used. CoV-2 was detected with an anti-NP rabbit polyclonal that was generously gifted to us by Thomas Moran in the Dept. of Microbiology at the Icahn School of Medicine at Mount Sinai. All fluorescence imaging was done on a Celigo Imaging Cytometer (Nexcelom Bioscience), and quantified in CellProfiler (Broad Institute)¹⁰.

All viruses except for CoV-2 are BSL2 and were handled accordingly to ensure safety. CoV-2, on the other hand, was strictly limited to the BSL3 facility at Mount Sinai, and all samples were thoroughly fixed in 4% PFA prior to removal from the facility according to BSL3 protocols.

### 5.2 RESULTS

ISG15-deficient individuals live with constant, low level ISG expression in their blood, presumably due to environmental and commensal stimuli that lead higher tonic IFN-I activity that cannot be completely shut down. However, without these minor immunostimulatory factors to initiate the IFN response and unveil a defect in negative regulation, ISG15^{-/-} cells in culture do not experience baseline persistent ISG expression. To recapitulate the *in vivo* effect of defective negative feedback *in vitro,* cells were stimulated with saturating levels of IFN for 12 hrs, washed off, and incubated for an additional 36 hours. By this point, 48 total hrs after IFN exposure, USP18, stabilized by ISG15, has downregulated most ISG transcription in WT cells, a process that cannot be completed in ISG15- and USP18-null systems. Going forward, cells stimulated with IFN in this fashion (12 hr IFN stim, 36 hr rest) will be referred to as "prime/rested."

Because ISG15 and USP18 are themselves ISGs, their absence has no impact on initial IFN-I activation or peak ISG expression levels, but, as explained above, does allow for persistent, low-level ISG expression (FIG. 1A). Remarkably, this residual ISG expression, though present at only ~1 % of peak IFN-induced activity, is sufficient to substantially restrict a broad-spectrum of viral agents

The impact of persistent ISG expression in ISG15^{-/-} and USP18⁻¹⁻ in hTERTs on Zika virus (ZIKV) was assessed. An overnight ZIKV challenge at an MOI of 1 in prime/rested hTERTS was inhibited 14-fold in ISG15^{-/-} and 188-fold in USP18^{-/-} compared to a similarly treated WT (FIG. 1B).

Just as striking as the antiviral potency of 1% peak ISG expression was the observation that this residual protection comes from only a small fraction of the complete ISG pool. The original description of persistent ISG expression in human ISG15-deficiency noted that only 30 of the 389 canonical ISGs had mRNA levels above WT in prime/rested hTERTS in an RNA microarray. Using RNAseq with a Log₂(fold change) cutoff of 1, and a significance cutoff of q < 0.05, 44 upregulated ISGs out of 107 total upregulated genes in prime/rested ISG15^{-/-} hTERTS, and 98 upregulated ISGs out of 426 total upregulated genes in USP18^{-/-} hTERTs, both compared to unstimulated WT controls were observed (FIGS. 2A-2B). While there are differentially expressed transcripts that are specific to the loss of either ISG15 or USP18, most of the ISGs that stay elevated in ISG15^{-/-} hTERTs were also upregulated in UPS18^{-/-} hTERTS at higher levels (FIGS. 2A, 2C). This overlap of differentially expressed ISGs between ISG15^{-/-} and USP18^{-/-} prime/rested cells suggests that there is clear redundancy within the ISG network that explains how multiple viruses can be shackled by so few antiviral effectors.

The other pattern noticed stemmed from in particular 10 ISGs that remained elevated in prime/rested WT hTERTS, were at higher levels in ISG15^{-/-} hTERTs (FIG. 2D). Further, while orders of magnitude less impressive than cells deficient in IFN negative regulation in most cases, prime/rested WT cells still demonstrated better infection control than IFN naive cells, presumably as a result of the few residual ISGs (FIG. 1B). Together with the notion of ISG redundancy, this modest antiviral activity in prime/rested WT cells indicated to us that perhaps those ISGs that remain active in the WT background have been evolutionarily selected to be the most resistant to IFN shutdown because they are sufficient to cover an appreciable portion of the IFN response. In addition, at the same time, this suggested that their antiviral function is dose dependent, or, in other words, the improved resistance in prime/rested cells from WT to ISG15^{-/-}to USP18^{-/-} may reflect the expression levels of select ISGs.

Building on these ideas, the antiviral potential of these 10 most persistently expressed ISGs was tested (FIG. 2D), along with the well-established PRR, IFIH1, in isolation. To do this, each ISG was stably transduced into a JAK1^{-/-} fibrosarcoma line known as U4Cs (FIG. 3A). The JAK1 deficiency means that these cells cannot respond to IFN, which allowed the antiviral capability of the individual ISG in question to be assessed, independent of any endogenous IFN-I produced by viral challenge.

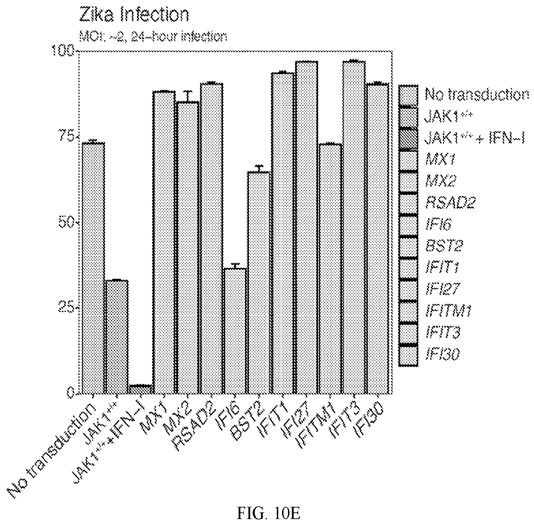

In total the ISGs were tested against 2 viruses: vesicular stomatitis virus (VSV) and herpes simplex virus 1 (HSV1). Certain ISGs act as potent restriction factors for specific viruses on their own. For example, MX1 and IFITM1 were effective against VSV (FIG. 3B), while MX2 proved capable against HSV1 (FIG. 3C), and, consistent with previous reports, MX1 was a potent antagonist of PR8. ZIKV, interestingly, was the only virus that was unaffected by any of these ISGs alone (FIG. 10E) despite being well controlled by prime/rested ISG15^{-/-} and USP18^{-/-}hTERTs.

Fueled by the ZIKV discrepancy, aware that the IFN system does not operate with any one antiviral effector in isolation, and curious about any potential impact of a cooperative effect, a single U4C line was engineered that expresses all 10 of the primer/rest-inspired ISGs, dubbed the sequentially transduced heterogenous population of ISGs (STHP-ISGs). The name STHP-ISG stems from the fact that the line was made by sequentially infecting U4Cs with one lentivirus after the other until a population had been made, which was confirmed by qPCR, that expressed each of the ISGs of interest in combination (FIG. 4A). Specifically, we generated the line by sequentially transducing U4Cs with one lentivirus after the other until we had a population, confirmed by qRT-PCR, that expressed all 10 ISGs (FIG. 4A). The resulting population likely contains multiple cellular subsets expressing different sets of the 10 ISGs of interest (not shown).
Now combined as a 10 ISG syndicate in the ISG combination cell line, the ISG collection not only confers broad-spectrum antiviral immunity, but also protects to the same or even greater levels than the sum of their antiviral effects alone. When challenged against viral insult, broad-spectrum restriction of viral infection by ISG combination transduced cells against VSV, IAV PR8, Zika Virus and SARS-CoV-2 was observed (FIGs, 4B, 5, 10D, and 10H). When challenged with VSV, HSV1, ZIKV and PR8, an impressive control of each by the STHP-ISG U4Cs, including ZIKV (FIGs. 4B-4F). The fact that the STHP-ISG cells resisted ZIKV (FIGs. 4C and 4D), which was unaffected by any these ISGs in isolation, demonstrates that theISGs work in concert to carry out antiviral functions that are not accessible to any one of them in isolation, and that the right combination of only a few could unlock to door to the next generation of broad-spectrum antiviral therapeutics.

FIG. 2K shows that the combination ISG modRNA transfection inhibits SARS-CoV-2 virus replication. U4C JAK1null-ACE2 expressing cells were plated at a density of 15,000 cells/well and allowed to settle in a 96 well cell culture plate. Cells were treated with modified RNAs in the following conditions: non-transfected (control), luciferase (transfection control), and the ISG combination (STHP). All treatments were carried out in triplicate. At 18 hours post-transfection, treated cells were challenged with serially diluted SARS-CoV-2 virus (USA-WA1) for 24 hours. Infections were fixed and stained with primary polyclonal mouse anti-NP antibody (Clone 4G2, 1:10,000 dilution) at 4° C overnight in the dark. Next, primary antibody was removed, cells were washed and incubated with rabbit anti-mouse Alexa Flour 647 (1:1,000 dilution) and DAPI (1: 10,000 dilution) for 1 hour at room temperature in the dark. Cells were washed and suspended in 200 µL/well PBS and imaged using the Celigo Imaging Cytometer.

### 5.3 DISCUSSION

None of the 67 antiviral drugs currently approved by the FDA is clinically used as an effective broad-spectrum treatment. Some viral infections are self-resolving, but many cause severe morbidity and mortality. In 2014, the Ebola virus killed 11,000 people in West Africa. Since 2015, Zika virus in the Americas has resulted in thousands of infants being born with microcephaly. Just under 100 years ago, Spanish Flu is estimated to have killed 20-50 million Europeans. Even today, flu kills 3,000-50,000 individuals annually in the US alone, depending on the flu stain concerned, with the elderly and children most affected. Currently the world is witnessing unprecedented global pandemic caused by SARS-CoV2, which has claimed millions of lives. History shows that every two to three years, there is an epidemic of an extremely contagious, often deadly, viral infection, with tremendous associated morbidities. We, as a society, are not ready for the current outbreak, or for future outbreaks of either novel strains of existing viruses (*e.g. SARS-CoV2, or a* repeat of Spanish-like flu) or of an as yet unknown highly pathogenic virus. The availability of a broad-spectrum antiviral drug would significantly increase the ability to fight off known and unknown viral diseases alike.

It is difficult to argue the case for the development of drugs active against viruses that have not yet discovered. However, by developing a broad-spectrum antiviral drug active against a number of existing viruses, the chance of it being repurposed for use against an unknown, potentially highly pathogenic virus increases. Developing a drug against the flu could save 3,000-50,000 lives in the US every year; a drug active against Zika virus could prevent significant morbidities; and a drug effective against hepatitis B could prevent acute disease and severe long-term consequences and a drug against SARS-CoV2 would have saved millions of lives. The development of such drugs would be a major leap forward, well worth the effort in terms of readiness to deal with unknown viruses or biological warfare.

In all other types of disease, drugs that target the host are used: blood pressure-lowering drugs, anti-inflammatory drugs, anti-depressants and almost all the other types of drug approved by the FDA. Mostly, but with a few notable exceptions (*e.g.* maraviroc), the drugs developed to treat infections have, with good reason, historically targeted the pathogen. However, targeting the host in infections, as in other diseases, can have exceptionally good results, with low levels of adverse effects and broad effectiveness. This example demonstrates that reinforcing host immunity in a safe manner, leads to increased resistance of the host to viral infection.

Genetic studies in humans with inborn errors in IFN-I system have clearly manifested how indispensable this system is for fighting viral infections. On the other side of the coin, examples of overt IFN-I singling due genetic defects in controlling production or response to IFN-I also define how detrimental long term overt IFN-I signaling can be. ISG15 deficient patients have perhaps defined the Goldilocks zone of safe levels of IFN-I signaling, which is enough for augmented control of every virus tested, with minimal side effects (isolated early childhood skin lesion and BCG vaccine localized lymphadenopathy) given that most of these individuals are doing well now and some are in their 20s without any symptoms. Here a system has been engineered where the ISG repertoire has been reduced to a system of 10 effector proteins. With this the essence of antiviral effects was extracted while reducing the inflammation by omitting the other 450+ ISGs. As such the modality of transient gene therapy where inhalation of modified 10 host RNAs is poised to serve as a true broad spectrum antiviral to be used against an existing or yet unknown virus, with a la carte reduction of 10 to perhaps 1 or 2 or select 3 should experiments demonstrate the feasibility.

### 6. EXAMPLE 2: INHIBITION OF VIRUS REPLICATION

Humans with ISG15 deficiency, 5, 6, 7, 35 have mild symptoms, are doing well, with some currently in their twenties. They present with largely asymptomatic intracranial calcifications, isolated adverse reactions to BCG vaccination at birth (no susceptibility to tuberculous bacteria even in individuals living in regions in which TB is endemic) and the persistence of low levels of ISGs. This low-level persistence of ISGs results in enhanced resistance to diverse viruses, including both RNA and DNA viruses of various degrees of pathogenicity (e.g. IAV, HSV-1, VSV, Sindbis virus, RVFV, NiV, ZIKV and HIV). Temporary recreation of these ISGs in WT individuals would, therefore, be a safe way to increase resistance to a broad spectrum of viral infections. *See* FIG. 6A for anti-IAV effects of the ISGs. All these effects, in terms of the higher levels of ISGs (here, IFIT1) or viral control (here, VSV) were lost if the WT allele was introduced (FIGs. 6B and 6C). These ISGs have been mapped by performing RNAseq analyses on WT, ISG15 null and USP18 null cells at 48h times point which mimics human ex vivo state. Speer, S.D., et al., ISG15 deficiency and increased viral resistance in humans but not mice. Nat Commun, 2016. 7: p. 11496 and Zhang, X., et al., Human intracellular ISG15 prevents interferon-alpha/beta over-amplification and auto-inflammation. Nature, 2015. 517(7532): p. 89-93.

46 ISGs diffentially expressed (at least 2-fold increased over WT IFN stimulated control) in ISG15 null system were detected. Subsequently, through selective screens and validations, 10 ISGs were identified (FIG. 7A) (MX1 was added due to its antiviral potency, and removed IFIH1 (as it was a nucleic acid sensor and not effector molecule)) which were most likely to recreate broad anti-viral state documented in ISG15 deficiency. These ISGs (MX2, IFIT3, IFITM1, IFI27, IFIT1, BST2, IFI6, RSAD2, MX1, and IFI30) were tested by cloning them in either a lentivirus or synthesizing modRNAs and using either a IFN-I unresponsive cell line (JAK1 null U4Cs) line that either stably expressed back JAK1, luciferase (LUC) or 10 ISGs (SHTP-ISGs -) to the levels induced by IFN. The same set-up was used for transfecting modRNAs (mock, luciferase or combination of 10 ISGs (STHP)) using RNAiMax tranfection reagent. These cell lines were then challenged with either SARS-CoV-2 (FIGs. 7A and 7B) or IAV PR8 (FIGs. 7C and 7D) and found that indeed combination of these 10 ISGs resulted in augmented control of both viruses, using either lentiviral gene delivery or modRNA gene delivery.

FIG. 8 shows that cells transfected with STHP-old or STHP-new inhibits the replication of Zika virus relative to mock transfected cells or luciferase transfected cells. 10 ISGs - STHP (MX2, IFIT3, IFITM1, IFI27, IFIT1, BST2, IFI6, RSAD2, MX1, and IFI30) in two different batched (denoting STPH -old and STHP-new) or gene ancoding luciferase (negative control) were synthesized into modRNAs and transfected into U4C cells using RNAiMax transfection reagent or they were mock transfected and rested for 48h. These cell lines were then challenged with ZIKA virus for 24h, fixed and stained for E protein. Using microsopy the number of infected cells was determined

FIGs. 9A-9C: ISG15-/- individuals live with constant, low level ISG expression in their blood. This is due to tonic, environmental and commensal induced IFN-I, which they incompletely control. In sterile conditions, ISG15-/- cells in culture do not experience baseline ISG expression. To recapitulate the in vivo effect of defective negative feedback in vitro, cells were stimulated with IFN-I for 12 hours, washed off, and incubated for an additional 36 hours (FIG. 9A). By this point, 48 total hours after IFN-I exposure, USP18, stabilized by ISG15, has downregulated most ISG transcription in fibroblasts from healthy controls, a process that is incomplete in ISG15-/- individuals (FIG. 9A). Cells stimulated with IFN-I in this fashion (12 hour IFN-I stimulation, 36 hour rest) may be referred to herein as "prime/rested.".

As ISG15 and USP18 are primarily induced by IFN-I, their absence has no impact on initial IFN-I activation or on peak ISG expression levels, but does allow for persistent, low-level ISG expression (FIG. 9A). Remarkably, this residual ISG expression, though present at only ~1 % of peak IFN-induced activity, is sufficient to substantially restrict a broad-spectrum of viral agents. The impact of persistent ISG expression in ISG15-/- human immortalized fibroblasts on a Zika virus (ZIKV) infection model was also assessed. An overnight ZIKV challenge at an MOI of 1 in prime/rested fibroblasts was inhibited 14-fold in ISG15-/- compared to similarly treated healthy control cells (FIG. 9B).

The impressive antiviral potency of 1% peak ISG expression was made even more so by the observation that only a small fraction of ISGs exhibit these kinetics. To precisely identify the ISGs responsible for broad-spectrum antiviral activity, RNA sequencing was performed on IFN-I prime/rested fibroblasts from ISG15-/- and healthy/WT individuals. Gene expression analyses identified 61 a number ISGs that were differentially responsive between the two conditions (FIG. 9C). Additionally, a small subset of the differentially regulated ISGs in ISG15-/- cells also remained elevated above baseline in the prime-rested fibroblasts from healthy controls, albeit at lower levels as compared to unstimulated WT cells (FIG. 2D). Given that healthy control cells primed with IFN-I retain some antiviral protection following a prime/rest stimulation (FIG. 9B), ISGs with lingering expression in a 'wild-type' background may have evolved the slowest shutdown kinetics because they are sufficient to exert some antiviral effects (FIG. 9B). At the same time, their antiviral function is dose-dependent and that the improved resistance in prime/rested ISG15-/- cells may, at least partially, reflect the expression levels of these select ISGs (FIG. 2D)

Based on this observation and the known antiviral functions of these ISGs, 10 persistently elevated ISG effectors were chosen for evaluation of resistance to viral infection such as documented in ISG15 deficiency. As disclosed herein, and without being limited to a particular mechanism of action, these 10 ISGs may confer antiviral resistance in a similar mechanism, akin to therapeutics strategies used for HIV (HAART) where multipes stages of the viral lifecycle are targeted.

To test the antiviral potential of these 10 ISGs, each ISG was stably transduced into a JAK1-/-, IFN-I non-responsive fibrosarcoma (U4C) cell lines (FIG. 2D) . Leveraging the inability of these cells to respond to IFN-I enabled assessment of the antiviral capacity of each individual ISG in question, independent of any endogenous IFN-I produced due to viral challenge (FIG. 10A).

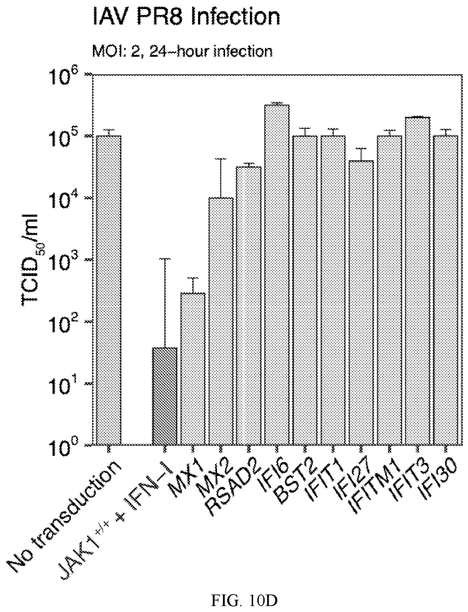

After validating select ISG expression in their respective cell lines (FIG. 10B), antiviral potency of these ISGs against a panel of RNA viruses was tested: Vesicular Stomatitis Virus (VSV), Influenza A virus (IAV PR8), and Zika Virus (ZIKV PR2015). MX1 was sufficient to restrict VSV infection and IAV PR8 infection (FIGs. 10C and 10D), while IFI6 proved capable against Zika Virus (FIG. 10E), although infection was controlled to even greater levels by prime/rested fibroblasts from ISG15-/- individuals (FIG. 9B).

Prime/rested ISG15-/- fibroblasts outperformed pan-flavivirus restriction factor IFI6 in a ZIKV challenge. As disclosed herein, the combination of the 10 ISGs work in a cooperative manner to restrict viral infection. A single U4C fibrosarcoma cell was lentivirally engineerd to expresses all 10 of the ISG effectors persistently elevated in healthy and ISG15-/- prime-rested fibroblasts (FIG. 10F). U4Cs were sequentially transduced with one lentivirus after the other to produce a population, confirmed by qRT-PCR, that expressed all 10 ISGs (FIG. 4A). High titer lentivirus stocks were used for transductions (MOI > 20), and we confirmed the resulting population contained multiple cellular subsets expressing different sets of the 10 ISGs of interest (not shown).

As a 10 ISG syndicate in the ISG combination cell line, the ISG collection not only confers broad-spectrum antiviral immunity, but also protects to the same or even greater levels than the sum of their antiviral effects alone. When challenged against viral insult, broad-spectrum restriction of viral infection by ISG combination transduced cells against VSV, IAV PR8, Zika Virus and SARS-CoV-2 was observed (FIGs. 4B, 5, 10D, and 10H). As the ISG combination line resisted ZIKV (FIG. 10G) demonstrates that these select ISGs may work in concert to carry out antiviral functions.

As disclosed herein, transient expression of these 10 persistently elevated ISGs in combination may be sufficient to restrict viral infection, prophylactically and/or therapeutically (FIG. 11A). U4Cs transfected with lipid nanoparticles (LNPs) carrying synthesized modified RNAs (modRNA) with ARCA cap and pseudouridine substituted for uridine encoding each of the 10 ISGs were challenged with a diverse set of viruses. First to assess the efficiency and immunogenicity of LNP-mRNA ISG transfection, modRNA levels of target ISGs in IFN-I non-responsive human cells were measured, 6 hours post-transfection (FIG. 11B). Expression of all 10 ISGs was detected (FIG. 11C), and the level of ectopic ISGs detected were comparable to peak expression induced by IFN stimulation (FIG. 11C). Expression of USP18, a control ISG not included in the STHP combination, was unchanged from the untreated and mock-transfected conditions, demonstrating specificity of the LNP-modRNA delivery (FIG. 11C). As disclosed herein, ISG expression inhibited viral infection, e.g. VSV (FIG. 11D) and Zika virus infection.

Antival effectiveness in vivo was also demonstrated, using the Syrian Golden Hamster (Mesocricetus auratus) model to assess SARS-CoV-2 infection FIG. 12A). Physiological effects of administering LNP-modRNA encoding enhanced GFP (eGFP) or the 10-ISG combination to hamsters, administered intranasally and intratracheally, was assessed. In harvested lung tissue, high efficiency of modRNA-LNP encoding eGFP to the lung in both intranasal and intratracheal delivery groups was demonstrated (FIG. 12B). Next to assess the antiviral capacity of the 10-ISG combination delivered intranasally, animals were treated with LNP-modRNA 1 day prior to SARS-CoV-2 viral challenge, at a sub-lethal does, and weight monitored and tissues collected over a 7-day period (FIG. 12C). Initially, all animals gained weight, however beginning at 2 days post-infection, experimental groups began to stratify. At the end of the experiment, compared to mock-treated animals or animals treated with LNP-modRNA encoding eGFP, animals treated with LNP-modRNA encoding the 10 ISGs were protected from losing weight to lethal levels by day 6-7 (FIG. 12C). Furthermore, lung RNA seq (FIG. 12D) and viral titer measurements in the nasal cavity at 1-day post-infection revealed a reduction in the levels of infectious SARS-CoV-2 virions in the 10-ISG combination treatment group compared to mock and eGFP groups (FIG. 12E), which was also reflected in histopathology scoring of post mortem lungs (FIGs. 12G and 12H). FIG. 12F shows PCA analyses of RNASeq from lungs of hamsters as indicated.

### 7. SEQUENCES:

MX2(1) (SEQ ID NO:1) 
ATGTCTAAGGCCCACAAGCCTTGGCCCTACCGGAGGAGAAGTCAATTTTCTTCTCGAAAATACC
TGAAAAAAGAAATGAATTCCTTCCAGCAACAGCCACCGCCATTCGGCACAGTGCCACCACAAAT
GATGTTTCCTCCAAACTGGCAGGGGGCAGAGAAGGACGCTGCTTTCCTCGCCAAGGACTTCAAC
TTTCTCACTTTGAACAATCAGCCACCACCAGGAAACAGGAGCCAACCAAGGGCAATGGGGCCCG
AGAACAACCTGTACAGCCAGTACGAGCAGAAGGTGCGCCCCTGCATTGACCTCATCGACTCCCT
GCGGGCTCTGGGTGTGGAGCAGGACCTGGCCCTGCCAGCCATCGCCGTCATCGGGGACCAGAGC
TCGGGCAAGAGCTCTGTGCTGGAGGCACTGTCAGGAGTCGCGCTTCCCAGAGGCAGCGGAATCG
TAACCAGGTGTCCGCTGGTGCTGAAACTGAAAAAGCAGCCCTGTGAGGCATGGGCCGGAAGGAT
CAGCTACCGGAACACCGAGCTAGAGCTTCAGGACCCTGGCCAGGTGGAGAAAGAGATACACAAA
GCCCAGAACGTCATGGCCGGGAATGGCCGGGGCATCAGCCATGAGCTCATCAGCCTGGAGATCA
CCTCCCCTGAGGTTCCAGACCTGACCATCATTGACCTTCCCGGCATCACCAGGGTGGCTGTGGA
CAACCAGCCCCGAGACATCGGACTGCAGATCAAGGCTCTCATCAAGAAGTACATCCAGAGGCAG
CAGACGATCAACTTGGTGGTGGTTCCCTGTAACGTGGACATTGCCACCACGGAGGCGCTGAGCA
TGGCCCATGAGGTGGACCCGGAAGGGGACAGGACCATCGGTATCCTGACCAAACCAGATCTAAT
GGACAGGGGCACTGAGAAAAGCGTCATGAATGTGGTGCGGAACCTCACGTACCCCCTCAAGAAG
GGCTACATGATTGTGAAGTGCCGGGGCCAGCAGGAGATCACAAACAGGCTGAGCTTGGCAGAGG
CAACCAAGAAAGAAATTACATTCTTTCAAACACATCCATATTTCAGAGTTCTCCTGGAGGAGGG
GTCAGCCACGGTTCCCCGACTGGCAGAAAGACTTACCACTGAACTCATCATGCATATCCAAAAA
 
TCGCTCCCGTTGTTAGAAGGACAAATAAGGGAGAGCCACCAGAAGGCGACCGAGGAGCTGCGGC
GTTGCGGGGCTGACATCCCCAGCCAGGAGGCCGACAAGATGTTCTTTCTAATTGAGAAAATCAA
GATGTTTAATCAGGACATCGAAAAGTTAGTAGAAGGAGAAGAAGTTGTAAGGGAGAATGAGACC
CGTTTATACAACAAAATCAGAGAGGATTTTAAAAACTGGGTAGGCATACTTGCAACTAATACCC
AAAAAGTTAAAAATATTATCCACGAAGAAGTTGAAAAATATGAAAAGCAGTATCGAGGCAAGGA
GCTTCTGGGATTTGTCAACTACAAGACATTTGAGATCATCGTGCATCAGTACATCCAGCAGCTG
GTGGAGCCCGCCCTTAGCATGCTCCAGAAAGCCATGGAAATTATCCAGCAAGCTTTCATTAACG
TGGCCAAAAAACATTTTGGCGAATTTTTCAACCTTAACCAAACTGTTCAGAGCACGATTGAAGA
CATAAAAGTGAAACACACAGCAAAGGCAGAAAACATGATCCAACTTCAGTTCAGAATGGAGCAG
ATGGTTTTTTGTCAAGATCAGATTTACAGTGTTGTTCTGAAGAAAGTCCGAGAAGAGATTTTTA
ACCCTCTGGGGACGCCTTCACAGAATATGAAGTTGAACTCTCATTTTCCCAGTAATGAGTCTTC
GGTTTCCTCCTTTACTGAAATAGGCATCCACCTGAATGCCTACTTCTTGGAAACCAGCAAACGT
CTCGCCAACCAGATCCCATTTATAATTCAGTATTTTATGCTCCGAGAGAATGGTGACTCCTTGC
AGAAAGCCATGATGCAGATACTACAGGAAAAAAATCGCTATTCCTGGCTGCTTCAAGAGCAGAG
TGAGACCGCTACCAAGAGAAGAATCCTTAAGGAGAGAATTTACCGGCTCACTCAGGCGCGACAC
GCACTCTGTCAATTCTCCAGCAAAGAGATCCACTGA
IFIT3(2) (SEQ ID NO:2) 
ATGAGTGAGGTCACCAAGAATTCCCTGGAGAAAATCCTTCCACAGCTGAAATGCCATTTCACCT
GGAACTTATTCAAGGAAGACAGTGTCTCAAGGGATCTAGAAGATAGAGTGTGTAACCAGATTGA
ATTTTTAAACACTGAGTTCAAAGCTACAATGTACAACTTGTTGGCCTACATAAAACACCTAGAT
GGTAACAACGAGGCAGCCCTGGAATGCTTACGGCAAGCTGAAGAGTTAATCCAGCAAGAACATG
CTGACCAAGCAGAAATCAGAAGTCTAGTCACTTGGGGAAACTACGCCTGGGTCTACTATCACTT
GGGCAGACTCTCAGATGCTCAGATTTATGTAGATAAGGTGAAACAAACCTGCAAGAAATTTTCA
AATCCATACAGTATTGAGTATTCTGAACTTGACTGTGAGGAAGGGTGGACACAACTGAAGTGTG
GAAGAAATGAAAGGGCGAAGGTGTGTTTTGAGAAGGCTCTGGAAGAAAAGCCCAACAACCCAGA
ATTCTCCTCTGGACTGGCAATTGCGATGTACCATCTGGATAATCACCCAGAGAAACAGTTCTCT
ACTGATGTTTTGAAGCAGGCCATTGAGCTGAGTCCTGATAACCAATACGTCAAGGTTCTCTTGG
GCCTGAAACTGCAGAAGATGAATAAAGAAGCTGAAGGAGAGCAGTTTGTTGAAGAAGCCTTGGA
AAAGTCTCCTTGCCAAACAGATGTCCTCCGCAGTGCAGCCAAATTTTACAGAAGAAAAGGTGAC
CTAGACAAAGCTATTGAACTGTTTCAACGGGTGTTGGAATCCACACCAAACAATGGCTACCTCT
ATCACCAGATTGGGTGCTGCTACAAGGCAAAAGTAAGACAAATGCAGAATACAGGAGAATCTGA
AGCTAGTGGAAATAAAGAGATGATTGAAGCACTAAAGCAATATGCTATGGACTATTCGAATAAA
GCTCTTGAGAAGGGACTGAATCCTCTGAATGCATACTCCGATCTCGCTGAGTTCCTGGAGACGG
AATGTTATCAGACACCATTCAATAAGGAAGTCCCTGATGCTGAAAAGCAACAATCCCATCAGCG
CTACTGCAACCTTCAGAAATATAATGGGAAGTCTGAAGACACTGCTGTGCAACATGGTTTAGAG
GGTTTGTCCATAAGCAAAAAATCAACTGACAAGGAAGAGATCAAAGACCAACCACAGAATGTAT
CTGAAAATCTGCTTCCACAAAATGCACCAAATTATTGGTATCTTCAAGGATTAATTCATAAGCA
GAATGGAGATCTGCTGCAAGCAGCCAAATGTTATGAGAAGGAACTGGGCCGCCTGCTAAGGGAT
GCCCCTTCAGGCATAGGCAGTATTTTCCTGTCAGCATCTGAGCTTGAGGATGGTAGTGAGGAAA
TGGGCCAGGGCGCAGTCAGCTCCAGTCCCAGAGAGCTCCTCTCTAACTCAGAGCAACTGAACTG
A
IFITM1(3) (SEQ ID NO:3) 
ATGCACAAGGAGGAACATGAGGTGGCTGTGCTGGGGGCACCCCCCAGCACCATCCTTCCAAGGT
CCACCGTGATCAACATCCACAGCGAGACCTCCGTGCCCGACCATGTCGTCTGGTCCCTGTTCAA
CACCCTCTTCTTGAACTGGTGCTGTCTGGGCTTCATAGCATTCGCCTACTCCGTGAAGTCTAGG
 
GACAGGAAGATGGTTGGCGACGTGACCGGGGCCCAGGCCTATGCCTCCACCGCCAAGTGCCTGA
ACATCTGGGCCCTGATTCTGGGCATCCTCATGACCATTGGATTCATCCTGTTACTGGTATTCGG
CTCTGTGACAGTCTACCATATTATGTTACAGATAATACAGGAAAAACGGGGTTACTAG
IFI27(4) (SEQ ID NO:4) 
ATGGAGGCCTCTGCTCTCACCTCATCAGCAGTGACCAGTGTGGCCAAAGTGGTCAGGGTGGCCT
CTGGCTCTGCCGTAGTTTTGCCCCTGGCCAGGATTGCTACAGTTGTGATTGGAGGAGTTGTGGC
CATGGCGGCTGTGCCCATGGTGCTCAGTGCCATGGGCTTCACTGCGGCGGGAATCGCCTCGTCC
TCCATAGCAGCCAAGATGATGTCCGCGGCGGCCATTGCCAATGGGGGTGGAGTTGCCTCGGGCA
GCCTTGTGGCTACTCTGCAGTCACTGGGAGCAACTGGACTCTCCGGATTGACCAAGTTCATCCT
GGGCTCCATTGGGTCTGCCATTGCGGCTGTCATTGCGAGGTTCTACTAG
IFIT1(5) (SEQ ID NO:5) 
ATGCCTGATTTAGAAAACAGAGTCTTGGATCAGATTGAATTCCTAGACACCAAATACAGTGTGG
GAATACACAACCTACTAGCCTATGTGAAACACCTGAAAGGCCAGAATGAGGAAGCCCTGAAGAG
CTTAAAAGAAGCTGAAAACTTAATGCAGGAAGAACATGACAACCAAGCAAATGTGAGGAGTCTG
GTGACCTGGGGCAACTTTGCCTGGATGTATTACCACATGGGCAGACTGGCAGAAGCCCAGACTT
ACCTGGACAAGGTGGAGAACATTTGCAAGAAGCTTTCAAATCCCTTCCGCTATAGAATGGAGTG
TCCAGAAATAGACTGTGAGGAAGGATGGGCCTTGCTGAAGTGTGGAGGAAAGAATTATGAACGG
GCCAAGGCCTGCTTTGAAAAGGTGCTTGAAGTGGACCCTGAAAACCCGGAATCCAGCGCTGGGT
ATGCGATCTCTGCCTATCGCCTGGATGGCTTTAAATTAGCCACAAAAAATCACAAGCCATTTTC
TTTGCTTCCCCTAAGGCAGGCTGTCCGCTTAAATCCAGACAATGGATATATTAAGGTTCTCCTT
GCCCTGAAGCTTCAGGATGAAGGACAGGAAGCTGAAGGAGAAAAGTACATTGAAGAAGCTCTAG
CCAACATGTCCTCACAGACCTATGTCTTTCGATATGCAGCCAAGTTTTACCGAAGAAAAGGCTC
TGTGGATAAAGCTCTTGAGTTATTAAAAAAGGCCTTGCAGGAAACACCCACTTCTGTCTTACTG
CATCACCAGATAGGGCTTTGCTACAAGGCACAAATGATCCAAATCAAGGAGGCTACAAAAGGGC
AGCCTAGAGGGCAGAACAGAGAAAAGCTAGACAAAATGATAAGATCAGCCATATTTCATTTTGA
ATCTGCAGTGGAAAAAAAGCCCACATTTGAGGTGGCTCATCTAGACCTGGCAAGAATGTATATA
GAAGCAGGCAATCACAGAAAAGCTGAAGAGAATTTTCAAAAATTGTTATGCATGAAACCAGTGG
TAGAAGAAACAATGCAAGACATACATTTCCACTATGGTCGGTTTCAGGAATTTCAAAAGAAATC
TGACGTCAATGCAATTATCCATTATTTAAAAGCTATAAAAATAGAACAGGCATCATTAACAAGG
GATAAAAGTATCAATTCTTTGAAGAAATTGGTTTTAAGGAAACTTCGGAGAAAGGCATTAGATC
TGGAAAGCTTGAGCCTCCTTGGGTTCGTCTACAAATTGGAAGGAAATATGAATGAAGCCCTGGA
GTACTATGAGCGGGCCCTGAGACTGGCTGCTGACTTTGAGAACTCTGTGAGACAAGGTCCTTAG
BST2(6) (SEQ ID NO:6) 
ATGGCATCTACTTCGTATGACTATTGCAGAGTGCCCATGGAAGACGGGGATAAGCGCTGTAAGC
TTCTGCTGGGGATAGGAATTCTGGTGCTCCTGATCATCGTGATTCTGGGGGTGCCCTTGATTAT
CTTCACCATCAAGGCCAACAGCGAGGCCTGCCGGGACGGCCTTCGGGCAGTGATGGAGTGTCGC
AATGTCACCCATCTCCTGCAACAAGAGCTGACCGAGGCCCAGAAGGGCTTTCAGGATGTGGAGG
CCCAGGCCGCCACCTGCAACCACACTGTGATGGCCCTAATGGCTTCCCTGGATGCAGAGAAGGC
CCAAGGACAAAAGAAAGTGGAGGAGCTTGAGGGAGAGATCACTACATTAAACCATAAGCTTCAG
GACGCGTCTGCAGAGGTGGAGCGACTGAGAAGAGAAAACCAGGTCTTAAGCGTGAGAATCGCGG
ACAAGAAGTACTACCCCAGCTCCCAGGACTCCAGCTCCGCTGCGGCGCCCCAGCTGCTGATTGT
GCTGCTGGGCCTCAGCGCTCTGCTGCAGTGA
IFI6(7) (SEQ ID NO:7) 
ATGCGGCAGAAGGCGGTATCGCTTTTCTTGTGCTACCTGCTGCTCTTCACTTGCAGTGGGGTGG
AGGCAGGTAAGAAAAAGTGCTCGGAGAGCTCGGACAGCGGCTCCGGGTTCTGGAAGGCCCTGAC
CTTCATGGCCGTCGGAGGAGGACTCGCAGTCGCCGGGCTGCCCGCGCTGGGCTTCACCGGCGCC
GGCATCGCGGCCAACTCGGTGGCTGCCTCGCTGATGAGCTGGTCTGCGATCCTGAATGGGGGCG
GCGTGCCCGCCGGGGGGCTAGTGGCCACGCTGCAGAGCCTCGGGGCTGGTGGCAGCAGCGTCGT
CATAGGTAATATTGGTGCCCTGATGGGCTACGCCACCCACAAGTATCTCGATAGTGAGGAGGAT
GAGGAGTAG
RSAD2(8) (SEQ ID NO:8) 
ATGTGGGTGCTTACACCTGCTGCTTTTGCTGGGAAGCTCTTGAGTGTGTTCAGGCAACCTCTGA
GCTCTCTGTGGAGGAGCCTGGTCCCGCTGTTCTGCTGGCTGAGGGCAACCTTCTGGCTGCTAGC
TACCAAGAGGAGAAAGCAGCAGCTGGTCCTGAGAGGGCCAGATGAGACCAAAGAGGAGGAAGAG
GACCCTCCTCTGCCCACCACCCCAACCAGCGTCAACTATCACTTCACTCGCCAGTGCAACTACA
AATGCGGCTTCTGTTTCCACACAGCCAAAACATCCTTTGTGCTGCCCCTTGAGGAAGCAAAGAG
AGGATTGCTTTTGCTTAAGGAAGCTGGTATGGAGAAGATCAACTTTTCAGGTGGAGAGCCATTT
CTTCAAGACCGGGGAGAATACCTGGGCAAGTTGGTGAGGTTCTGCAAAGTAGAGTTGCGGCTGC
CCAGCGTGAGCATCGTGAGCAATGGAAGCCTGATCCGGGAGAGGTGGTTCCAGAATTATGGTGA
GTATTTGGACATTCTCGCTATCTCCTGTGACAGCTTTGACGAGGAAGTCAATGTCCTTATTGGC
CGTGGCCAAGGAAAGAAGAACCATGTGGAAAACCTTCAAAAGCTGAGGAGGTGGTGTAGGGATT
ATAGAGTCGCTTTCAAGATAAATTCTGTCATTAATCGTTTCAACGTGGAAGAGGACATGACGGA
ACAGATCAAAGCACTAAACCCTGTCCGCTGGAAAGTGTTCCAGTGCCTCTTAATTGAGGGTGAG
AATTGTGGAGAAGATGCTCTAAGAGAAGCAGAAAGATTTGTTATTGGTGATGAAGAATTTGAAA
GATTCTTGGAGCGCCACAAAGAAGTGTCCTGCTTGGTGCCTGAATCTAACCAGAAGATGAAAGA
CTCCTACCTTATTCTGGATGAATATATGCGCTTTCTGAACTGTAGAAAGGGACGGAAGGACCCT
TCCAAGTCCATCCTGGATGTTGGTGTAGAAGAAGCTATAAAATTCAGTGGATTTGATGAAAAGA
TGTTTCTGAAGCGAGGAGGAAAATACATATGGAGTAAGGCTGATCTGAAGCTGGATTGGTAG
IFIH1(9) (SEQ ID NO:9) 
ATGTCGAATGGGTATTCCACAGACGAGAATTTCCGCTATCTCATCTCGTGCTTCAGGGCCAGGG
TGAAAATGTACATCCAGGTGGAGCCTGTGCTGGACTACCTGACCTTTCTGCCTGCAGAGGTGAA
GGAGCAGATTCAGAGGACAGTCGCCACCTCCGGGAACATGCAGGCAGTTGAACTGCTGCTGAGC
ACCTTGGAGAAGGGAGTCTGGCACCTTGGTTGGACTCGGGAATTCGTGGAGGCCCTCCGGAGAA
CCGGCAGCCCTCTGGCCGCCCGCTACATGAACCCTGAGCTCACGGACTTGCCCTCTCCATCGTT
TGAGAACGCTCATGATGAATATCTCCAACTGCTGAACCTCCTTCAGCCCACTCTGGTGGACAAG
CTTCTAGTTAGAGACGTCTTGGATAAGTGCATGGAGGAGGAACTGTTGACAATTGAAGACAGAA
ACCGGATTGCTGCTGCAGAAAACAATGGAAATGAATCAGGTGTAAGAGAGCTACTAAAAAGGAT
TGTGCAGAAAGAAAACTGGTTCTCTGCATTTCTGAATGTTCTTCGTCAAACAGGAAACAATGAA
CTTGTCCAAGAGTTAACAGGCTCTGATTGCTCAGAAAGCAATGCAGAGATTGAGAATTTATCAC
AAGTTGATGGTCCTCAAGTGGAAGAGCAACTTCTTTCAACCACAGTTCAGCCAAATCTGGAGAA
GGAGGTCTGGGGCATGGAGAATAACTCATCAGAATCATCTTTTGCAGATTCTTCTGTAGTTTCA
GAATCAGACACAAGTTTGGCAGAAGGAAGTGTCAGCTGCTTAGATGAAAGTCTTGGACATAACA
GCAACATGGGCAGTGATTCAGGCACCATGGGAAGTGATTCAGATGAAGAGAATGTGGCAGCAAG
AGCATCCCCGGAGCCAGAACTCCAGCTCAGGCCTTACCAAATGGAAGTTGCCCAGCCAGCCTTG
GAAGGGAAGAATATCATCATCTGCCTCCCTACAGGGAGTGGAAAAACCAGAGTGGCTGTTTACA
TTGCCAAGGATCACTTAGACAAGAAGAAAAAAGCATCTGAGCCTGGAAAAGTTATAGTTCTTGT
 
CAATAAGGTACTGCTAGTTGAACAGCTCTTCCGCAAGGAGTTCCAACCATTTTTGAAGAAATGG
TATCGTGTTATTGGATTAAGTGGTGATACCCAACTGAAAATATCATTTCCAGAAGTTGTCAAGT
CCTGTGATATTATTATCAGTACAGCTCAAATCCTTGAAAACTCCCTCTTAAACTTGGAAAATGG
AGAAGATGCTGGTGTTCAATTGTCAGACTTTTCCCTCATTATCATTGATGAATGTCATCACACC
AACAAAGAAGCAGTGTATAATAACATCATGAGGCATTATTTGATGCAGAAGTTGAAAAACAATA
GACTCAAGAAAGAAAACAAACCAGTGATTCCCCTTCCTCAGATACTGGGACTAACAGCTTCACC
TGGTGTTGGAGGGGCCACGAAGCAAGCCAAAGCTGAAGAACACATTTTAAAACTATGTGCCAAT
CTTGATGCATTTACTATTAAAACTGTTAAAGAAAACCTTGATCAACTGAAAAACCAAATACAGG
AGCCATGCAAGAAGTTTGCCATTGCAGATGCAACCAGAGAAGATCCATTTAAAGAGAAACTTCT
AGAAATAATGACAAGGATTCAAACTTATTGTCAAATGAGTCCAATGTCAGATTTTGGAACTCAA
CCCTATGAACAATGGGCCATTCAAATGGAAAAAAAAGCTGCAAAAGAAGGAAATCGCAAAGAAC
GTGTTTGTGCAGAACATTTGAGGAAGTACAATGAGGCCCTACAAATTAATGACACAATTCGAAT
GATAGATGCGTATACTCATCTTGAAACTTTCTATAATGAAGAGAAAGATAAGAAGTTTGCAGTC
ATAGAAGATGATAGTGATGAGGGTGGTGATGATGAGTATTGTGATGGTGATGAAGATGAGGATG
ATTTAAAGAAACCTTTGAAACTGGATGAAACAGATAGATTTCTCATGACTTTATTTTTTGAAAA
CAATAAAATGTTGAAAAGGCTGGCTGAAAACCCAGAATATGAAAATGAAAAGCTGACCAAATTA
AGAAATACCATAATGGAGCAATATACTAGGACTGAGGAATCAGCACGAGGAATAATCTTTACAA
AAACACGACAGAGTGCATATGCGCTTTCCCAGTGGATTACTGAAAATGAAAAATTTGCTGAAGT
AGGAGTCAAAGCCCACCATCTGATTGGAGCTGGACACAGCAGTGAGTTCAAACCCATGACACAG
AATGAACAAAAAGAAGTCATTAGTAAATTTCGCACTGGAAAAATAAATCTGCTTATCGCTACCA
CAGTGGCAGAAGAAGGTCTGGATATTAAAGAATGTAACATTGTTATCCGTTATGGTCTCGTCAC
CAATGAAATAGCCATGGTCCAGGCCCGTGGTCGAGCCAGAGCTGATGAGAGCACCTACGTCCTG
GTTGCTCACAGTGGTTCAGGAGTTATCGAACATGAGACAGTTAATGATTTCCGAGAGAAGATGA
TGTATAAAGCTATACATTGTGTTCAAAATATGAAACCAGAGGAGTATGCTCATAAGATTTTGGA
ATTACAGATGCAAAGTATAATGGAAAAGAAAATGAAAACCAAGAGAAATATTGCCAAGCATTAC
AAGAATAACCCATCACTAATAACTTTCCTTTGCAAAAACTGCAGTGTGCTAGCCTGTTCTGGGG
AAGATATCCATGTAATTGAGAAAATGCATCACGTCAATATGACCCCAGAATTCAAGGAACTTTA
CATTGTAAGAGAAAACAAAGCACTGCAAAAGAAGTGTGCCGACTATCAAATAAATGGTGAAATC
ATCTGCAAATGTGGCCAGGCTTGGGGAACAATGATGGTGCACAAAGGCTTAGATTTGCCTTGTC
TCAAAATAAGGAATTTTGTAGTGGTTTTCAAAAATAATTCAACAAAGAAACAATACAAAAAGTG
GGTAGAATTACCTATCACATTTCCCAATCTTGACTATTCAGAATGCTGTTTATTTAGTGATGAG
GATTAG
MX1(10) (SEQ ID NO:10) 
ATGGTTGTTTCCGAAGTGGACATCGCAAAAGCTGATCCAGCTGCTGCATCCCACCCTCTATTAC
TGAATGGAGATGCTACTGTGGCCCAGAAAAATCCAGGCTCGGTGGCTGAGAACAACCTGTGCAG
CCAGTATGAGGAGAAGGTGCGCCCCTGCATCGACCTCATTGACTCCCTGCGGGCTCTAGGTGTG
GAGCAGGACCTGGCCCTGCCAGCCATCGCCGTCATCGGGGACCAGAGCTCGGGCAAGAGCTCCG
TGTTGGAGGCACTGTCAGGAGTTGCCCTTCCCAGAGGCAGCGGGATCGTGACCAGATGCCCGCT
GGTGCTGAAACTGAAGAAACTTGTGAACGAAGATAAGTGGAGAGGCAAGGTCAGTTACCAGGAC
TACGAGATTGAGATTTCGGATGCTTCAGAGGTAGAAAAGGAAATTAATAAAGCCCAGAATGCCA
TCGCCGGGGAAGGAATGGGAATCAGTCATGAGCTAATCACCCTGGAGATCAGCTCCCGAGATGT
CCCGGATCTGACTCTAATAGACCTTCCTGGCATAACCAGAGTGGCTGTGGGCAATCAGCCTGCT
GACATTGGGTATAAGATCAAGACACTCATCAAGAAGTACATCCAGAGGCAGGAGACAATCAGCC
TGGTGGTGGTCCCCAGTAATGTGGACATCGCCACCACAGAGGCTCTCAGCATGGCCCAGGAGGT
GGACCCCGAGGGAGACAGGACCATCGGAATCTTGACGAAGCCTGATCTGGTGGACAAAGGAACT
GAAGACAAGGTTGTGGACGTGGTGCGGAACCTCGTGTTCCACCTGAAGAAGGGTTACATGATTG
 
TCAAGTGCCGGGGCCAGCAGGAGATCCAGGACCAGCTGAGCCTGTCCGAAGCCCTGCAGAGAGA
GAAGATCTTCTTTGAGAACCACCCATATTTCAGGGATCTGCTGGAGGAAGGAAAGGCCACGGTT
CCCTGCCTGGCAGAAAAACTTACCAGCGAGCTCATCACACATATCTGTAAATCTCTGCCCCTGT
TAGAAAATCAAATCAAGGAGACTCACCAGAGAATAACAGAGGAGCTACAAAAGTATGGTGTCGA
CATACCGGAAGACGAAAATGAAAAAATGTTCTTCCTGATAGATAAAGTTAATGCCTTTAATCAG
GACATCACTGCTCTCATGCAAGGAGAGGAAACTGTAGGGGAGGAAGACATTCGGCTGTTTACCA
GACTCCGACACGAGTTCCACAAATGGAGTACAATAATTGAAAACAATTTTCAAGAAGGCCATAA
AATTTTGAGTAGAAAAATCCAGAAATTTGAAAATCAGTATCGTGGTAGAGAGCTGCCAGGCTTT
GTGAATTACAGGACATTTGAGACAATCGTGAAACAGCAAATCAAGGCACTGGAAGAGCCGGCTG
TGGATATGCTACACACCGTGACGGATATGGTCCGGCTTGCTTTCACAGATGTTTCGATAAAAAA
TTTTGAAGAGTTTTTTAACCTCCACAGAACCGCCAAGTCCAAAATTGAAGACATTAGAGCAGAA
CAAGAGAGAGAAGGTGAGAAGCTGATCCGCCTCCACTTCCAGATGGAACAGATTGTCTACTGCC
AGGACCAGGTATACAGGGGTGCATTGCAGAAGGTCAGAGAGAAGGAGCTGGAAGAAGAAAAGAA
GAAGAAATCCTGGGATTTTGGGGCTTTCCAATCCAGCTCGGCAACAGACTCTTCCATGGAGGAG
ATCTTTCAGCACCTGATGGCCTATCACCAGGAGGCCAGCAAGCGCATCTCCAGCCACATCCCTT
TGATCATCCAGTTCTTCATGCTCCAGACGTACGGCCAGCAGCTTCAGAAGGCCATGCTGCAGCT
CCTGCAGGACAAGGACACCTACAGCTGGCTCCTGAAGGAGCGGAGCGACACCAGCGACAAGCGG
AAGTTCCTGAAGGAGCGGCTTGCACGGCTGACGCAGGCTCGGCGCCGGCTTGCCCAGTTCCCCG
GTTAA
IFI30(11) (SEQ ID NO:11)

ATGACCCTGTCGCCACTTCTGCTGTTCCTGCCACCGCTGCTGCTGCTGGACGTCCCCACGG
CGGCGGTGCAGGCGTCCCCTCTGCAAGCGTTAGACTTCTTTGGGAATGGGCCACCAGTTAACTA
CAAGACAGGCAATCTATACCTGCGGGGGCCCCTGAAGAAGTCCAATGCACCGCTTGTCAATGTG
ACCCTCTACTATGAAGCACTGTGCGGTGGCTGCCGAGCCTTCCTGATCCGGGAGCTCTTCCCAA
CATGGCTGTTGGTCATGGAGATCCTCAATGTCACGCTGGTGCCCTACGGAAACGCACAGGAACA
AAATGTCAGTGGCAGGTGGGAGTTCAAGTGCCAGCATGGAGAAGAGGAGTGCAAATTCAACAAG
GTGGAGGCCTGCGTGTTGGATGAACTTGACATGGAGCTAGCCTTCCTGACCATTGTCTGCATGG
AAGAGTTTGAGGACATGGAGAGAAGTCTGCCACTATGCCTGCAGCTCTACGCCCCAGGGCTGTC
GCCAGACACTATCATGGAGTGTGCAATGGGGGACCGCGGCATGCAGCTCATGCACGCCAACGCC
CAGCGGACAGATGCTCTCCAGCCACCGCACGAGTATGTGCCCTGGGTCACCGTCAATGGGAAAC
CCTTGGAAGATCAGACCCAGCTCCTTACCCTTGTCTGCCAGTTGTACCAGGGCAAGAAGCCGGA
TGTCTGCCCTTCCTCAACCAGCTCCCTCAGGAGTGTTTGCTTCAAGTGA

## Claims

1. Polynucleotides for use in a method for treating or preventing a virus infection, wherein the method comprises administering an effective amount of the following to a human subject in need thereof: (i) a polynucleotide encoding MX2, (ii) a polynucleotide encoding IFIT3, (iii) a polynucleotide encoding IFITM1, (iv) a polynucleotide encoding IFI27, (v) a polynucleotide encoding IFIT1, (vi) a polynucleotide encoding BST2, (vii) a polynucleotide encoding IFI6, (viii) a polynucleotide encoding RSAD2, (ix) a polynucleotide encoding MX1, and (x) a polynucleotide encoding IFI30.

2. The polynucleotides for use in the method of claim 1, wherein the nucleic acid sequences are modRNA.

3. A recombinant virus for use in a method for treating or preventing a virus infection, the method comprising administering an effective amount of recombinant virus to a human subject in need thereof, wherein the recombinant virus comprises a nucleotide sequence encoding the following: MX2, IFIT3, IFITM1, IFI27, IFIT1, BST2, IFI6, RSAD2, MX1, and IFI30.

4. Ten recombinant viruses for use in a method for treating or preventing a virus infection, the method comprising administering an effective amount of 10 recombinant viruses to a human subject in need thereof, wherein each recombinant virus comprises a different polynucleotide, and wherein (i) a first recombinant virus comprises a polynucleotide encoding MX2, (ii) a second recombinant virus comprises a polynucleotide encoding IFIT3, (iii) a third recombinant virus comprises a polynucleotide encoding IFITM1, (iv) a fourth recombinant virus comprises a polynucleotide encoding IFI27, (v) a fifth recombinant virus comprises a polynucleotide encoding IFIT1, (vi) a sixth recombinant virus comprises a polynucleotide encoding BST2, (vii) a seventh recombinant virus comprises a polynucleotide encoding IFI6, (viii) an eighth recombinant virus comprises a polynucleotide encoding RSAD2, (ix) a ninth recombinant virus comprises a polynucleotide encoding MX1, and (x) a tenth recombinant virus comprises a transgene comprising a nucleotide sequence encoding IFI30.

5. A lipid nanoparticle for use in a method for treating or preventing a virus infection, the method comprising administering an effective amount of a lipid nanoparticle to a human subject in need thereof, wherein the lipid nanoparticle comprises the following: a nucleotide sequence encoding MX2, a nucleotide sequence encoding IFIT3, a nucleotide sequence encoding IFITM1, a nucleotide sequence encoding IFI27, a nucleotide sequence encoding IFIT1, a nucleotide sequence encoding BST2, a nucleotide sequence encoding IFI6, a nucleotide sequence encoding RSAD2, a nucleotide sequence encoding MX1, and a nucleotide sequence encoding IFI30.

6. The polynucleotides for use of claim 1 or claim 2, the recombinant virus for use of claim 3, the ten recombinant viruses for use of claim 4, or the lipid nanoparticle for use of claim 5, wherein the virus infection is a coronavirus, optionally SARS-CoV-2, an influenza virus, optionally influenza A, vesicular stomatitis virus (VSV), a flavivirus, optionally Zika, Sendai virus, a herpes simplex virus 1 (HSV-1), or a Rift valley fever virus infection.

7. A pharmaceutical composition comprising the polynucleotides as defined in claim 1 or claim 2, the recombinant virus or ten recombinant viruses as defined in claim 3 or claim 4, or the lipid nanoparticle as defined in claim 5, and a pharmaceutically acceptable carrier.

8. The pharmaceutical composition of claim 7, wherein the polynucleotides are modRNA.

9. The pharmaceutical composition of claim 7 or claim 8, wherein the pharmaceutical composition comprising the polynucleotides as defined in claim 1 or claim 2 further comprises a lipid nanoparticle.

## Patentansprüche

1. Polynukleotide zur Verwendung in einem Verfahren zur Behandlung oder Vorbeugung einer Virusinfektion, wobei das Verfahren die Verabreichung einer wirksamen Menge von Folgendem an einen Menschen umfasst, der dies benötigt: (i) ein Polynukleotid, das für MX2 kodiert, (ii) ein Polynukleotid, das für IFIT3 kodiert, (iii) ein Polynukleotid, das für IFITM1 kodiert, (iv) ein Polynukleotid, das für IFI27 kodiert, (v) ein Polynukleotid, das für IFIT1 kodiert, (vi) ein Polynukleotid, das für BST2 kodiert, (vii) ein Polynukleotid, das für IFI6 kodiert, (viii) ein Polynukleotid, das für RSAD2 kodiert, (ix) ein Polynukleotid, das für MX1 kodiert, und (x) ein Polynukleotid, das für IFI30 kodiert.

2. Polynukleotide zur Verwendung in dem Verfahren nach Anspruch 1, wobei es sich bei den Nukleinsäuresequenzen um modRNA handelt.

3. Rekombinantes Virus zur Verwendung in einem Verfahren zur Behandlung oder Vorbeugung einer Virusinfektion, wobei das Verfahren die Verabreichung einer wirksamen Menge des rekombinanten Virus an einen Menschen umfasst, der dies benötigt, wobei das rekombinante Virus eine Nukleotidsequenz umfasst, die für Folgendes kodiert: MX2, IFIT3, IFITM1, IFI27, IFIT1, BST2, IFI6, RSAD2, MX1, und IFI30.

4. Zehn rekombinante Viren zur Verwendung in einem Verfahren zur Behandlung oder Vorbeugung einer Virusinfektion, wobei das Verfahren die Verabreichung einer wirksamen Menge der 10 rekombinanten Viren an einen Menschen umfasst, der dies benötigt, wobei jedes rekombinante Virus ein verschiedenes Polynukleotid umfasst und wobei (i) ein erstes rekombinantes Virus ein Polynukleotid umfasst, das für MX2 kodiert, (ii) ein zweites rekombinantes Virus ein Polynukleotid umfasst, das für IFIT3 kodiert, (iii) ein drittes rekombinantes Virus ein Polynukleotid umfasst, das für IFITM1 kodiert, (iv) ein viertes rekombinantes Virus ein Polynukleotid umfasst, das für IFI27 kodiert, (v) ein fünftes rekombinantes Virus ein Polynukleotid umfasst, das für IFIT1 kodiert, (vi) ein sechstes rekombinantes Virus ein Polynukleotid umfasst, das für BST2 kodiert, (vii) ein siebtes rekombinantes Virus ein Polynukleotid umfasst, das für IFI6 kodiert, (viii) ein achtes rekombinantes Virus ein Polynukleotid umfasst, das für RSAD2 kodiert, (ix) ein neuntes rekombinantes Virus ein Polynukleotid umfasst, das für MX1 kodiert, und (x) ein zehntes rekombinantes Virus ein Transgen umfasst, das eine Nukleotidsequenz umfasst, die für IFI30 kodiert.

5. Lipidnanopartikel zur Verwendung in einem Verfahren zur Behandlung oder Vorbeugung einer Virusinfektion, wobei das Verfahren die Verabreichung einer wirksamen Menge eines Lipidnanopartikels an einen Menschen umfasst, der dies benötigt, wobei der Lipidnanopartikel Folgendes umfasst: eine Nukleotidsequenz, die für MX2 kodiert, eine Nukleotidsequenz, die für IFIT3 kodiert, eine Nukleotidsequenz, die für IFITM1 kodiert, eine Nukleotidsequenz, die für IFI27 kodiert, eine Nukleotidsequenz, die für IFIT1 kodiert, eine Nukleotidsequenz, die für BST2 kodiert, eine Nukleotidsequenz, die für IFI6 kodiert, eine Nukleotidsequenz, die für RSAD2 kodiert, eine Nukleotidsequenz, die für MX1 kodiert, und eine Nukleotidsequenz, die für IFI30 kodiert.

6. Polynukleotide zur Verwendung nach Anspruch 1 oder Anspruch 2, das rekombinante Virus zur Verwendung nach Anspruch 3, die zehn rekombinanten Viren zur Verwendung nach Anspruch 4, oder der Lipidnanopartikel zur Verwendung nach Anspruch 5, wobei es sich bei der Virusinfektion um ein Coronavirus, gegebenenfalls SARS-CoV-2, ein Influenzavirus, gegebenenfalls Influenza A, das Vesikuläre Stomatitis-Virus (VSV), ein Flavivirus, gegebenenfalls Zika, das Sendai-Virus, das Herpes-simplex-Virus 1 (HSV-1) oder eine Infektion mit dem Rift-Valley-Fieber-Virus handelt.

7. Pharmazeutische Zusammensetzung, die die Polynukleotide nach Anspruch 1 oder Anspruch 2, das rekombinante Virus oder zehn rekombinante Viren nach Anspruch 3 oder Anspruch 4, oder das Lipidnanopartikel nach Anspruch 5, und einen pharmazeutisch akzeptablen Trägerstoff umfasst.

8. Pharmazeutische Zusammensetzung nach Anspruch 7, wobei es sich bei den Polynukleotiden um modRNA handelt.

9. Pharmazeutische Zusammensetzung nach Anspruch 7 oder Anspruch 8, wobei die pharmazeutische Zusammensetzung, die die Polynukleotide nach Anspruch 1 oder Anspruch 2 umfasst, weiter ein Lipidnanopartikel umfasst.

## Revendications

1. Polynucléotides destinés à une utilisation dans un procédé destiné à traiter ou prévenir une infection virale, dans lesquels le procédé comprend l'administration d'une quantité efficace de ce qui suit à un sujet humain qui en a besoin : (i) un polynucléotide codant pour MX2, (ii) un polynucléotide codant pour IFIT3, (iii) un polynucléotide codant pour IFITM1, (iv) un polynucléotide codant pour IFI27, (v) un polynucélotide codant pour IFIT1, (vi) un polynucélotide codant pour BST2, (vii) un polynucélotide codant pour IFI6, (viii) un polynucélotide codant pour RSAD2, (ix) un polynucélotide codant pour MX1 et (x) un polynucélotide codant pour IFI30.

2. Polynucléotides destinés à une utilisation dans le procédé selon la revendication 1, dans lesquels les séquences d'acide nucléique sont du modARN.

3. Virus recombinant destiné à une utilisation dans un procédé destiné à traiter ou prévenir une infection virale, le procédé comprenant l'administration d'une quantité efficace de virus recombinant à un sujet humain qui en a besoin, dans lequel le virus recombinant comprend une séquence nucléotidique codant pour les protéines suivantes : MX2, IFIT3, IFITM1, IFI27, IFIT1, BST2, IFI6, RSAD2, MX1 et IFI30.

4. Dix virus recombinants destinés à une utilisation dans un procédé destiné au traitement ou à la prévention d'une infection virale, le procédé comprenant l'administration d'une quantité efficace de 10 virus recombinants à un sujet humain qui en a besoin, dans lesquels chaque virus recombinant comprend un polynucléotide différent et dans lesquels (i) un premier virus recombinant comprend un polynucléotide codant pour MX2, (ii) un deuxième virus recombinant comprend un polynucléotide codant pour IFIT3, (iii) un troisième virus recombinant comprend un polynucléotide codant pour IFITM1, (iv) un quatrième virus recombinant comprend un polynucléotide codant pour IFI27, (v) un cinquième virus recombinant comprend un polynucléotide codant pour IFIT1, (vi) un sixième virus recombinant comprend un polynucléotide codant pour BST2, (vii) un septième virus recombinant comprend un polynucléotide codant pour IFI6, (viii) un huitième virus recombinant comprend un polynucléotide codant pour RSAD2, (ix) un neuvième virus recombinant comprend un polynucléotide codant pour MX1 et(x) un dixième virus recombinant comprend un transgène comprenant une séquence nucléotidique codant pour IFI30.

5. Nanoparticule lipidique destinée à une utilisation dans un procédé destiné à traiter ou à prévenir une infection virale, le procédé comprenant l'administration d'une quantité efficace d'une nanoparticule lipidique à un sujet humain qui en a besoin, dans laquelle la nanoparticule lipidique comprend les séquences suivantes : une séquence nucléotidique codant pour MX2, une séquence nucléotidique codant pour IFIT3, une séquence nucléotidique codant pour IFITM1, une séquence nucléotidique codant pour IFI27, une séquence nucléotidique codant pour IFIT1, une séquence nucléotidique codant pour BST2, une séquence nucléotidique codant pour IFI6, une séquence nucléotidique codant pour RSAD2, une séquence nucléotidique codant pour MX1, et une séquence nucléotidique codant pour IFI30.

6. Polynucléotides destinés à une utilisation selon la revendication 1 ou la revendication 2, virus recombinant destiné à une utilisation selon la revendication 3, dix virus recombinants destinés à une utilisation selon la revendication 4, ou nanoparticule lipidique destinée à une utilisation selon la revendication 5, dans lesquels l'infection virale est un coronavirus, facultativement SARS-COV-2, un virus grippal, facultativement la grippe A, un virus de la stomatite vésiculeuse (VSV), un flavivirus, facultativement le virus Zika, le virus Sendai, le virus de l'herpès simplex 1 (HSV-1) ou une infection par le virus de la fièvre de la vallée du Rift.

7. Composition pharmaceutique comprenant les polynucléotides tels que définis dans la revendication 1 ou la revendication 2, le virus recombinant ou dix virus recombinants tels que définis dans la revendication 3 ou la revendication 4, ou la nanoparticule lipidique telle que définie dans la revendication 5, et un vecteur pharmaceutiquement acceptable.

8. Composition pharmaceutique selon la revendication 7, dans laquelle les polynucléotides sont du modARN.

9. Composition pharmaceutique selon la revendication 7 ou la revendication 8, dans laquelle la composition pharmaceutique comprenant les polynucléotides tels que définis dans la revendication 1 ou la revendication 2 comprend en outre une nanoparticule lipidique.
